# EUROPEAN PATENT APPLICATION

(11) **EP 4 541 794 A1**
(43) Date of publication of application: **23.04.2025**
(21) Application number: 23845685.9
(22) Date of filing: 28.07.2023
(51) Int. Cl.: C07D 413/04, C07D 405/04, A61K 31/343, A61K 31/4365, A61K 31/437, A61K 31/4375, A61P 13/12, A61P 9/12, A61P 9/04, A61P 9/10, A61P 9/08

(54) **PHENYL-SUBSTITUTED DIHYDRONAPHTHYRIDINE COMPOUNDS, AND PREPARATION AND USE THEREOF**

(30) Priority: 29.07.2022 CN 202210914046
(71) Applicant: Suzhong Pharmaceutical Group Co., Ltd., Taizhou, Jiangsu 225500 (CN); Jiangsu Suzhong Pharmaceutical Research Institute Co., Ltd., Nanjing, Jiangsu 210061 (CN)
(72) Inventor: TANG, Haitao, Taizhou, Jiangsu 225500 (CN); TANG, Fan, Taizhou, Jiangsu 225500 (CN); GE, Haitao, Taizhou, Jiangsu 225500 (CN)
(74) Representative: HGF
(86) International application number: PCT/CN2023/109818
(87) International publication number: WO 2024/022481

(57) **Abstract**

The present invention discloses phenyl-substituted dihydronaphthyridine compounds a preparation therefor and a use thereof, and belongs to the technical field of medicine. The compounds disclosed in the present invention are represented by formula I, and can be used as a mineralocorticoid receptor antagonist for treating, preventing or alleviating aldosteronism hyperplasia, diabetic nephropathy, hypertension, heart failure (comprising chronic heart failure, etc.), sequelae of myocardial infarction, liver cirrhosis, renal failure, stroke and other diseases in patients.

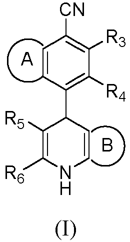

## Description

### TECHNICAL FIELD

The present invention belongs to the technical field of medicine, and in particular relates to phenyl-substituted dihydronaphthyridine compounds, and a preparation therefor and a use thereof, and the compound may be used as a mineralocorticoid receptor antagonist.

### BACKGROUND

Mineralocorticoid receptor (MR) is an aldosterone-activated nuclear hormone receptor, which regulates the expression of many genes involved in electrolyte homeostasis and cardiovascular diseases. The increase of circulating aldosterone increases a blood pressure by its effect on natriuresis, and potentially affects the brain, heart and vascular system at the same time. In addition, aldosteronism hyperplasia is related to many physiological processes leading to kidney and cardiovascular diseases. Although the aldosteronism hyperplasia is usually caused by an aldosterone-producing adenoma, an aldosterone level in a patient with refractory hypertension is often increased, which is often called "aldosterone escape", and caused by the increase of serum potassium content or residual ATIR activity. The aldosteronism hyperplasia and the aldosterone escape typically lead to the increase of MR activity, and it has been proved that an MR antagonist may be used as an effective antihypertensive agent and can also effectively treat heart failure and primary aldosteronism hyperplasia. In addition, the MR antagonist has also been proved to be effective in a preclinical model of nephropathy, and may be combined with standard therapies to reduce proteinuria in a patient with nephropathy, such as chronic nephropathy, comprising diabetic nephropathy.

The aldosterone is a steroid hormone formed in adrenal cortex, and its production depends greatly on a renal blood flow to be indirectly regulated. Any decrease of the renal blood flow leads to the release of renin in kidneys, and the renin enters circulating blood, which further activates the formation of angiotensin II. The angiotensin II has a constriction effect on arterial blood vessels on one hand, but also stimulates the formation of aldosterone in adrenal cortex on the other hand. Therefore, the kidney is used as a blood pressure sensor in blood circulation and indirectly used as a volume sensor, and a serious volume loss is counteracted by a renin-angiotensin-aldosterone system, which is realized by rebalancing a filling state of a vascular system (an effect of aldosterone) through the increase of the blood pressure (an effect of angiotensin II) on one hand and through the reabsorption of sodium and water in kidneys on the other hand. The control system may be pathologically damaged in various ways. For example, the chronic decrease of renal blood flow (such as heart failure and the resulting blood blockage in a venous system) leads to excessive release of aldosterone, followed by the expansion of blood volume, thus weakening the heart by increasing the supply of blood volume to the heart. Obstruction of blood in lungs, shortness of breath, edema of limbs, ascites and pleural effusion may be caused by this; and the renal blood flow is further decreased. In addition, the effect of excessive aldosterone leads to the decrease of potassium concentration in blood and extracellular fluid. In myocardium that has been damaged in other ways before, arrhythmia with fatal results may be induced if there is a level below a critical minimum value, which is probably one of the main causes of sudden cardiac death in patients with heart failure.

In addition, the aldosterone is also considered to be the cause of many myocardial remodeling processes commonly observed in heart failure. Therefore, the aldosteronism hyperplasia is the key to the pathogenesis and prognosis of heart failure (which is initially induced by various types of injuries such as myocardial infarction, myocardial inflammation or hypertension). This hypothesis is supported by the following fact: in extensive clinical studies on patients with chronic heart failure and acute myocardial infarction treated with the aldosterone antagonist, an overall mortality of the patients is significantly reduced (B. Pitt, F. Zannad, W. J. Remme, et al., N. Engl. J. Med. ML 709-717 (1999); B. Pitt, W. Remme, F. Zannad, et al., N. Engl. J. Med 1309-1321 (2003)).

In addition, in visceral tissues, such as kidneys and intestines, the MR regulates sodium retention, potassium excretion and water balance in response to the aldosterone. The expression of MR in the brain also seems to play a role in controlling the excitability of neurons, the negative feedback regulation of hypothalamus-pituitary adrenal axis and the cognition of behavior (Castren, et al., J. of Neuroendocrinology, 3, 461-66(1993)).

The increase of aldosterone level or the excessive stimulation of mineralocorticoid receptor are related to some physiological disorders or pathological disease states, comprising Conn's syndrome, primary and secondary aldosteronism hyperplasia, increased sodium retention, increased magnesium and potassium excretion (polyuria), increased water retention, hypertension (isolated systolic hypertension and combined systolic/diastolic hypertension, etc.), arrhythmia, myocardial fibrosis, myocardial infarction, Bartter's syndrome, and diseases associated with excessive catecholamine level (Hadley, M. E., ENDOCRINOLOGY, 2nd Ed., pp366-81, (1988); and Brilla, et al., Journal of Molecular and Cellular Cardiology, 25(5), pp563-75(1993)). A compound and/or pharmaceutical composition with MR antagonism has a therapeutic value for any of the above diseases.

Although the mineralocorticoid receptor antagonist has made remarkable progress in the treatment of hypertension and heart failure, a current nursing standard is only close to the best standard, and there are still obviously unsatisfied medical needs for other treatments/pharmacological interventions. The present invention meets those needs by providing a compound and composition for treating or preventing diabetic nephropathy, hypertension, heart failure, other cardiovascular diseases and other aldosterone diseases.

### SUMMARY

The present invention provides phenyl-substituted dihydronaphthyridine compounds with mineralocorticoid receptor (MR) antagonism, compound crystals and uses of the compounds in preparation of a drug. The drug is used for treating, preventing or alleviating aldosteronism hyperplasia, diabetic nephropathy, hypertension, heart failure (comprising chronic heart failure, etc.), sequelae of myocardial infarction, liver cirrhosis, renal failure, stroke and other diseases of patients.

In one aspect, the present invention relates to a compound of formula (I), and a stereoisomer, geometric isomer, tautomer, nitrogen oxide, hydrate, solvate, metabolite, ester, pharmaceutically acceptable salt or prodrug thereof; 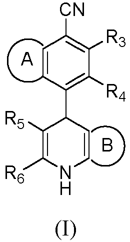 wherein, 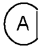 is 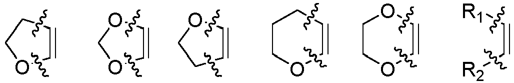
R₁, R₂, R₃ and R₄ are independently hydrogen, deuterium, amino, hydroxyl, sulfhydryl, cyano, nitro, C₁₋₆ alkyl, C₁₋₆ alkoxy, C₁₋₆ haloalkyl, C₁₋₆ haloalkoxy, C₁₋₆ alkylamino, carboxyl, C₁₋₆ alkylacyl, C₁₋₆ alkylsulfonyl, aminoacyl, aminosulfonyl, C₃₋₈ cycloalkyl, C₆₋₁₀ aryl, heterocyclyl consisting of 3 to 8 atoms or heteroaryl consisting of 5 to 10 atoms;
R₅ is 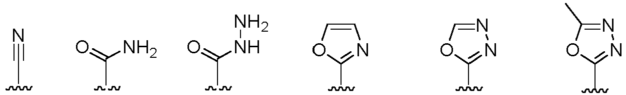
R₆ is hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms; 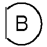 is 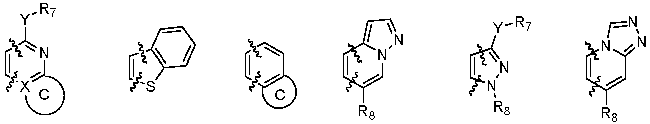
X is C or N;
Y is O or S;
R₇ is C₁₋₆ alkyl, C₃₋₈ cycloalkyl, heterocyclyl consisting of 3 to 8 atoms, heteroaryl consisting of 5 to 6 atoms, C₃₋₈ cycloalkyl C₁₋₆ alkyl, (heterocyclyl consisting of 3 to 8 atoms) C₁₋₆ alkyl, (heteroaryl consisting of 5 to 6 atoms) C₁₋₆ alkyl, phenyl or phenyl C₁₋₆ alkyl; and R₇ is unsubstituted, or substituted by 1, 2, 3 or 4 Rz;
each Rz is independently O, deuterium, fluorine, chlorine, bromine, iodine, hydroxyl, cyano, NH₂, C₁₋₆ alkyl, C₁₋₆ alkoxy, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; wherein each Rz is independently unsubstituted, or substituted by 1, 2, 3 or 4 Rw;
each Rw is independently O, deuterium, fluorine, chlorine, bromine, iodine, hydroxyl, cyano, amino, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; 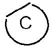 is 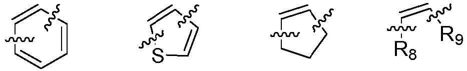
R₈ and R₉ are hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms.

In one aspect, the present invention relates to a compound of formula (I), and a stereoisomer, geometric isomer, tautomer, nitrogen oxide, hydrate, solvate, metabolite, ester, pharmaceutically acceptable salt or prodrug thereof; 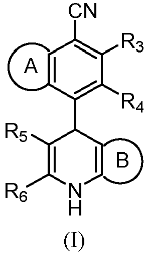 wherein, 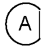 is 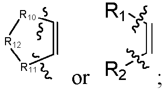
R₁, R₂, R₃ and R₄ are independently hydrogen, deuterium, amino, hydroxyl, sulfhydryl, cyano, nitro, C₁₋₆ alkyl, C₁₋₆ alkoxy, C₁₋₆ haloalkyl, C₁₋₆ haloalkoxy, C₁₋₆ alkylamino, carboxyl, C₁₋₆ alkylacyl, C₁₋₆ alkylsulfonyl, aminoacyl, aminosulfonyl, C₃₋₈ cycloalkyl, C₆₋₁₀ aryl, heterocyclyl consisting of 3 to 8 atoms or heteroaryl consisting of 5 to 10 atoms;
R₅ is 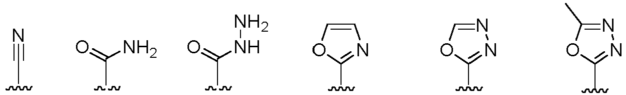
R₆ is hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms; 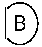 is 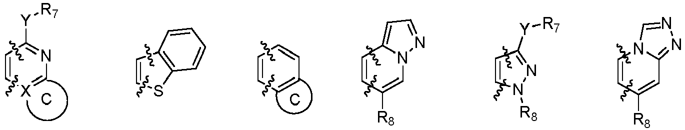
X is C or N;
Y is O or S;
R₇ is C₁₋₆ alkyl, C₃₋₈ cycloalkyl, heterocyclyl consisting of 3 to 8 atoms, heteroaryl consisting of 5 to 6 atoms, C₃₋₈ cycloalkyl C₁₋₆ alkyl, (heterocyclyl consisting of 3 to 8 atoms) C₁₋₆ alkyl, (heteroaryl consisting of 5 to 6 atoms) C₁₋₆ alkyl, phenyl or phenyl C₁₋₆ alkyl; and R₇ is unsubstituted, or substituted by 1, 2, 3 or 4 Rz;
each Rz is independently O, deuterium, fluorine, chlorine, bromine, iodine, hydroxyl, cyano, NH₂, C₁₋₆ alkyl, C₁₋₆ alkoxy, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; wherein each Rz is independently unsubstituted, or substituted by 1, 2, 3 or 4 Rw;
each Rw is independently O, deuterium, fluorine, chlorine, bromine, iodine, hydroxyl, cyano, amino, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; 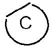 is 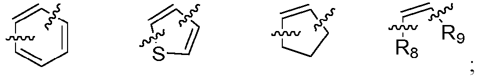
R₈ and R₉ are hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms;
R₁₀ and R₁₁ are independently selected from -CH₂- or O, and at least one of which is O;
R₁₂ is -CH₂- or -CH₂-CH₂-; when R₁₂ is -CH₂-, R₁₀ and R₁₁ are independently selected from -CH₂- or O, and at least one of which is O; and when R₁₂ is -CH₂-CH₂-, R₁₀ and R₁₁ are both O, or R₁₀ is -CH₂-, R₁₁ is O.

In some embodiments, the compound represented by formula (I) of the present invention is selected from the group consisting of compounds represented by formula (Ia) or formula (Ib); and further preferred is the compound represented by formula (Ia);

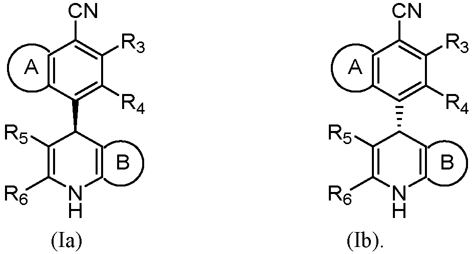

In some embodiments, the compound represented by formula (I) of the present invention is selected from the group consisting of compounds represented by formula (Ia) or formula (Ib); and further preferred is the compound represented by formula (Ia); 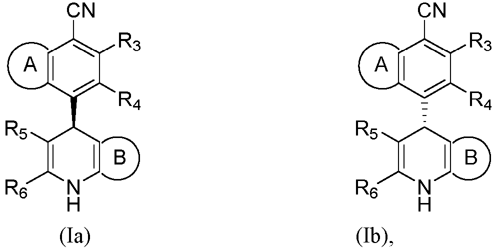 wherein, 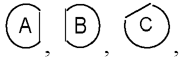 R₁, R₂, R₃, R₄, R₅, R₆, R₇, R₈, R₉, X and Y have the meanings as described in the present invention.

In some embodiments, R₁, R₂, R₃ and R₄ are independently hydrogen, deuterium, amino, hydroxyl, sulfhydryl, cyano, nitro, C₁₋₄ alkyl, C₁₋₄ alkoxy, C₁₋₄ haloalkyl, C₁₋₄ haloalkoxy, C₁₋₄ alkylamino, carboxyl, C₁₋₄ alkylacyl, C₁₋₄ alkylsulfonyl, aminoacyl, aminosulfonyl, C₃₋₆ cycloalkyl, C₆₋₁₀ aryl, heterocyclyl consisting of 3 to 6 atoms or heteroaryl consisting of 5 to 6 atoms;
R₆ is hydrogen, deuterium, C₁₋₄ alkyl, C₁₋₄ alkoxy, C₁₋₄ haloalkyl, C₁₋₄ haloalkoxy, C₁₋₄ alkylamino, C₃₋₆ cycloalkyl, C₆₋₁₀ aryl, heterocyclyl consisting of 3 to 6 atoms or heteroaryl consisting of 5 to 6 atoms; and
R₈ and R₉ are independently hydrogen, deuterium, halogen, cyano, C₁₋₄ alkoxyacyl, carboxyl, C₁₋₄ alkyl, C₁₋₄ alkoxy, C₁₋₄ haloalkyl, C₁₋₄ haloalkoxy, C₁₋₄ alkylamino, C₁₋₄ alkanoyl, C₁₋₄ alkylsulfonyl, aminoacyl or aminosulfonyl.

In some embodiments, R₁, R₂, R₃ and R₄ are independently hydrogen, deuterium, amino, hydroxyl, sulfydryl, cyano, nitro, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino, dimethylamino, carboxyl, methacryl, ethylacyl, methylsulfonyl, aminoacyl or aminosulfonyl;
R₆ is hydrogen, deuterium, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethyl, difluoromethoxy, monofluoromethoxy, methylamino, dimethylamino, cyclopropyl, cyclobutyl, cyclopentyl, cyclohexyl, phenyl, naphthyl, epoxyethyl, pyrrolidinyl, piperidinyl, piperazinyl, morpholinyl, pyridyl, pyrrolyl, thiazolyl, pyrazolyl or pyrimidinyl; and
R₈ is hydrogen, deuterium, cyano, methylacyl, ethylacyl, propylacyl, methoxyacyl, ethoxyacyl, propoxyacyl, carboxyl, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethyl, difluoromethoxy, monofluoromethoxy, methylamino or dimethylamino.

**In** some embodiments, the compound of the present invention is selected from the group consisting of compounds represented by formula (II):

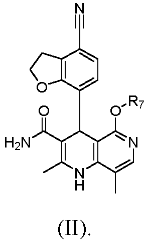

In some embodiments, the compound of the present invention is selected from the group consisting of compounds represented by formula (IIa) or formula (IIb):

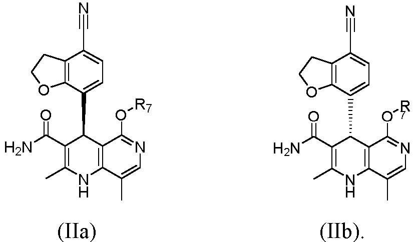

In some embodiments, R₇ is C₃₋₆ cycloalkyl, heterocyclyl consisting of 3 to 6 atoms, heteroaryl consisting of 5 to 6 atoms, C₃₋₆ cycloalkyl C₁₋₄ alkyl, (heterocyclyl consisting of 3 to 6 atoms) C₁₋₄ alkyl or (heteroaryl consisting of 5 to 6 atoms) C₁₋₄ alkyl; wherein R₇ is unsubstituted, or substituted by 1, 2, 3 or 4 Rz.

In some embodiments, R₇ is C₁₋₃ alkyl, wherein R₇ is unsubstituted, or substituted by 1, 2, 3 or 4 Rz; and preferably, R₇ is methyl, ethyl or isopropyl, wherein R₇ is unsubstituted, or substituted by 1, 2, 3 or 4 Rz.

In some embodiments, R₇ is cyclopropyl, cyclobutyl, cyclopentyl, cyclohexyl, oxiranyl, azacyclobutyl, oxacyclobutyl, pyrrolidinyl, tetrahydrofuryl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl, cyclopropyl methyl, cyclopropyl ethyl, cyclobutyl methyl, cyclobutyl ethyl, cyclopentyl methyl, cyclopentyl ethyl, cyclohexyl methyl, cyclohexyl ethyl, azacyclobutyl methyl, azacyclobutyl ethyl, oxacyclobutyl methyl, oxacyclobutyl ethyl, pyrrolidinyl methyl, pyrrolidinyl ethyl, tetrahydrofuryl methyl, tetrahydrofuryl ethyl, tetrahydrothienyl methyl, tetrahydrothienyl ethyl, piperidinyl methyl, piperidinyl ethyl, piperazinyl methyl, piperazinyl ethyl, morpholinyl methyl, morpholinyl ethyl, pyrrolyl methyl, pyrrolyl ethyl, furyl methyl, furyl ethyl, thienyl methyl, thienyl ethyl, thiazolyl methyl, thiazolyl ethyl, pyrazolyl methyl, pyrazolyl ethyl, imidazolyl methyl, imidazolyl ethyl, triazolyl methyl, triazolyl ethyl, tetrazolyl methyl, tetrazolyl ethyl, pyridyl methyl, pyridyl ethyl, pyrimidinyl methyl or pyrimidinyl ethyl; wherein R₇ is unsubstituted, or substituted by 1, 2, 3 or 4 Rz.

In some embodiments, each Rz is independently O, deuterium, fluorine, chlorine, bromine, iodine, hydroxyl, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkylacyl, C₃₋₆ cycloalkyl, heterocyclyl consisting of 3 to 6 atoms, heteroaryl consisting of 5 to 6 atoms or C₆₋₁₀ aryl; wherein each Rz is independently unsubstituted, or substituted by 1, 2, 3 or 4 Rw; each Rw is independently O, deuterium, fluorine, chlorine, bromine, iodine, hydroxyl, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkylacyl, C₃₋₆ cycloalkyl, heterocyclyl consisiting of 3 to 6 atoms, heteroaryl consisting of 5 to 6 atoms or C₆₋₁₀ aryl.

In some embodiments, each Rz is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl; wherein each Rz is independently unsubstituted, or substituted by 1, 2, 3 or 4 Rw; each Rw is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl.

In some embodiments, the compound of the present invention is selected from the group consisting of compounds represented by formula (III): 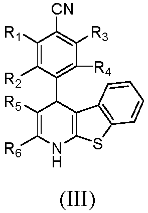 wherein, R₅ is selected from

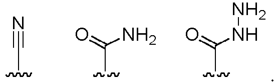

In some embodiments,
R₁, R₂, R₃ and R₄ are independently hydrogen, deuterium or C₁₋₆ alkoxy;
R₅ is 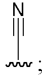 and
R₆ is selected from hydrogen, deuterium or C₁₋₆ alkyl.

In some embodiments,
R₁, R₂, R₃ and R₄ are independently hydrogen, deuterium or C₁₋₃ alkoxy;
R₅ is 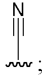 and
R₆ is selected from hydrogen, deuterium or C₁₋₃ alkyl.

In some embodiments,
R₁, R₂, R₃ and R₄ are independently hydrogen, deuterium or methoxy;
R₅ is 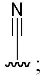 and
R₆ is selected from hydrogen, deuterium or methyl.

In some embodiments, the compound of the present invention is selected from the group consisting of compounds represented by formula (IV): 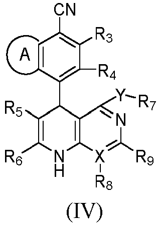 wherein, 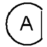 is 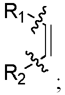
R₁, R₂, R₃ and R₄ are independently hydrogen, deuterium or C₁₋₆ alkoxy;
R₅ is 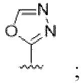
R₆ is C₁₋₆ alkyl;
R₇ is C₁₋₆ alkyl;
R₈ and R₉ are hydrogen, deuterium or C₁₋₆ alkyl;
Y is O; and
X is C.

In some embodiments,
R₁, R₂, R₃ and R₄ are independently hydrogen, deuterium or C₁₋₃ alkoxy;
R₆ is C₁₋₃ alkyl;
R₇ is C₁₋₃ alkyl; and
R₈ and R₉ are hydrogen, deuterium or C₁₋₃ alkyl.

In some embodiments, the compound of the present invention is selected from the group consisting of compounds represented by formula (V): 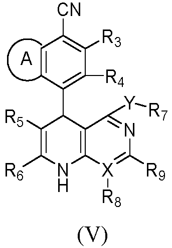 wherein, 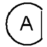 is 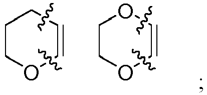
R₃ and R₄ are independently hydrogen or deuterium;
R₅ is 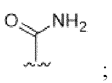
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted, or substituted by 1, 2, 3 or 4 Rz; and each Rz is independently: halogen;
R₈ and R₉ are hydrogen, deuterium or C₁₋₆ alkyl;
X is C; and
Y is O.

In some embodiments,
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted, or substituted by 1, 2, 3 or 4 Rz; and each Rz is independently: fluorine, chlorine, bromine and iodine; and
R₈ and R₉ are hydrogen, deuterium or C₁₋₃ alkyl.

In some embodiments,
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted, or substituted by 1, 2, 3 or 4 Rz; and each Rz is independently: fluorine; and
R₈ and R₉ are hydrogen or methyl.

In some embodiments, the compound of the present invention is selected from the group consisting of compounds represented by formula (V): 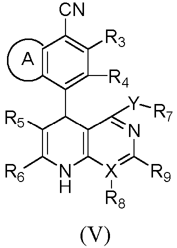 wherein, 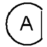 is 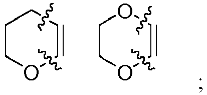
R₃ and R₄ are independently hydrogen or deuterium;
R₅ is 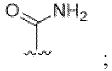
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted, or substituted by 1, 2, 3 or 4 Rz; and each Rz is independently: halogen and C₁₋₆ alkyl;
R₈ and R₉ are hydrogen, deuterium or C₁₋₆ alkyl;
X is C; and
Y is O.

In some embodiments, the compound of the present invention is selected from the group consisting of compounds represented by formula (Va) or formula (Vb); 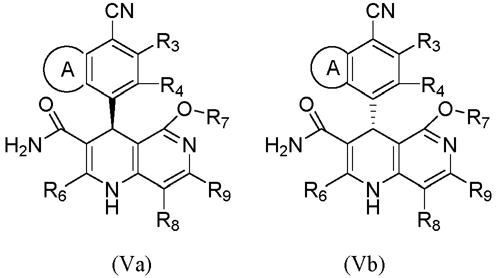 wherein, 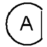 is 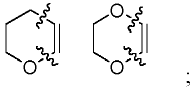
R₃ and R₄ are independently hydrogen or deuterium;
R₅ is 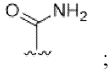
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted, or substituted by 1, 2, 3 or 4 Rz; and each Rz is independently: halogen and C₁₋₆ alkyl;
R₈ and R₉ are hydrogen, deuterium or C₁₋₆ alkyl;
X is C; and
Y is O.

In the above formula (V), formula (Va) and formula (Vb),
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted, or substituted by 1, 2, 3 or 4 Rz; and each Rz is independently: fluorine, chlorine, bromine, iodine and C₁₋₄ alkyl;
R₈ and R₉ are hydrogen, deuterium or C₁₋₃ alkyl.

Further,
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted, or substituted by 1, 2, 3 or 4 Rz; and each Rz is independently: fluorine and methyl; and
R₈ and R₉ are hydrogen or methyl.

In some embodiments, the compound is selected from the group consisting of compounds represented by formula (VI), formula (VIa), or formula (VIb): 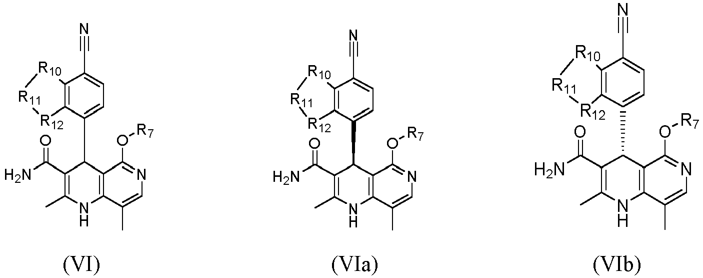
wherein, R₁₀ and R₁₁ are independently selected from -CH₂- or O, and at least one of which is O;
R₁₂ is -CH₂- or -CH₂-CH₂-; when R₁₂ is -CH₂-, R₁₀ and R₁₁ are independently selected from -CH₂- or O, and at least one of which is O; and when R₁₂ is -CH₂-CH₂-, R₁₀ and R₁₁; are both O, or R₁₀ is -CH₂-, R₁₁ is O;
R₇ is C₁₋₃ alkyl, C₃₋₆ cycloalkyl, heterocyclyl consisting of 3 to 6 atoms, heteroaryl consisting of 5 to 6 atoms, C₃₋₆ cycloalkyl C₁₋₄ alkyl, (heterocyclyl consisting of 3 to 6 atoms) C₁₋₄ alkyl or (heteroaryl consisting of 5 to 6 atoms) C₁₋₄ alkyl; wherein R₇ is unsubstituted, or substituted by 1, 2, 3 or 4 Rz; preferably, R₇ is methyl, ethyl, isopropyl, cyclopropyl, cyclobutyl, cyclopentyl, cyclohexyl, oxiranyl, azacyclobutyl, oxacyclobutyl, pyrrolidinyl, tetrahydrofuryl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl, cyclopropyl methyl, cyclopropyl ethyl, cyclobutyl methyl, cyclobutyl ethyl, cyclopentyl methyl, cyclopentyl ethyl, cyclohexyl methyl, cyclohexyl ethyl, azacyclobutyl methyl, azacyclobutyl ethyl, oxacyclobutyl methyl, oxacyclobutyl ethyl, pyrrolidinyl methyl, pyrrolidinyl ethyl, tetrahydrofuryl methyl, tetrahydrofuryl ethyl, tetrahydrothienyl methyl, tetrahydrothienyl ethyl, piperidinyl methyl, piperidinyl ethyl, piperazinyl methyl, piperazinyl ethyl, morpholinyl methyl, morpholinyl ethyl, pyrrolyl methyl, pyrrolyl ethyl, furyl methyl, furyl ethyl, thienyl methyl, thienyl ethyl, thiazolyl methyl, thiazolyl ethyl, pyrazolyl methyl, pyrazolyl ethyl, imidazolyl methyl, imidazolyl ethyl, triazolyl methyl, triazolyl ethyl, tetrazolyl methyl, tetrazolyl ethyl, pyridyl methyl, pyridyl ethyl, pyrimidinyl methyl or pyrimidinyl ethyl; wherein R₇ is unsubstituted, or substituted by 1, 2, 3 or 4 Rz;
preferably, each Rz is independently O, deuterium, fluorine, chlorine, bromine, iodine, hydroxyl, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkylacyl, C₃₋₆ cycloalkyl, heterocyclyl consisting of 3 to 6 atoms, heteroaryl consisting of 5 to 6 atoms or C₆₋₁₀ aryl; wherein each Rz is independently unsubstituted, or substituted by 1, 2, 3 or 4 Rw; each Rw is independently O, deuterium, fluorine, chlorine, bromine, iodine, hydroxyl, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkylacyl, C₃₋₆ cycloalkyl, heterocyclyl consisiting of 3 to 6 atoms, heteroaryl consisting of 5 to 6 atoms or C₆₋₁₀ aryl; and
preferably, each Rz is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl; wherein each Rz is independently unsubstituted, or substituted by 1, 2, 3 or 4 Rw; each Rw is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl.

In some embodiments, the compound of formula (I) of the present invention is specifically a compound of formula (I-10), formula (I-11) or formula (I-12), and a stereoisomer, geometric isomer, tautomer, nitrogen oxide, hydrate, solvate, metabolite, ester, pharmaceutically acceptable salt or prodrug thereof; 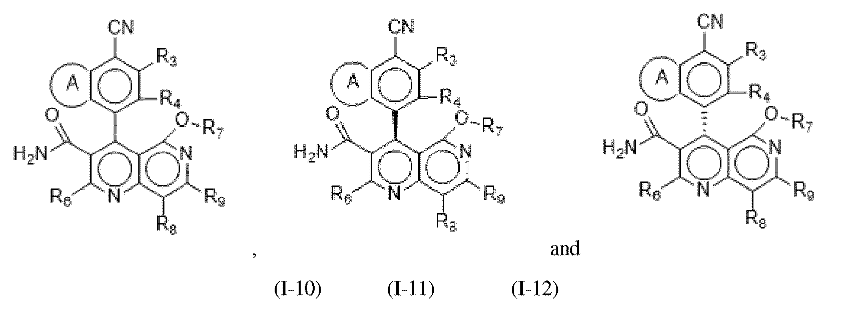
wherein, 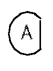 is selected from 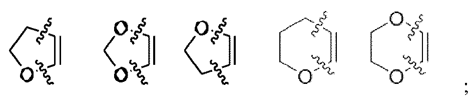 R₃ and R₄ are independently hydrogen or deuterium; R₆ is hydrogen, deuterium or C₁₋₆ alkyl; R₇ is C₁₋₆ alkyl, C₃₋₈ cycloalkyl, C₃₋₈ cycloalkyl C₁₋₆ alkyl; R₇ is unsubstituted, or substituted by 1, 2, 3 or 4 Rz; each Rz is independently O, deuterium, fluorine, chlorine, bromine, iodine, hydroxyl, cyano and NH₂; and R₈ and R₉ are hydrogen, deuterium or C₁₋₆ alkyl;
in the compound represented by formula (I-10), (I-11) or (I-12), R₆ is C₁₋₄ alkyl, and further selected from hydrogen, methyl, ethyl, propyl and butyl;
in the compound represented by formula (I-10), (I-11) or (I-12), R₈ and R₉ are independently hydrogen, deuterium, halogen or C₁₋₄ alkyl;
in the compound represented by formula (I-10), (I-11) or (I-12), R₈ is independently hydrogen, methyl, ethyl, propyl or butyl, and R₉ is independently hydrogen or deuterium;
in the compound represented by formula (I-10), (I-11) or (I-12), R₇ is C₃₋₆ cycloalkyl, C₃₋₈ cycloalkyl or C₃₋₅ cycloalkyl C₁₋₃ alkyl; R₇ is unsubstituted, or substituted by 1, 2, 3 or 4 Rz; and each Rz is independently O, deuterium, fluorine, chlorine, bromine, iodine, hydroxyl, cyano or NH₂; and
in the compound represented by formula (I-10), (I-11) or (I-12), R₇ is cyclopropyl, cyclobutyl, cyclopentyl, cyclohexyl, cyclopropyl methyl, cyclopropyl ethyl, cyclobutyl methyl, cyclobutyl ethyl, cyclopentyl methyl, cyclopentyl ethyl, cyclohexyl methyl or cyclohexyl ethyl.

In the compound represented by formula (I'-10), (I'-11) or (I'-12), R₇ is methyl or ethyl.

In the compound represented by formula (I'-10), (I'-11) or (I'-12), R₇ is substituted by 1 to 3 F.

In some embodiments, the compound is selected from:

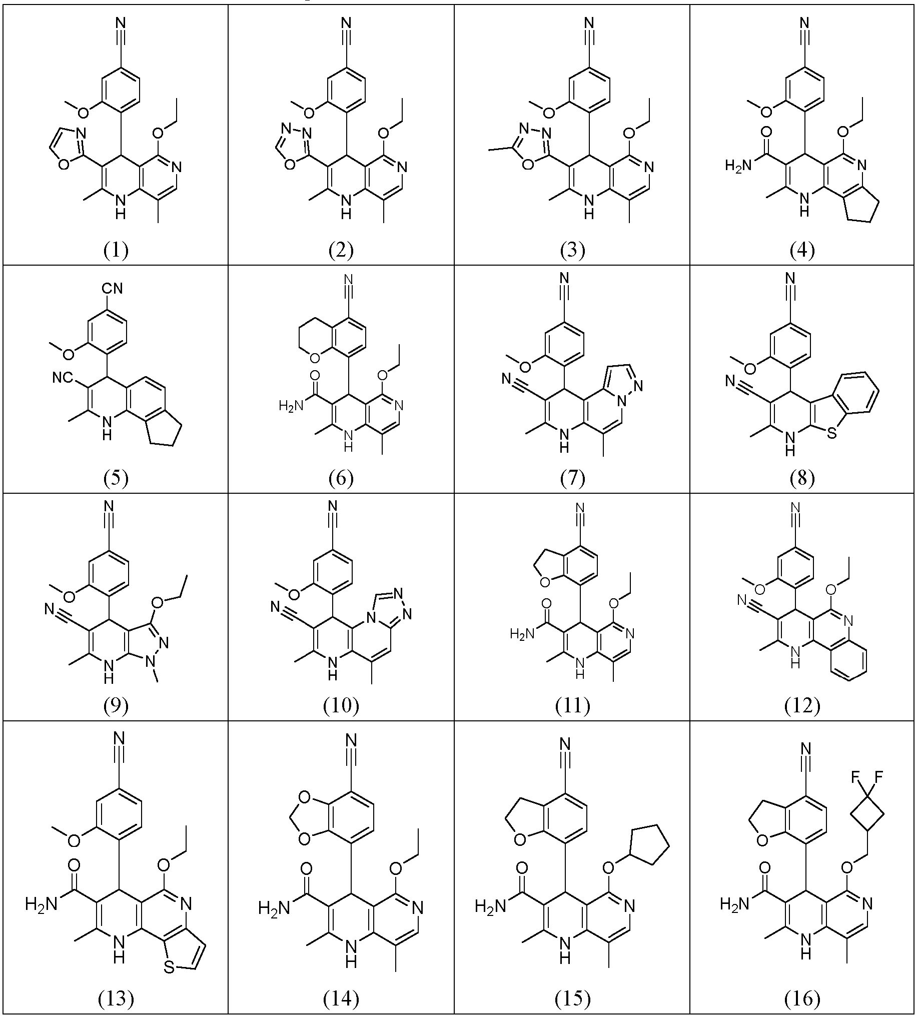

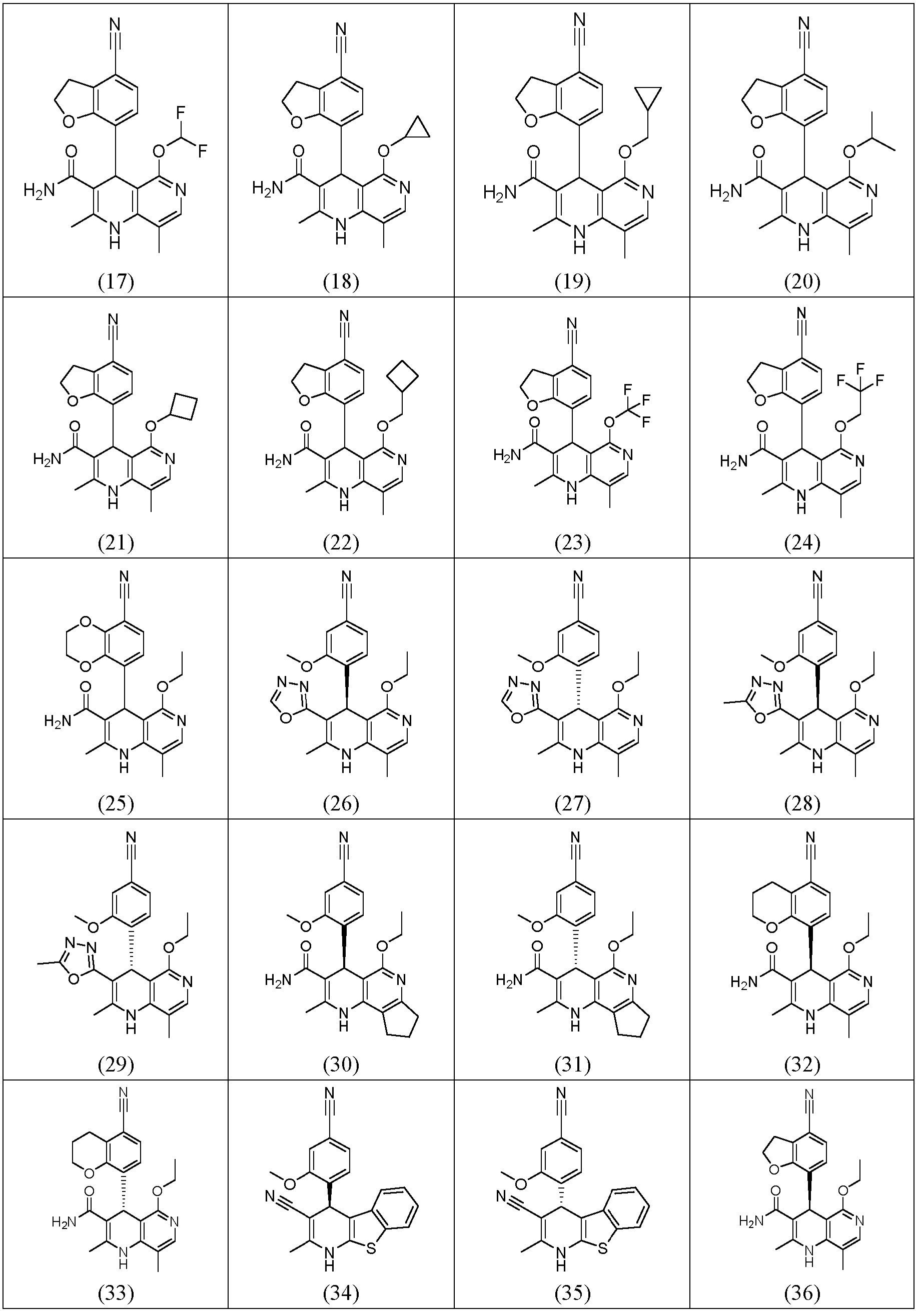

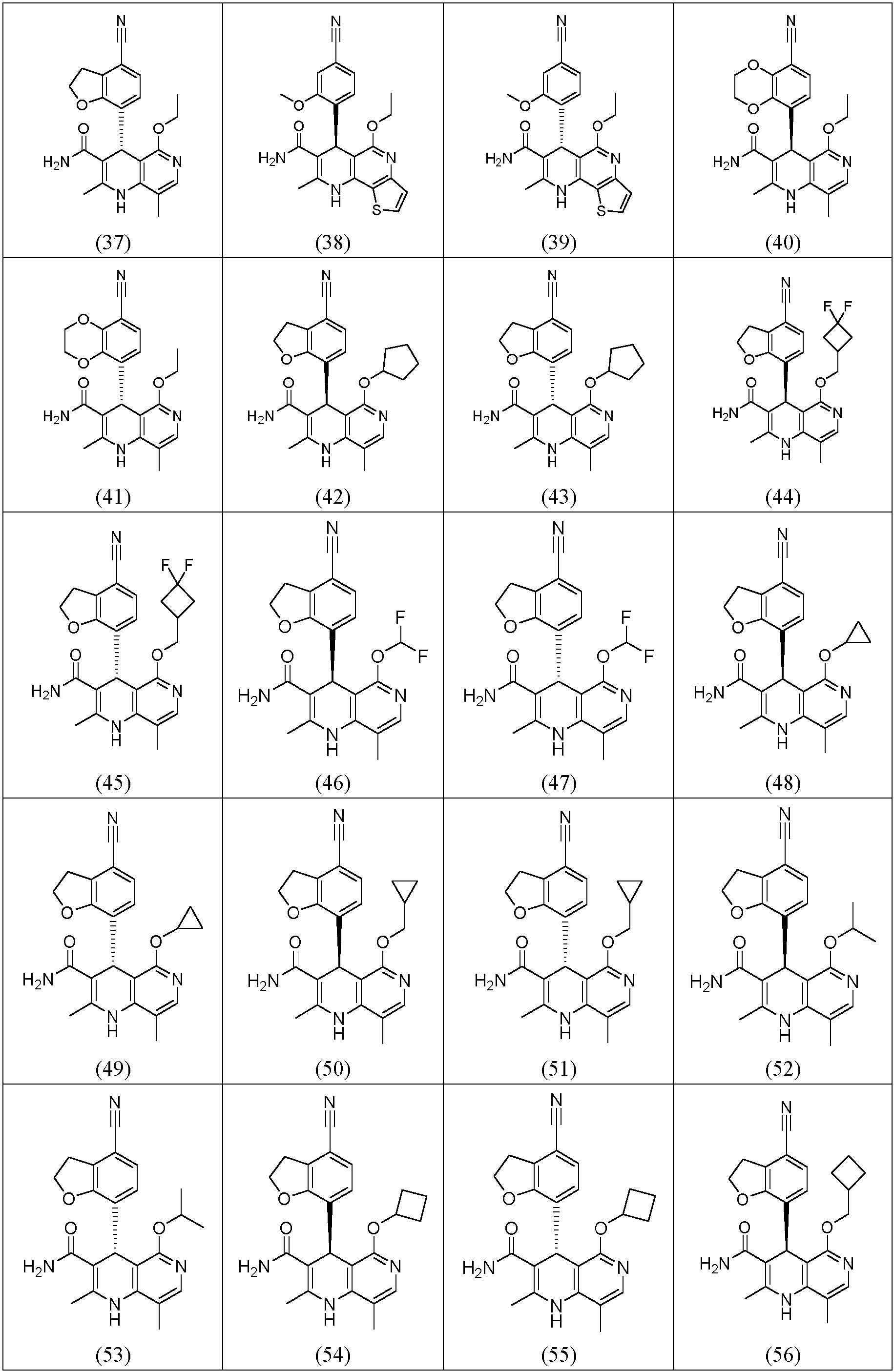

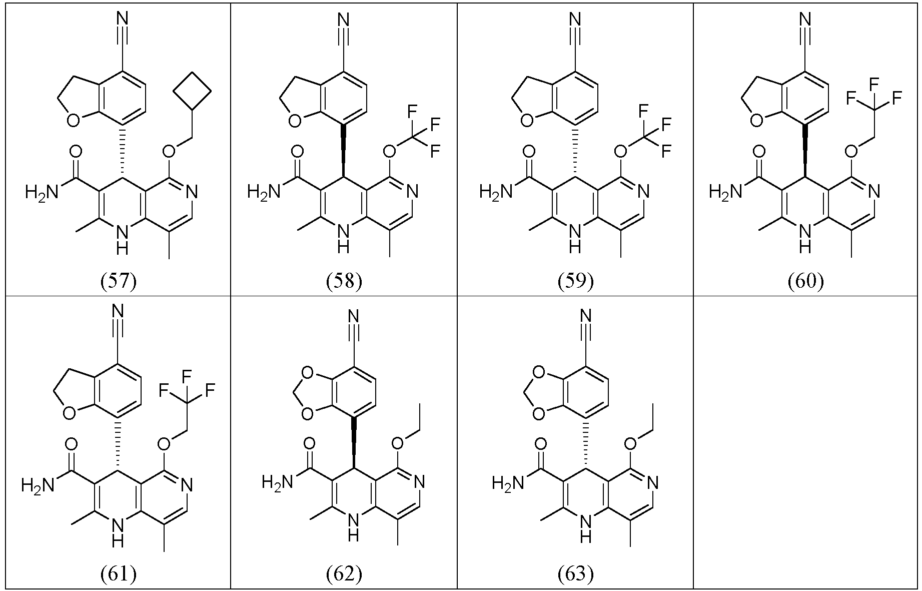

In the present invention, there is no situation that R₁, R₂ and R₃ are hydrogen, R₄ is methoxy, R₅ is -CONH₂ and R₆ is hydrogen in the compounds.

In one aspect, the present invention provides a crystal of one compound, wherein the compound 36 has a structural formula as follows: 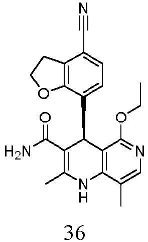 wherein, the crystal of the compound 36 has a structure belonging to a monoclinic system and a C2 space group, and has lattice parameters: a=23.1853(2) [Å], b=8.74723(7) [Å], c=10.94032(10) [Å], α=90°, β=112.7428(8)°, γ=90°, unit cell volume V=2046.27(3) [Å]³, and minimum number of asymmetric units in unit cell Z=4.

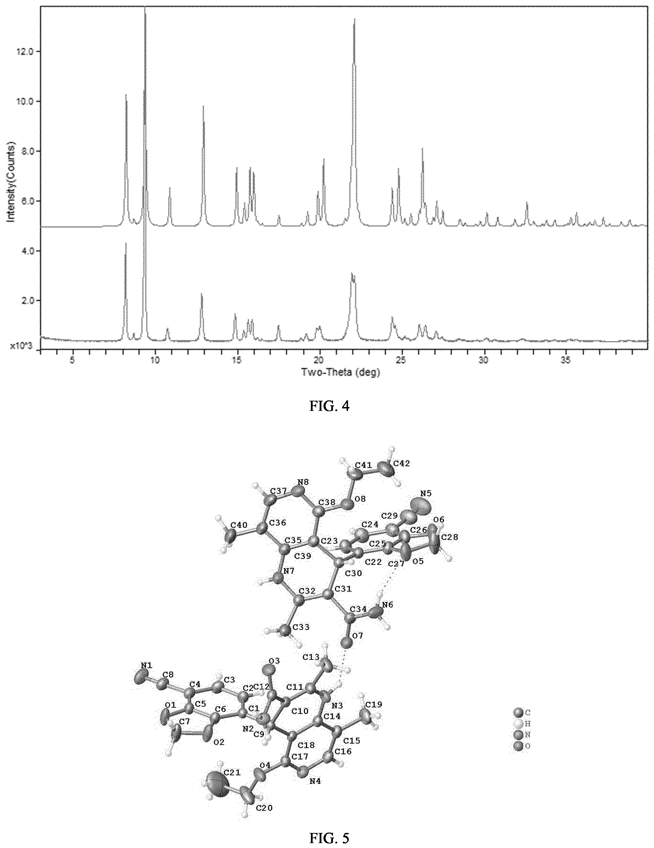

The crystal of the compound 36 or a crystal form thereof has an XRPD pattern as shown in FIG. 4 or basically as shown in FIG. 4, and the term 'basically' means that a difference in number of peaks is within 85%.

The present invention further provides a crystal of another compound 62, wherein the compound 62 has a structural formula as follows: 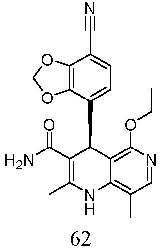 wherein, the crystal of the compound 62 has a structure belonging to a monoclinic system and a C2 space group, and has lattice parameters: a=24.92890(19) [Å], b=8.89441(5) [Å], c=21.18548(18) [Å], α=90°, β=121.6750(10)°, γ=90°, unit cell volume V=3997.68(6) [Å]³, and minimum number of asymmetric units in unit cell Z=8.

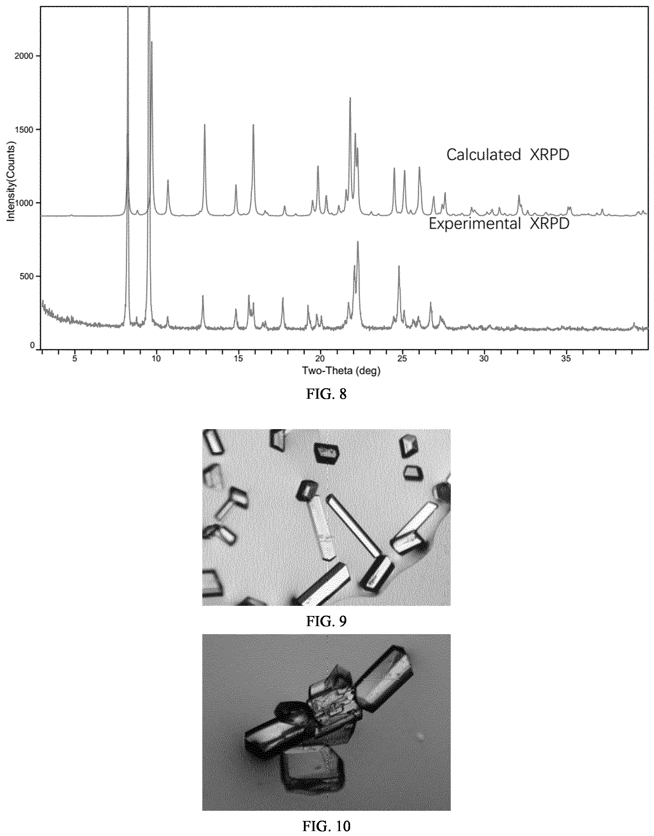

The crystal of the compound 62 or a crystal form thereof has an XRPD pattern as shown in FIG. 8 or basically as shown in FIG. 8, and the term 'basically' means that a difference in number of peaks is within 85%.

As for the crystal form, specifically, the present invention provides the crystal of one compound, wherein the compound is the compound 36, which has the structural formula as shown in the above figure. The crystal of the compound 36 has the structure belonging to the monoclinic system and the C2 space group, and has the lattice parameters: a=23.1853(2) [Å], b=8.74723(7) [Å], c=10.94032(10) [Å], α=90°, β=112.7428(8)°, γ=90°, unit cell volume V=2046.27(3) [Å]3, and minimum number of asymmetric units in unit cell Z=4. Further, the crystal of the compound 36 or the crystal form thereof has an XRPD pattern as shown in FIG. 4 or basically as shown in FIG. 4, and the term 'basically' means that a difference in number of peaks is within 85%. The present invention further provides a crystal of the compound 62, which has a structure belonging to a monoclinic system and a C2 space group, and has lattice parameters: a=24.92890(19) [Å], b=8.89441(5) [Å], C2 21.18548(18) [Å], α=90°, β=121.6750(10)°, γ=90°, unit cell volume V=3997.68(6) [Å]3, and minimum number of asymmetric units in unit cell Z=8. Further, the crystal of the compound 62 or a crystal form thereof has an XRPD pattern as shown in FIG. 8 or basically as shown in FIG. 8, and the term 'basically' means that a difference in number of peaks is within 85%. The present invention further provides a use of the crystal of the compound in preparation of a drug, wherein the drug is used for treating or preventing a mineralocorticoid-related disease or symptom, or used for treating, preventing or alleviating the following disease of a patient: diabetic nephropathy, aldosteronism hyperplasia, hypertension, heart failure, sequelae of myocardial infarction, liver cirrhosis, renal failure or stroke; or the drug is used as a mineralocorticoid receptor antagonist.

Another aspect relates to a use of the compound of the present invention and the stereoisomer, geometric isomer, tautomer, nitrogen oxide, hydrate, solvate, metabolite, ester, pharmaceutically acceptable salt or prodrug thereof in preparation of a drug; or relates to a use of the compound of the present invention and the stereoisomer, geometric isomer, tautomer, nitrogen oxide, hydrate, solvate, metabolite, ester, pharmaceutically acceptable salt or prodrug thereof in preparation of a drug for treating or preventing a mineralocorticoid-related disease or symptom.

The drug is used for treating, preventing or alleviating the following disease of a patient: diabetic nephropathy, aldosteronism hyperplasia, hypertension, heart failure, sequelae of myocardial infarction, liver cirrhosis, renal failure or stroke.

Alternatively, the drug is used as a mineralocorticoid receptor antagonist.

The mineralocorticoid-related disease or symptom is selected from the following disease: diabetic nephropathy, aldosteronism hyperplasia, hypertension, heart failure, sequelae of myocardial infarction, liver cirrhosis, renal failure or stroke.

### Definitions and general terms

Certain embodiments of the present invention are now described in detail, and examples in the embodiments are illustrated by the attached structural formulas and chemical formulas. The present invention is intended to cover all substitutions, modifications and equivalent technical solutions, which are all included within the scope of the present invention as defined by the claims. Those skilled in the art should recognize that many methods and materials similar or equivalent to those described herein can be used to practice the present invention. The present invention is in no way limited to the methods and materials described herein. In the case that one or more of the combined documents, patents and similar materials are different from or contradictory to the present application (comprising, but being not limited to, defined terms, term applications, described technologies, etc.), the present application should prevail.

It should be further recognized that certain features of the present invention have been described in several independent embodiments for clarity, but may also be provided in combination in a single embodiment. On the contrary, various features of the present invention are described in a single embodiment for brevity, but may also be provided individually or in any suitable sub-combination.

Unless otherwise specified, all technical and scientific terms used in the present invention have the same meanings as those commonly understood by those skilled in the art to which the present invention belongs. All patents and publications related to the present invention are incorporated in the present invention as a whole by reference.

Unless otherwise specified, the following definitions used herein should be applied. For the objective of the present invention, chemical elements are consistent with the CAS version of the periodic table of elements and the Handbook of Chemistry and Physics, 75th edition, 1994. In addition, general principles of organic chemistry may refer to the descriptions in "Organic Chemistry", Thomas Sorrell, University Science Books, Sausalito: 1999, and "March's Advanced Organic Chemistry" by Michael B. Smith and Jerry March, John Wiley&Sons, New York: 2007, the entire contents of which are incorporated herein by reference.

The articles "a", "an" and "the" used herein are intended to include "at least one" or "one or more" unless otherwise specified or there is clear contradiction in the context. Therefore, as used herein, these articles refer to articles for one or more than one (that is, at least one) object. For example, "an ingredient" refers to one or more ingredients, that is, there may be more than one ingredient to be considered for adoption or use in the implementation of the embodiment.

The term "patient" used in the present invention refers to human (including adults and children) or other animals. In some embodiments, the "patient" refers to human.

The term "comprise" is an open-type expression, which means to comprise the content indicated in the present invention, but does not exclude contents in other aspects.

The "stereoisomer" refers to a compound with the same chemical composition but different spatial arrangement of atoms or groups. The stereoisomer comprises an enantiomer, a diastereomer, a conformational isomer (rotational isomer), a geometric isomer (cis/trans) isomer, a trans-hindered isomer, and the like.

The "enantiomer" refers to two isomers of one compound that cannot overlap, but are in a mirror-image relation.

The "diastereomer" refers to a stereoisomer that has two or more chiral centers, and has molecules not in a mirror-image relation. The diastereomer has different physical properties, such as a melting point, a boiling point, spectral properties and reactivity. A mixture of diastereomers may be separated by a high-resolution analytical operation of electrophoresis and chromatography, such as HPLC.

Stereochemical definitions and rules used in the present invention generally follow: S. P. Parker, Ed., McGraw-Hill Dictionary of Chemical Terms (1984) McGraw-Hill Book Company, New York; and Eliel, E.and Wilen, S., "Stereochemistry of Organic Compounds", John Wiley&Sons, Inc., New York, 1994.

Any asymmetric atom (such as carbon, etc.) of the compounds disclosed in the present invention may exist in a racemic or enantiomerically enriched form, such as (R)-, (S)- or (R ,S)- configuration form. In some embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)- or (S)-configuration.

A mixture of any stereoisomers obtained may be separated into pure or substantially pure geometric isomers, enantiomers and diastereomers according to differences of ingredients in physical and chemical properties, such as by chromatography and/or fractional crystallization.

The term "tautomer" or "tautomeric form" refers to structural isomers with different energies that may be mutually transformed through a low energy barrier. If tautomerism is possible (such as in a solution), the chemical equilibrium of tautomers can be achieved. For example, a protontautomer (also called a prototropic tautomer) comprises mutual transformation by proton transfer, such as ketone-enol isomerization and imine-enamine isomerization. A valence tautomer comprises mutual transformation by recombination of some bonding electrons. A specific example of keto-enol tautomerism is the tautomerism of pentane-2,4-dione and 4-hydroxypent-3-ene-2-one. Another example of tautomerism is phenol-ketone tautomerism. A specific example of phenol-ketone tautomerism is the tautomerism of pyridine-4-ol and pyridine-4(1H)-ketone. Unless otherwise specified, all tautomer forms of the compounds of the present invention are included in the scope of the present invention.

As described in the present invention, the compounds of the present invention may be optionally substituted by one or more substituents, such as the compounds of the above general formulas, or special examples in the embodiments, subclasses, and a class of compounds included in the present invention.

In addition, it should be noted that, unless otherwise explicitly specified, the description modes of "each ... independently" as well as "... respectively independently" and "...independently" used in the present invention are interchangeable, and should be understood in the broad sense, which means that specific options expressed by the same symbols in different groups do not affect each other, or specific options expressed by the same symbols in the same group do not affect each other. In the same way, the "independently" in the description mode of "... independently optionally" should also be understood in the broad sense above.

The term "optional" or "optionally" means that the subsequently described event or situation may, but does not necessarily, occur, that is, the description comprises the case in which the event or situation occurs and the case in which the event or situation does not occur.

In various parts of the specification, the substituents of the compounds disclosed in the present invention are disclosed according to the type or range of the groups. In particular, the present invention comprises each independent sub-combination of various members in the type or range of the groups. For example, the term "C₁-C₆ alkyl" or "C₁-₆ alkyl" particularly refers to methyl, ethyl, C₃ alkyl, C₄ alkyl, C₅ alkyl and C₆ alkyl which are independently disclosed; and "C₁-₄ alkyl" refers to methyl, ethyl, C₃ alkyl (that is, propyl, comprising n-propyl and isopropyl) and C₄ alkyl (that is, butyl, comprising n-butyl, isobutyl, sec-butyl and tert-butyl) which are independently disclosed.

In various parts of the present invention, the linkage of substituents is described. When the structure clearly requires a linking group, a Markush variable listed for the group should be understood as the linking group. For example, if the structure requires the linking group and "alkyl" or "aryl" is enumerated according to the definition of the Markush group for this variable, it should be understood that the "alkyl" or "aryl" represents a linking alkylene group or arylene group respectively.

The term "alkyl" or "alkyl group" used in the present invention repesents a saturated linear or branched monovalent hydrocarbon group containing 1 to 20 carbon atoms, wherein the alkyl group may be optionally substituted by one or more substituents described in the present invention. In some embodiments, the alkyl group contains 1 to 12 carbon atoms; in other embodiments, the alkyl group contains 1 to 6 carbon atoms, that is, C₁₋₆ alkyl; in yet other embodiments, the alkyl group contains 1 to 4 carbon atoms, that is, C₁₋₄ alkyl; and in yet still other embodiments, the alkyl group contains 1 to 3 carbon atoms, that is, C₁₋₃ alkyl. In some embodiments, the C₁₋₆ alkyl in the present invention comprises the C₁₋₄ alkyl; and in other embodiments, the C₁₋₆ alkyl in the present invention comprises the C₁₋₃ alkyl.

Examples of the alkyl group comprise, but are not limited to, methyl, ethyl, propyl (comprising n-propyl and isopropyl), butyl (comprising n-butyl, isobutyl, sec-butyl and tert-butyl), n-pentyl, 2-pentyl, 3-pentyl, 2-methyl-2-butyl, 3-methyl-2-butyl, 3-methyl-1-butyl, 2-methyl-1-butyl, n-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, n-heptyl, n-octyl, and the like.

The term "alkoxy" means that the alkyl group is linked to the rest of molecule through an oxygen atom, wherein the alkyl group has the meaning as defined in the present invention. Examples of the alkoxy group comprise, but are not limited to, methoxy, ethoxy, propoxy (comprising 1-propoxy or 2-propoxy), butoxy (comprising n-butoxy, isobutoxy, sec-butoxy, tert-butoxy), and the like.

The term "haloalkyl" or "haloalkoxy" means that the alkyl or alkoxy group is substituted by one or more halogen atoms, and such examples comprise, but are not limited to, trifluoromethyl, trifluoromethoxy, chloroethyl (such as 2-chloroethyl), trifluoroethyl (comprising, but being not limited to, 2,2-trifluoroethyl), 2,2-difluoroethyl, 2-chloro-1-methyl ethyl, and the like.

The term "amino" represents a group -NH₂. The term "carboxyl" represents a group -COOH. The terms "hydroxyl", "cyano", "nitro" and "sulfydryl" respectively represent groups -OH, -CN, -NO2 and -SH. The term "oxo" represents a group =O.

The term "alkylamino" or "alkylamino group" means that the group -NH₂ is substituted by one or two alkyl groups, that is, the "alkylamino" or "alkylamino group" comprises monoalkylamino and dialkylamino; wherein the alkyl has the meaning according to the present invention. Examples of the alkylamino group comprise, but are not limited to, methylamino, ethylamino, methylethylamino, dimethylamino, and the like.

The term "cycloalkyl" represents a saturated monocyclic, bicyclic or tricyclic system containing 3 to 12 ring carbon atoms. In some embodiments, the cycloalkyl contains 3 to 10 ring carbon atoms, such as C₃₋₁₀ cycloalkyl; in other embodiments, the cycloalkyl contains 3 to 8 ring carbon atoms, such as C₃₋₈ cycloalkyl; and in yet other embodiments, the cycloalkyl contains 3 to 6 ring carbon atoms, such as C₃₋₆ cycloalkyl. Examples of the cycloalkyl group comprise, but are not limited to, cyclopropyl, cyclobutyl, cyclopentyl, cyclohexyl, cycloheptyl, cyclooctyl, and the like. According to the present invention, the C₃₋₈ cycloalkyl comprises the C₃₋₆ cycloalkyl; and the C₃₋₆ cycloalkyl comprises cyclopropyl, cyclobutyl, cyclopentyl and cyclohexyl. The cycloalkyl group may optionally be substituted by one or more substituents described in the present invention.

The term "heterocyclyl" refers to a saturated or partially unsaturated monocyclic, bicyclic or tricyclic system containing 3 to 12 ring atoms, wherein at least one ring atom is selected from nitrogen, sulfur and oxygen atoms; and the heterocyclyl is non-aromatic, and does not contain any aromatic ring. Unless otherwise specified, the heterocyclyl may be carbon-based or nitrogen-based, and the -CH₂- group may optionally be substituted by -C(=O)-. The sulfur atom of the ring may optionally be oxidized into an S-oxide. The nitrogen atom of the ring may optionally be oxidized into an N-oxide. The term "heterocyclyl" may be used interchangeably with the term "heterocycle". Examples of the heterocyclyl comprise, but are not limited to, oxiranyl, azacyclobutyl, oxetanyl, pyrrolidinyl, tetrahydrofuranyl, tetrahydrothienyl, thiazolidinyl, pyrazolidinyl, oxazolidinyl, imidazolidinyl, isoxazolinyl, piperidinyl, piperazinyl or morpholinyl, and the like. According to the present invention, the heterocyclyl may be consisting of 3 to 8 atoms or 3 to 6 atoms, the atom is C, N, O or S, and at least one atom is N, O or S; wherein, the heterocyclyl consisting of 3 to 8 atoms comprises the heterocyclyl consisting of 3 to 6 atoms; the heterocyclyl consisting of 3 to 6 atoms comprises the heterocyclyl consisting of 3 to 5 atoms; and specifically, the heterocyclyl consisting of 3 to 6 atoms comprises, but is not limited to, oxiranyl 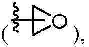 aziridinyl 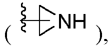 azacyclobutyl 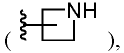 oxetanyl 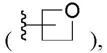 pyrrolidinyl 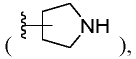 tetrahydrofuranyl 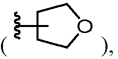 tetrahydrothienyl 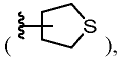 thiazolidinyl 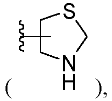 pyrazolidinyl 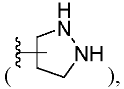 pyrazolinyl 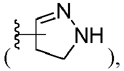 oxazolidinyl 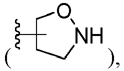 imidazolidinyl 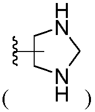 piperidinyl 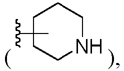 piperazinyl 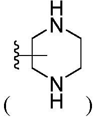 or morpholinyl 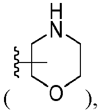 and the like. The heterocyclyl group may optionally be substituted by one or more substituents described in the present invention.

The term "halogen" refers to fluorine (F), chlorine (Cl), bromine (Br) or iodine (I).

The term "aryl" represents a monocyclic, bicyclic or tricyclic carbocyclic system containing 6 to 14 ring atoms, or 6 to 12 ring atoms, or 6 to 10 ring atoms, wherein at least one ring is aromatic, and one or more attachment points are connected with the rest of molecule. The term "aryl" may be used interchangeably with the term "aromatic nucleus" or "aromatic ring". Examples of the aryl group may comprise phenyl, 2,3-dihydro-1H-indenyl, naphthyl and anthryl. The aryl group may optionally be substituted by one or more substituents described in the present invention. Unless otherwise specified, the group "C₆₋₁₀ aryl" represents an aryl group containing 6 to 10 ring carbon atoms.

The term "heteroaryl" represents a monocyclic, bicyclic or tricyclic system containing 5 to 12 ring atoms, or 5 to 10 ring atoms, or 5 to 6 ring atoms, wherein at least one ring is aromatic, and at least one ring contains 1, 2, 3 or 4 ring heteroatoms selected from nitrogen, oxygen and sulfur, and meanwhile, the heteroaryl has one or more attachment points connected with the rest of molecule. When the -CH₂- group exists in the heteroaryl group, the -CH₂- group may optionally be substituted by -C(=O)-. Unless otherwise specified, the heteroaryl group may be linked to the rest of molecule (such as the main structure in the general formula) through any reasonable site (which may be C in CH or N in NH). The term "heteroaryl" may be used interchangeably with the term "heteroaromatic ring" or "heteroaromatic compound". Examples of the heteroaryl comprise, but are not limited to, furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, and the like. The heteroaryl group may optionally be substituted by one or more substituents described in the present invention. In some embodiments, the heteroaryl is the heteroaryl consisting of 5 to 10 atoms, which means that the heteroaryl contains 1 to 9 ring carbon atoms, and 1, 2, 3 or 4 ring heteroatoms selected from O, S and N; in other embodiments, the heteroaryl is the heteroaryl consisting of 5 to 6 atoms, which means that the heteroaryl contains 1 to 5 ring carbon atoms, and 1, 2, 3 or 4 ring heteroatoms selected from O, S and N. Examples of the heteroaryl consisting of 5 to 6 atoms comprise, but are not limited to, furyl, imidazolyl, isoxazolyl, oxazolyl, pyrrolyl, pyrazolyl, pyridyl, pyrimidinyl, pyridazinyl, pyrazinyl, thienyl, thiazolyl, and the like.

The term "consisting of j-k atoms (j and k are independently any non-zero natural number, and k>j)" means that the cyclic group consists of j-k ring atoms, and the ring atoms comprise carbon atoms and/or heteroatoms such as O, N, S and P; and the "j-k" comprises j, k and any natural number between them. For example, "consisting of 3 to 8 atoms", "consisting of 5 to 10 atoms" or "consisting of 5 to 6 atoms" means that the cyclic group consists of 3 to 8, 5 to 10 or 5 to 6 ring atoms, and the ring atoms comprise carbon atoms and/or heteroatoms such as O, N, S and P.

When a composite group with two or more groups linked appears herein, the linking site refers to the general principle of chemistry, that is, the linking site is the last noun group in the name of the composite group. For example, when "C₃₋₈ cycloalkyl C₁₋₆ alkyl, 3-8 membered heterocycloalkyl C₁₋₆ alkyl, phenyl C₁₋₆ alkyl and 5-6 membered heteroaryl C₁₋₆ alkyl" appear, the linking sites with the main structural group or other groups are all "C₁₋₆ alkyl"; and other similar groups are understood with reference to the principle unless otherwise specified.

The "heterocyclyl consisting of 3 to 8 atoms" and the "heterocyclyl consisting of 3-6 atoms" in the present invention refer to the heterocyclyl consisting of 3 to 8 ring atoms or the heterocyclyl consisting of 3 to 6 ring atoms, wherein the heterocyclyl contains 1, 2, 3 or 4 heteroatoms selected from N, O and S, and CH₂ in the heterocyclyl may be further oxidized to form C(=O). In the same way, S or N in the heterocycle may also be further oxidized to form S(=O), S(=O)₂ or N(=O).

The "heteroaryl consisting of 5 to 10 atoms" and the "heteroaryl consisting of 5 to 6 atoms" in the present invention refer to the heteroaryl containing 5 to 10 ring atoms and the heteroaryl containing 5 to 6 ring atoms, wherein the heteroaryl contains 1, 2, 3 or 4 heteroatoms selected from N, O and S. In some embodiments, specific examples of the "heteroaryl consisting of 5 to 10 atoms" or the "heteroaryl consisting of 5 to 6 atoms" in the present invention comprise, but are not limited to, pyridyl, pyrimidinyl, pyrazinyl, thienyl, thiazolyl, furyl, pyrrolyl, pyrazolyl, imidazolyl, triazolyl, and the like.

The term "alkanoyl" or "alkylacyl" represents the group -C(=O)- alkyl, wherein the alkyl has the meaning as defined in the present invention, and such examples comprise, but are not limited to, methyl acyl (-C(=O)CH₃), ethyl acyl (-C(=O)CH₂CH₃), and the like.

The term "alkoxyacyl" represents the group -C(=O)-R, wherein R is alkoxy, the alkoxy has the meaning as defined in the present invention, and such examples comprise, but are not limited to, methoxyacyl (-C(=O)OCH₃), ethoxyacyl (-C(=O)OCH₂CH₃), and the like.

The term "alkylsulfonyl" represents the group -S(=O)₂- alkyl, wherein the alkyl has the meaning as described in the present invention, and such examples comprise, but are not limited to, methylsulfonyl (-S(=O)₂CH₃), ethylsulfonyl (-S(=O)₂CH₂CH₃), and the like.

The term "aminosulfonyl" represents the group -S(=O)₂NH₂; and the term "aminoacyl" represents the group -C(=O)NH₂.

The term "pharmaceutically acceptable" refers to molecular entities and compositions that are physiologically tolerable when administered to humans and generally do not produce allergies or similar inappropriate reactions, such as gastrointestinal discomfort and dizziness. Preferably, the term "pharmaceutically acceptable" used herein refers to those used in animals, especially in humans, which are approved by federal regulatory agencies or national governments, or listed in the United States Pharmacopoeia or other generally recognized Pharmacopoeia.

The term "carrier" refers to a diluent, an adjuvant, an excipient or a matrix applied with the compounds. These drug carriers may be a sterile liquid, such as water and oil, comprising oil from petroleum, animals, plants or synthetic sources, such as peanut oil, soybean oil, mineral oil and sesame oil. The carrier is preferably water and an aqueous solution (such as a saline solution, a glucose aqueous solution and a glycerol aqueous solution), especially an injectable solution. A suitable pharmaceutical carrier is described in "Remington's Pharmaceutical Sciences" of E. W. Martin.

The term "prodrug" used in the present invention means that a compound is transformed into a compound represented by formula (I) in vivo. Such transformation is influenced by the hydrolysis of the prodrug in blood or the enzymatic transformation of the prodrug into a parent structure in blood or tissues. The prodrug compound of the present invention may be an ester, and the ester capable of being used as the prodrug in the prior art comprises phenyl esters, aliphatic (C₁₋₂₄) esters, acyloxymethyl esters, carbonic esters, carbamates and amino acid esters. For example, one compound in the present invention contains hydroxyl, which is a compound in which the hydroxyl may be acylated to obtain a prodrug form. Other prodrug forms comprise phosphate, for example, these phosphate compounds are obtained by phosphorylation of hydroxyl on the parent structure. The complete discussion about the prodrug refers to the following documents: T. Higuchi and V. Stella, Pro-drugs as Novel Delivery Systems, Vol. 14 of the A. C. S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

The "metabolite" refers to a product obtained by metabolism of a specific compound or its salt in vivo. A metabolite of a compound may be identified by the technologies well known in the art, and its activity may be characterized by an experimental method as described in the present invention. Such product may be obtained by oxidation, reduction, hydrolysis, amidation, deamidation, esterification, degreasing, enzymatic cracking, and the like of an administering compound. Accordingly, the present invention comprises the metabolites of the compounds, comprising the metabolites produced by fully contacting the compounds of the present invention with mammals for a period of time.

The "pharmaceutically acceptable salts" used in the present invention refer to organic salts and inorganic salts of the compounds of the present invention. The pharmaceutically acceptable salts are well known in the art, such as the salts recorded in the document: S. M. Berge et al., describe pharmaceutically acceptable salts in detail in J. Pharmaceutical Sciences, 1977, 66: 1-19. Pharmaceutically acceptable non-toxic acid-forming salts comprise, but are not limited to, inorganic acid salts, such as hydrochloride, hydrobromide, phosphate, sulfate and perchlorate; organic acid salts, such as acetate, oxalate, maleate, tartrate, citrate, succinate and malonate; or these salts obtained by other methods recorded in books and documents, such as ion exchange. Other pharmaceutically acceptable salts comprise adipate, alginate, ascorbate, aspartate, benzenesulfonate, benzoate, bisulfate, borate, butyrate, camphorate, camphorsulfonate, cyclopentyl propionate, digluconate, dodecyl sulfate, ethanesulfonate, formate, fumarate, gluceptate, glycerophosphate, gluconate, hemisulfate, heptanate, caproate, hydroiodate, 2-hydroxy-ethanesulfonate, lactouronate, lactate, laurate, lauryl sulfate, malate, methanesulfonate, 2-naphthalenesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, valerate, and the like. Salts obtained by reaction with appropriate bases comprise salts of alkali metals, alkaline-earth metals, ammonium and N⁺(C₁₋₄ alkyl)4. The present invention is also intended to provide quaternary ammonium salts formed by any compounds containing N groups. Water soluble or oil soluble or dispersed products may be obtained by quaternization. The alkali or alkaline-earth metals capable of forming the salts comprise sodium, lithium, potassium, calcium, magnesium, and the like. The pharmaceutically acceptable salts further comprise appropriate and nontoxic ammonium, quaternary ammonium salts and amine cations formed by counterions, such as a halide, a hydroxide, a carboxylate, a sulfate, a phosphide, a nitrate, a C₁₋₈ sulfonate and an aromatic sulfonate.

The "solvate" in the present invention refers to an associated complex formed by one or more solvent molecules and the compounds of the present invention. Solvents that form the solvate comprise, but are not limited to, water, isopropanol, ethanol, methanol, dimethyl sulfoxide, ethyl acetate, acetic acid and aminoethanol. The term "hydrate" means that the solvent molecule is an associated complex formed by water.

The "ester" in the present invention refers to an in-vivo hydrolyzable ester formed by a compound containing hydroxyl or carboxyl. Such ester is, for example, a pharmaceutically acceptable ester hydrolyzed in bodies of humans or animals to produce a parent alcohol or acid. The compound of formula (I) of the present invention contains carboxyl, and may form the in-vivo hydrolyzable ester with appropriate groups, and such groups comprise, but are not limited to, alkyl, arylalkyl, and the like.

The "nitrogen oxide" in the present invention means that when the compound contains several amine functional groups, one or more nitrogen atoms may be oxidized to form an N⁻ oxide. Special examples of the N⁻ oxide are an N⁻ oxide of tertiary amine or an N⁻ oxide containing nitrogen atoms of nitrogen heterocycles. Corresponding amine may be treated with an oxidizing agent such as hydrogen peroxide or peracid (such as peroxycarboxylic acid) to form the N⁻ oxide (referring to Advanced Organic Chemistry, Wiley Interscience, 4th edition, Jerry March, pages). In particular, the N⁻ oxide may be prepared by the method of L. W. Deady (Syn. Comm. 1977, 7, 509-514), for example, an amine compound reacts with m-chloroperoxybenzoic acid (MCPBA) in an inert solvent such as dichloromethane.

"The compound of the present invention", "the compound described in the present invention", "the compound mentioned in the present invention", or similar expression as used in the present invention refers to the compound represented by any general structure described in the present invention. For example, the compound of the present invention may refer to the compound represented by formula (I) or formula (Ia) or formula (Ib) or formula (IIa) or formula (IIb) or formula (III) or formula (IV) of the present invention. The compound of the present invention further comprises a specific compound in any one embodiment.

The term "treatment" as used in the present invention for any disease or symptom refers to improving the disease or symptom (that is, slowing down or preventing or alleviating the development of the disease or at least one of clinical symptoms) in some embodiments. In other embodiments, the "treatment" refers to alleviating or improving at least one physical parameter, comprising physical parameters that may not be perceived by the patient. In other embodiments, the "treatment" refers to regulating the disease or symptom physically (such as stabilizing perceptible symptoms) or physiologically (such as stabilizing physical parameters) or both physically and physiologically. In other embodiments, the "treatment" refers to preventing or delaying the attack, occurrence or deterioration of the disease or symptom.

Any structural formula given in the present invention is also intended to indicate the forms of these compounds that are not isotopically enriched and are isotopically enriched. The compounds that are isotopically enriched have the structure described by the general formula given in the present invention, except that one or more atoms are substituted by atoms with a selected atomic weight or mass number. Exemplary isotopes that may introduced into the compounds of the present invention comprise isotopes of hydrogen, carbon, nitrogen, oxygen, phosphorus, sulfur, fluorine and chlorine, such as 2H, 3H, 11C, 13C, 14C, 15N, 170, 180, 18F, 31P, 32P, 35S, 36Cl and 125I.

In addition, the substitution of heavier isotopes, especially deuterium (that is, 2H or d), can provide some therapeutic advantages, and these advantages are brought about by higher metabolic stability, such as being brought about by the increase of half-life in vivo or the decrease of dosage requirement or the improvement of therapeutic index. It should be understood that the deuterium in the present invention is regarded as a substituent of the compound of formula (I). Concentrations of these heavier isotopes, especially deuterium, may be defined by an isotope enrichment factor. The term "isotope enrichment factor" used in the present invention refers to a ratio of an isotopic abundance to a natural abundance of a specified isotope. If the substituent of the compound of the present invention is designated as the deuterium, the compound has at least 3500 (52.5% deuterium doping at each designated deuterium atom), at least 4000 (60% deuterium doping), at least 4500 (67.5% deuterium doping), at least 5000 (75% deuterium doping), at least 5500 (82.5% deuterium doping), at least 6000 (90% deuterium doping), at least 6333.3 (95% deuterium doping), at least 6466.7 (97% deuterium doping), at least 6600 (99% deuterium doping) or at least 6633.3 (99.5% deuterium doping) isotope enrichment factor. The pharmaceutically acceptable solvates of the present invention comprise those in which a crystallization solvent may be isotopically substituted, such as D₂O, acetone-d₆ and DMSO-d₆.

Unless otherwise specified, all tautomerism forms of the compounds of the present invention are included in the scope of the present invention. In addition, unless otherwise specified, the structural formulas of the compounds described in the present invention comprise enriched isotopes of one or more different atoms.

Unless otherwise specified, abbreviations of any protective groups, amino acids and other compounds used in the present invention are subject to their commonly used and recognized abbreviations, or refer to IUPAC-IUB Commission on Biochemical Nomenclature (referring to Biochem. 1972, 11: 942-944).

### Description for compound of the present invention

The present invention provides a phenyl-substituted dihydronaphthyridine compound capable of competitively antagonizing an aldosterone receptor (MR) and a use of the compound or the pharmaceutical composition in preparation of a drug, wherein the drug is used for treating, preventing or alleviating diabetic nephropathy, aldosteronism hyperplasia, hypertension, heart failure (comprising chronic heart failure), sequelae of myocardial infarction, liver cirrhosis, renal failure, stroke and other diseases of patients.

In one aspect, the present invention relates to a compound, which is a compound represented by formula (I), or a stereoisomer, geometric isomer, tautomer, nitrogen oxide, hydrate, solvate, metabolite, ester, pharmaceutically acceptable salt or prodrug of the compound represented by formula (I);

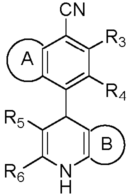

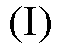

In one aspect, the present invention relates to a use of the compound or the pharmaceutical composition of the present invention in preparation of a drug, wherein the drug is used for treating, preventing or alleviating the following disease of a patient: diabetic nephropathy, aldosteronism hyperplasia, hypertension, heart failure (comprising chronic heart failure, and the like), sequelae of myocardial infarction, liver cirrhosis, renal failure or stroke.

In another aspect, the present invention further relates a use of the compound or the pharmaceutical composition of the present invention in preparation of a drug, wherein the drug is used as a mineralocorticoid receptor antagonist.

In one aspect, the compound or the pharmaceutical composition of the present invention is used for treating, preventing or alleviating the following disease of a patient: diabetic nephropathy, aldosteronism hyperplasia, hypertension, heart failure (comprising chronic heart failure, and the like), sequelae of myocardial infarction, liver cirrhosis, renal failure or stroke.

In another aspect, the compound or the pharmaceutical composition of the present invention may be used for antagonizing a mineralocorticoid receptor.

In one aspect, the present invention relates to a method of using the compound or the pharmaceutical composition of the present invention for treating, preventing or alleviating the following disease of a patient: diabetic nephropathy, aldosteronism hyperplasia, hypertension, heart failure (comprising chronic heart failure, and the like), sequelae of myocardial infarction, liver cirrhosis, renal failure or stroke, wherein the method comprises treating the patient with a therapeutically effective dose of the compound or the pharmaceutical composition of the present invention.

In another aspect, the present invention also relates to a method of using the compound or the pharmaceutical composition of the present invention for antagonizing a mineralocorticoid receptor, wherein the method comprises contacting an organism (in vivo or in vitro) with an effective dose of the compound or the pharmaceutical composition of the present invention.

The compound or the pharmaceutical composition of the present invention competitively antagonize the aldosterone receptors (MR), so that the compound and the pharmaceutical composition may be useful drugs for treating and preventing a disease related to increase of an aldosterone level.

The compound or the pharmaceutical composition of the present invention may be used for treating or preventing an aldosterone receptor-mediated disease. The present invention further comprises a method for treating or alleviating an aldosterone receptor-mediated disease of a patient, or sensitive to these diseases, wherein the method comprises treating the patient with a therapeutically effective dose of the compound or the pharmaceutical composition of the present invention.

The present invention comprises an application of the compound of the present invention and the pharmaceutically acceptable salt thereof to produce a pharmaceutical product for treating diseases related to the mineralocorticoid receptor or aldosterone of patients, comprising those diseases described in the present invention. The present invention comprises a pharmaceutical composition, wherein the pharmaceutical composition comprises a therapeutically effective dose required by combining the compound represented by formula (I) with at least one pharmaceutically acceptable carrier, excipient, diluent, adjuvant and intermedium.

Unless otherwise indicated, all hydrates, solvates and pharmaceutically acceptable salts of the compound of the present invention belong to the scope of the present invention.

Specifically, the salts are pharmaceutically acceptable salts. The term "pharmaceutically acceptable" means that the substance or composition must be suitable for chemistry or toxicology, and related to other components composing the preparation and mammals for treatment.

The salts of the compound of the present invention further comprise a salt used for preparing or purifying an intermediate of the compound represented by formula (I) or formula (Ia) or formula (Ib) or formula (IIa) or formula (IIb) or formula (III) or formula (IV), or an enantiomer separated from the compound represented by formula (I) or formula (Ia) or formula (Ib) or formula (IIa) or formula (IIb) or formula (III) or formula (IV).

The salts of the compound of the present invention may be prepared by any suitable method provided in the literature. For example, inorganic acids, such as hydrochloric acid, hydrobromic acid, sulfuric acid, nitric acid and phosphoric acid, are used. Alternatively, organic acids, such as acetic acid, maleic acid, succinic acid, mandelic acid, fumaric acid, malonic acid, pyruvic acid, oxalic acid, glycolic acid and salicylic acid; pyranose acids, such as glucuronic acid and galacturonic acid; α-hydroxy acids, such as citric acid and tartaric acid; amino acids, such as aspartic acid and glutamic acid; aromatic acids, such as benzoic acid and cinnamic acid; and sulfonic acids, such as p-toluenesulfonic acid and ethanesulfonic acid, are used.

The biological activity of the compound of the present invention may be evaluated by any conventionally known method. Suitable detection methods are well known in the art. For example, the MR antagonistic activity, pharmacokinetic activity and/or liver microsomal stability of the compound of the present invention may be detected by suitable conventional methods. The detection method provided by the present invention is only presented as an example and does not limit the present invention. It is detected that the compound of the present invention has the activity in at least one detection method provided by the present invention. For example, the compound of the present invention has good antagonistic activity to the aldosterone receptor, and has good in-vivo pharmacokinetic properties, such as good absorption and exposure, and high bioavailability. For another example, the compound of the present invention has low toxic and side effects.

### Administration and use of compound of the present invention

The therapeutically effective dose of the compound of the present invention should be present in the above pharmaceutical preparation at a concentration of about 0.1 to 99.5% by weight, preferably about 0.5 to 95% by weight, of the whole mixture. The therapeutically effective dose may first be estimated by various methods well known in the art. The initial dose for animal research may be based on an effective concentration established in cell culture assays. A dose range suitable for human individuals may be determined by, for example, data obtained from animal studies and cell culture assays. In some embodiments, the compound of the present invention may be prepared as a drug for oral administration. An exemplary dose of the compound of the present invention in the drug for oral administration ranges from about 0. 01 to about 100 mg/kg (wherein kg represents the body weight of the subject). In some embodiments, the drug comprises the dose ranging from about 0.01 to about 20 mg/kg (wherein kg represents the body weight of the subject), or optionally ranging from about 0. 01 to about 10 mg/kg (wherein kg represents the body weight of the subject), or optionally ranging from about 0.01 to about 5.0 mg/kg (wherein kg represents the body weight of the subject). In some embodiments, the compound of the present invention is administered by gastrointestinal tract, and the effective dose is about 0.001 to 1 mg/kg, and preferably about 0.01 to 0.5 mg/kg, by the body weight.

Generally, a dosage regimen for an orally administered drug is three times a week, twice a week, once a week, three times a day, twice a day or once a day. In some embodiments, the compound of the present invention is administered as an active ingredient in a total amount of about 0.001 to about 50 mg/kg, preferably 0.001 to 10 mg/kg, by the body weight every 24 hours, and in order to obtain desired results, a plurality of single doses may be optionally used for administration One single dose may preferably contain the compound of the present invention in an amount of about 0.001 to about 30 mg/kg, especially 0.001 to 3 mg/kg, by the body weight.

The effective amount or the therapeutically effective amount or dose of the drug (such as the compound of the present invention) refers to an amount of the drug or the compound that causes symptom improvement or prolonged survival of an individual. The toxicity and therapeutic efficacy of the molecule may be determined by standard medical procedures in cell cultures or experimental animals, such as determining LD50 (a dose causing 50% lethality of the population) and ED50 (a dose causing 50% effective treatment of the population). A dose ratio of a toxic effect to a therapeutic effect is a therapeutic index, which may be expressed as LD50/ED50. A drug with a high therapeutic index is preferably shown.

The effective amount or the therapeutically effective amount is an amount of a compound or a pharmaceutical composition that will trigger a biological or medical reaction of tissue, system, animal or human being sought by researchers, veterinarians, doctors or other clinicians. The dose is preferably within a range of circulating concentration of ED50 with minimal toxicity or no toxicity. The dose may vary within this range, which depends on the dosage form used and/or the administration route used. According to the methods known in the art, the correct preparation, administration route, dose and administration interval should be selected in consideration of the particularity of individual conditions.

The dose and interval time may be regulated individually to provide a plasma level of an active part sufficient to obtain the desired effect; that is, a minimum effective concentration (MEC). The MEC of each compound will be different, but may be estimated, for example, from in-vitro data and animal experiments. The dose necessary to obtain the MEC will depend on individual characteristics and the administration route. In the case of local administration or selective ingestion, an effective local concentration of the drug may be independent of a plasma concentration.

The amount of the drug or composition administered may depend on various factors, comprising the sex, age and weight of an individual to be treated, the illness severity, the administration mode and the judgment made by a prescriber.

When necessary, the composition of the present invention may be provided by a package or a dispensing device which contains one or more unit dosage forms (containing active ingredients). For example, the package or the device may comprise a metal or plastic foil (such as a foamed package) or a glass and rubber stopper. The package or the dispensing device may be accompanied by an instruction for medication. A composition containing the compound of the present invention prepared in a compatible pharmaceutical carrier may also be prepared, which is placed in an appropriate container, and added with a label for the treatment of a specified symptom.

The compound of the present invention has good safety, wherein a maximum tolerated dose of the compound 62 after repeated administration to mice for 14 days shows that the compound is highly safe. In particular, a maximum tolerated dose of the compound 36 after repeated administration for 14 days is more than 2.7 times that of the compound 62, especially a safety window of the dose is more than 230 times that of the equivalent dose, so that the compound has high safety.

In addition, the compound 36 of the present invention has a small inhibitory effect on CYP3A4, which can reduce the contraindications of Finerenone, and also has a lower AUC (tissue distribution) ratio of heart to kidney than Finerenone, which is significantly conductive to reducing the side effects of Finerenone.

The compound of the present invention is suitable for preventing and/or treating various related situations of diseases and symptoms, especially those characterized by an increase of plasma aldosterone concentration or a change of plasma aldosterone concentration relative to a plasma renin concentration, or those related to these changes. Examples that may be mentioned are: spontaneous primary aldosteronism hyperplasia, aldosteronism hyperplasia associated with adrenal hyperplasia, adrenal adenoma and/or adrenal carcinoma, aldosteronism hyperplasia associated with liver cirrhosis, aldosteronism hyperplasia associated with heart failure, (relative) aldosteronism hyperplasia associated with essential hypertension, and the like.

Due to the mechanism of action, the compound of the present invention is also suitable for preventing sudden cardiac death of patients with an increased risk of sudden cardiac death. These patients particularly refer to a patient suffering from one of the following diseases: primary and secondary hypertension, hypertensive heart disease with or without congestive heart failure, refractory hypertension, acute and chronic heart failure, coronary heart disease, stable and unstable angina pectoris, myocardial ischemia, myocardial infarction, dilated cardiomyopathy, congenital primary cardiomyopathy (such as Bmgada syndrome), cardiomyopathy caused by Chagas disease, shock, arteriosclerosis, atrial and ventricular arrhythmias, transient and ischemic attack, stroke, inflammatory cardiovascular disease, peripheral and cardiac vascular disease, peripheral blood flow disorder, arterial occlusive disease such as intermittent claudication, asymptomatic left ventricular dysfunction, myocarditis, cardiac hypertrophy change, pulmonary hypertension, coronary and peripheral arterial spasm, thrombosis, thromboembolic disease and vasculitis.

The compound of the present invention may additionally be used for preventing and/or treating edema formation, such as pulmonary edema, nephrogenic edema or floating lung associated with heart failure, and such as restenosis after thrombolytic therapy, percutaneous transluminal angioplasty (PTA) and percutaneous transluminal coronary angioplasty (PTCA), heart transplantation and bypass surgery.

The compound of the present invention is also suitable for being used as a potassium-sparing diuretic and being used for treating electrolyte disorders such as hypercalcemia, hypernatremia or hypokalemia.

The compound of the present invention is also suitable for being used for treating renal diseases such as acute and chronic renal failure, hypertensive nephropathy, arteriosclerosis nephritis (chronic and interstitial), nephrosclerosis, chronic renal failure and cystic nephropathy, being used for preventing renal injury (such as renal injury caused by an immunosuppressant related to organ transplantation (such as cyclosporine A)), and being used for treating renal cancer.

The compound of the present invention may be additionally used for preventing and/or treating diabetes and diabetic sequelae, such as neuropathy and nephropathy.

The compound of the present invention may be further used for preventing and/or treating microalbuminuria, such as being caused by diabetes or hypertension, and proteinuria.

The compound of the present invention is also suitable for being used for preventing and/or treating diseases associated with an increase of plasma glucocorticoid concentration or a local increase of glucocorticoid concentration in tissues (such as the heart). Examples that may be mentioned are: adrenal dysfunction (Cushing's syndrome) leading to excessive production of glucocorticoid, adrenal cortical tumor leading to excessive production of glucocorticoid, and pituitary adenoma which spontaneously produces ACTH (adrenocorticotropic hormone) so as to lead to adrenal hyperplasia and Cushing's disease.

The compound of the present invention may be additionally used for preventing and/or treating obesity, metabolic syndrome and obstructive sleep apnea.

The compound of the present invention may be further used for preventing and/or treating inflammatory diseases caused by viruses, spirochetes, fungi, bacteria or mycobacteria, and agnogenic inflammatory diseases, such as polyarthritis, lupus erythematosus, periarthritis or polyarthritis, dermatomyositis, scleroderma and sarcoidosis.

The compound of the present invention may be further used for treating central nervous system disorders such as depression, anxiety and chronic pain, especially migraine, and neurodegenerative diseases such as Alzheimer's disease and Parkinson's syndrome.

The compound of the present invention is also suitable for being used for preventing and/or treating vascular injury, such as vascular injury after percutaneous transluminal coronary angioplasty (PTCA), stent implantation, coronary artery angioscopy, and re-occlusion or restenosis after bypass surgery, and endothelial dysfunction, Raynaud's disease, thromboangiitis obliterans (Buerger's syndrome) and tinnitus syndrome.

The compound of the present invention may be used alone or in combination with other active ingredients if necessary. The present invention further relates to a combined use of at least one compound of the present invention and a drug of one or more other active ingredients (especially for treating and/or preventing the above-mentioned diseases), especially a drug for treating and/or preventing the diseases in the present invention. Suitable active ingredients for composition comprise, but are not limited to: active ingredients for lowering blood pressure, such as and preferably selected from a calcium antagonist, an angiotensin II receptor antagonist, an ACE inhibitor, an endothelin antagonist, a renin inhibitor, an α-receptor blocker, a β-receptor blocker and an Rho kinase inhibitor; diuretics, especially a loop diuretic, and thiazide diuretics; agents having an antithrombotic effect, such as and preferably selected from a platelet aggregation inhibitor, an anticoagulant, or a fibrinolytic promoter; active ingredients for changing lipid metabolism, such as and preferably selected from a thyroid receptor agonist, a cholesterol synthesis inhibitor such as and preferably an HMG- CoA reductase inhibitor or squalene synthesis inhibitor, an ACAT inhibitor, a CETP inhibitor, a bile acid reuptake inhibitor and a lipoprotein (a) antagonist; organic nitrates and NO donors, such as sodium nitroprusside, nitroglycerin, isosorbide mononitrate, isosorbide dinitrate, molsidomine or SIN-1, and inhaled NO; compounds with a positive inotropic effect, such as cardiac glycoside (digoxin), isoproterenol, epinephrine, norepinephrine, dopamine and dobutamine; compounds inhibiting the decomposition of cyclic guanosine monophosphate (cGMP) and/or cyclic adenosine monophosphate (cAMP), such as phosphodiesterase (PDE) 1, 2, 3, 4 and/or 5 inhibitor (such as sildenafil, vardenafil, tadanafil, amrinone and milrinone); natriuretic peptides, such as atrial natriuretic polypeptide, B-type natriuretic peptide or brain natriuretic peptide, C-type natriuretic peptide (CNP) and urodilatin; calcium sensitizers, such as and preferably Levosimendan; NO-independent but heme-dependent guanylate cyclase stimulators, especially the compounds described in WO00/06568, WO00/06569, WO02/42301 and WO03/095451 (such as Riociguat); NO- and heme-independent guanylate cyclase activators, especially the compounds described in WO 01/19355, WO 01/19776, WO 01/19778, WO 02/070462 and WO 02/070510; human neutrophil elastase (HNE) inhibitors, such as sivelestone or DX-890 (Reltran); compounds inhibiting signal transduction cascade, such as a tyrosine kinase inhibitor, especially sorafenib, imatinib, gefitinib and erlotinib; and/or compounds affecting cardiac energy metabolism, such as etomoxir, dichloroacetate, ranolazine or trimetazidine.

The compound of the present invention may also be administered in combination with other active ingredients other than the above-mentioned active ingredients. For example, in preferred embodiments of the present invention, the compound of the present invention is administered in combination with diuretics such as furosemide, bumetanide, torasemide, benfluthiazide, chlorothiazide, dihydrochlorothiazide, hydrofluomethiazide, methyclothiazide, polithiazide, trichlorothiazide, chlorthalidone, indapamide, metolazone, quinethazone, acetazolamide, dichlorophenamide, methazolamide, glycerol, isosorbide, mannitol, amiloride or triamterene.

The present invention has the beneficial effects as follows:

A new compound is provided, which may be used as a mineralocorticoid receptor inhibitor, has a good median inhibition concentration, and can effectively treat and prevent diabetic nephropathy, hypertension, heart failure, other cardiovascular diseases and other aldosterone-related diseases.

### BRIEF DESCRIPTION OF THE DRAWINGS

The present invention is further described in detail hereinafter with reference to the drawings and specific embodiments, and the advantages of the above and/or other aspects of the present invention will be clearer.
FIG. 1 is an asymmetric unit diagram of the crystal structure of the compound 36.
FIG. 2 is a diagram of a single unit cell of the crystal structure of the compound 36.
FIG. 3 is a filing diagram of the crystal structure of the compound 36.
FIG. 4 is a calculation and experimental comparison diagram of the crystal XRPD diagram of the compound 36.
FIG. 5 is an asymmetric unit diagram of the crystal structure of the compound 62.
FIG. 6 is a diagram of a single unit cell of the crystal structure of the compound 62.
FIG. 7 is a filing diagram of the crystal structure of the compound 62.
FIG. 8 is a calculation and experimental comparison diagram of the crystal XRPD diagram of the compound 62.
FIG. 9 is a photomicrograph of a single crystal of the compound 36.
FIG. 10 is a photomicrograph of a single crystal of the compound 62.

### DETAILED DESCRIPTION

In the specification, if there is any difference between the chemical name and the chemical structure, the structure is dominant.

Generally, the compounds of the present invention may be prepared by the method described in the present invention, and unless otherwise specified, the definition of the substituent is represented by formula (I). The following reaction solutions and embodiments are used to further illustrate the contents of the present invention.

Those skilled in the art will realize that the chemical reaction described in the present invention may be used to prepare other compounds of the present invention appropriately, and other methods for preparing the compounds of the present invention are considered to be within the scope of the present invention. For example, the synthesis of those non-exemplary compounds according to the present invention may be successfully completed by those skilled in the art through modification methods, such as appropriately protecting interfering groups, using other known reagents (other than those described in the present invention), or making some conventional modifications to the reaction conditions. In addition, the reaction disclosed in the present invention or the known reaction conditions are also generally applicable to the preparation of other compounds of the present invention.

In the embodiments described below, unless otherwise specified, all temperatures are set as degrees Celsius. Unless otherwise specified in other aspects, the reagents are purchased from commodity suppliers such as Aldrich Chemical Company, Arco Chemical Company and Alfa Chemical Company, and are not further purified when used; and the general reagents are purchased from Shantou Xilong Chemical Factory, Guangdong GHTECH Chemical Reagent Factory, Guangzhou Chemical Reagent Factory, Tianjin Haoyuyu Chemical Co., Ltd., Qingdao Tenglong Chemical Reagent Co., Ltd. and Qingdao Ocean Chemical Factory.

Anhydrous tetrahydrofuran, dioxane, toluene and ether are obtained by reflux drying of metallic sodium. Anhydrous dichloromethane and chloroform are obtained by reflux drying of calcium hydride. Ethyl acetate, petroleum ether, n-hexane, N,N-dimethylacetamide and N,N-dimethylformamide are dried by anhydrous sodium sulfate in advance for use.

Generally, the following reactions are carried out under a positive pressure of nitrogen or argon or by sleeving a drying tube on an anhydrous solvent (unless otherwise specified), reaction bottles are all plugged with suitable rubber stoppers, and a substrate is injected through a syringe. Glass wares are all dried.

Chromatographic columns are silica gel columns. Silica gel (300 meshes to 400 meshes) is purchased from Qingdao Ocean Chemical Factory. Nuclear magnetic resonance spectrum data are determined by a Bruker Avance 400 nuclear magnetic resonance spectrometer or a Bruker Avance III HD 600 nuclear magnetic resonance spectrometer, CDCl3, DMSO-d6, CD3OD or Acetone-d6 is a solvent (reported in ppm), and TMS (0 ppm) or chloroform (7.25 ppm) is used as a reference standard. When multiple peaks appear, the following abbreviations will be used: s (singlet), d (doublet), t (triplet), m (multiplet), q (quartet), br (broadened), dd (doublet of doublets), dt (doublet of triplets), dq (doublet of quartets), ddd (doublet of doublet of doublets),ddt (doublet of doublet of triplets), and dddd (doublet of doublet of doublet of doublets). A coupling constant is expressed by hertz (Hz).

Low-resolution mass spectrometry (MS) data are determined by an Agilent 6320 series LC-MS spectrometer equipped with a G1312A binary pump and a G1316A TCC (with a column temperature kept at 30°C), a G1329A automatic sampler and a G1315B DAD detector are used for analysis, and an ESI source is used in the LC-MS spectrometer.

Low-resolution mass spectrometry (MS) data are determined by an Agilent 6120 series LC-MS spectrometer equipped with a G1311A quaternary pump and a G1316A TCC (with a column temperature kept at 30°C), a G1329A automatic sampler and a G1315D DAD detector are used for analysis, and an ESI source is used in the LC-MS spectrometer.

The above two spectrometers are both equipped with an Agilent Zorbax SB-C18 column, with specifications of 2.1×30 mm and 5 µm. An injection volume is determined through a sample concentration; the flow rate is 0.6 mL/min; and the peak value of HPLC is recorded and read through UV-Vis wavelengths at 210 nm and 254 nm. The mobile phase is 0.1% formic acid acetonitrile solution (phase A) and 0.1% formic acid ultrapure water solution (phase B). Gradient elution conditions are as shown in Table 1:

**Table 1 Gradient elution conditions of mobile phase in low resolution mass spectrometry**

| Time (min) | A (CH₃CN, 0.1% HCOOH) | B (H₂O, 0.1% HCOOH) |
|---|---|---|
| 0-3 | 5-100 | 95-0 |
| 3-6 | 100 | 0 |
| 6-6.1 | 100-5 | 0-95 |
| 6.1-8 | 5 | 95 |

The following abbreviations are used throughout the present invention:
DMSO-d₆: deuterated dimethyl sulfoxide; g: gram; mg: milligram; mol: mole; mmol: millimole; mL: milligram; and µL: microliter.

The following reaction solutions described the steps of preparing the compounds disclosed in the present invention. Unless otherwise specified, R₁, R₂, R₃, R₄, R₆, R₇, R₈ and R₉ all have the meanings as described in the present invention. Unless otherwise specified, various reaction steps in various reaction solutions of the present invention are all implemented in solvents inert to the reactions, and the solvents inert to the reactions comprising, but are not limited to, solvents involved in the embodiments of the present invention or their substitutes.

Preparation methods of 4-(4-cyano-2-methoxyphenyl)-5-ethoxy-2,8-dimethyl-1,4-dihydro-1,6-naphthyridine-3-carboxylic acid in the following embodiments are as follows.

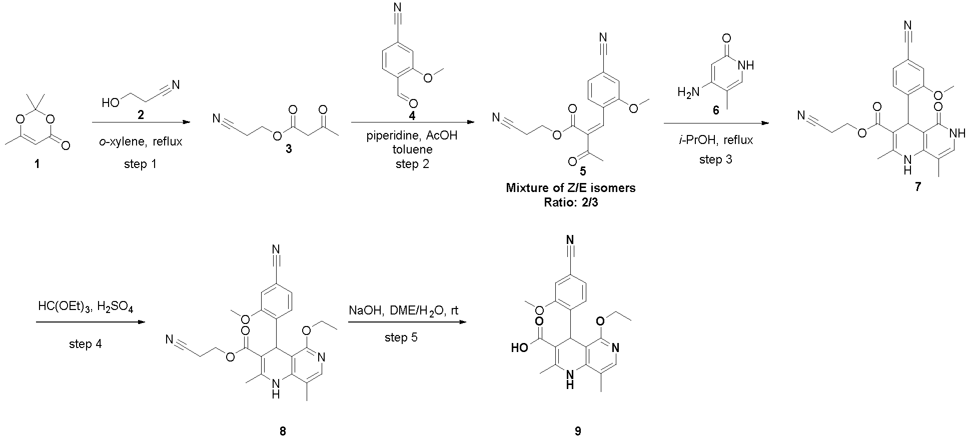

### Example 1 4-(5-ethoxy-2,8-dimethyl-3-oxazol-2-yl)-1,4-dihydro-1,6-naphthyridine-4-yl)-3-methoxybenzonit rile

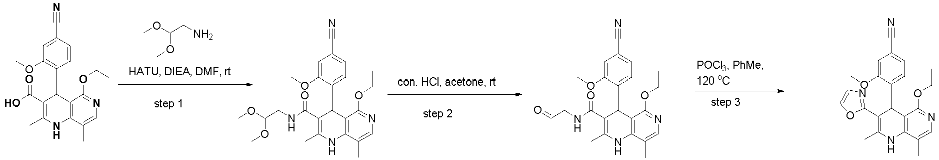

### Step 1) 4-(4-cyano-2-methoxyphenyl)-N-(2,2-dimethoxyethyl)-5-ethoxy-2,8-dimethyl-1,4-dihydro-1,6-naphth yridine-3-formamide

4-(4-cyano-2-methoxyphenyl)-5-ethoxy-2,8-dimethyl-1,4-dihydro-1,6-naphthyridine-3-carboxylic acid (150 mg, 0.395 mmol), 2-(7-azobenzotriazole)-*N,N,N',N'*-tetramethylurea hexafluorophosphate (180 mg, 0.474 mmol), *N*,*N*-diisopropylethylamine (102 mg, 0.790 mmol) and *N*,*N*-dimethylformamide (1.5 mL) were added into an 8 mL bottle at room temperature to react at room temperature for 2 hours, added with aminoacetaldehyde dimethyl acetal (83 mg, 0.790 mmol) to react at room temperature overnight, added with water (15 mL) for quenching, and then extracted with ethyl acetate (10 mL × 3 times). Organic phases were combined, washed with a saturated saline solution (10 mL), dried with anhydrous sodium sulfate, filtered, and then concentrated under reduced pressure. A residue obtained was purified by silica gel column chromatography (eluent: dichloromethane/methanol (v/v) = 10/1) to obtain a pale yellow solid (86 mg, yield 47.0%).
MS (ESI) M/Z: 467.6 [M+H]⁺

### Step 2) 4-(4-cyano-2-methoxyphenyl)-5-ethoxy-2,8-dimethyl-N-(2-oxoethyl)-1,4-dihydro-1,6-naphthyridine-3 -formamide

4-(4-cyano-2-methoxyphenyl)-*N*-(2,2-dimethoxyethyl)-5-ethoxy-2,8-dimethyl-1,4-dihydro-1,6-naphth yridine-3-formamide (65 mg, 0.139 mmol), acetone (6 mL) and 12.0 M 36% concentrated hydrochloric acid solution (42 µL) were added into a 25 mL single-mouth bottle at room temperature to react at room temperature for 1 hour, added with saturated sodium bicarbonate aqueous solution under ice bath to adjust a pH value to be 7, and then added with ethyl acetate (10 mL × 3 times) for extraction. Organic phases were combined, washed with a saturated saline solution (10 mL), dried with anhydrous sodium sulfate, filtered, and then concentrated under reduced pressure. A residue obtained was purified by silica gel column chromatography (eluent: dichloromethane/methanol (v/v) = 10/1) to obtain a pale yellow solid (25 mg, yield 42.7%).
MS (ESI) M/Z: 421.5 [M+H]⁺

### Step 3) 4-(5-ethoxy-2,8-dimethyl-3-oxazol-2-yl)-1,4-dihydro-1,6-naphthyridine-4-yl)-3-methoxybenzonitrile

4-(4-cyano-2-methoxyphenyl)-5-ethoxy-2,8-dimethyl-N-(2-oxoethyl)-1,4-dihydro-1,6-naphthyridine-3 -formamide (20 mg, 48.0 µmol), toluene (0.5 mL) and phosphorus oxychloride (40.0 µL) were added into an 8 mL bottle at room temperature, heated to 120°C to react for 1 hour, cooled to room temperature, added with water (2 mL), and then added with ethyl acetate (2 mL × 3 times) for extraction. Organic phases were combined, washed with a saturated saline solution (2 mL), dried with anhydrous sodium sulfate, filtered, and then concentrated under reduced pressure. A residue obtained was purified by preparative high-performance liquid chromatography (under the following purification conditions: chromatographic column: XBridge Prep C18 OBD 30 mm * 150 mm, 5 µm; mobile phase: water (containing 0.1% formic acid) and acetonitrile; flow rate: 60 mL/min; gradient: increase of acetonitrile from 37% to 52% within 7 minutes; and detection wavelength: 254/220 nm) to obtain a white solid (1.7 mg, yield 8.8%).
MS (ESI) M/Z: 403.50 [M+H]⁺

¹H NMR (400 MHz, Chloroform-*d*) δ 7.72 (s, 1H), 7.50 (s, 1H), 7.42 (d, *J* = 7.8 Hz, 1H), 7.21 (d, *J* = 7.8, 1H), 7.05 (s, 1H), 6.99 (s, 1H), 5.80 (s, 1H), 5.70 (s, 1H), 4.23-4.09 (m, 2H), 3.78 (s, 3H), 2.46 (s, 3H), 2.17 (s, 3H), 1.28-1.18 (m, 3H).

### Example 2 4-(5-ethoxy-2,8-dimethyl-3-(1,3,4-oxadiazole-2-yl)-1,4-dihydro-1,6-naphthyridine-4-yl)-3-methox ybenzonitrile

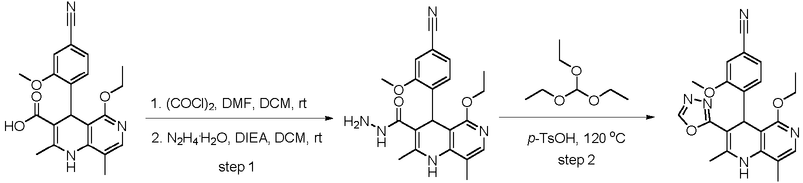

### Step 1) 4-(4-cyano-2-methoxyphenyl)-5-ethoxy-2,8-dimethyl-1,4-dihydro-1,6-naphthyridine-3-carbohydrazid e

4-(4-cyano-2-methoxyphenyl)-5-ethoxy-2,8-dimethyl-1,4-dihydro-1,6-naphthyridine-3-carboxylic acid (28 mg, 0.0740 mmol), dichloromethane (0.2 mL) and *N*,*N*-dimethylformamide (0.05 mL) were added into an 8 mL bottle at room temperature, and then dropwise added with oxalyl chloride (0.1 mL) under ice water bath after nitrogen replacement to react at room temperature for 30 minutes after addition. A dichloromethane (0.5 mL) solution of hydrazine hydrate (2 mL) and *N*,*N*-diisopropylethylamine (0.1 mL) was added into the reaction solution at room temperature, and stirred for 1 hour. Water (10 mL) was added into the reaction solution, and then extracted with ethyl acetate (10 mL × 2). Organic phases were combined, washed with a saturated saline solution (10 mL), dried with anhydrous sodium sulfate, sucked and filtered, concentrated under reduced pressure to obtain a dark yellow oil substance (50 mg, crude product), which was directly used in the reaction of the next step.

### Step 2) 4-[5-ethoxy-2,8-dimethyl-3-(1,3,4-oxadiazole-2-yl)-1,4-dihydro-1,6-naphthyridine-4-yl]-3-methoxyben zonitrile

4-(4-cyano-2-methoxyphenyl)-5-ethoxy-2,8-dimethyl-1,4-dihydro-1,6-naphthyridine-3-carbohydrazid e (35 mg, 0.0890 mmol), triethyl orthoformate (2 mL) and p-toluenesulfonic acid (40 mg, 0.232 mmol) were sequentially added into an 8 mL bottle at room temperature, stirred at 120°C for 1 hour after nitrogen replacement, cooled to room temperature, and then concentrated under reduced pressure. A residue was purified by a preparative chromatoplate (petroleum ether/ethyl acetate (v/v) = 2/1) to obtain a white solid (2.5 mg, 6.92%).
MS (ESI) M/Z: 403.95 [M+H]⁺

¹H NMR (400 MHz, Chloroform-*d*) δ 8.24 (s, 1H), 7.71 (s, 1H), 7.42 (d, *J* = 7.8 Hz, 1H), 7.13 (d, *J* = 7.8 Hz, 1H), 7.01 (s, 1H), 6.07 (s, 1H), 5.64 (s, 1H), 4.28-4.12 (m, 2H), 3.77 (s, 3H), 2.51 (s, 3H), 2.19 (s, 3H), 1.23 (t, *J* = 6.8 Hz, 3H).

### Example 3 4-(5-ethoxy-2,8-dimethyl-3-(5-methyl-1,3,4-oxadiazole-2-yl)-1,4-dihydro-1,6-naphthyridine-4-yl)-3-methoxybenzonitrile

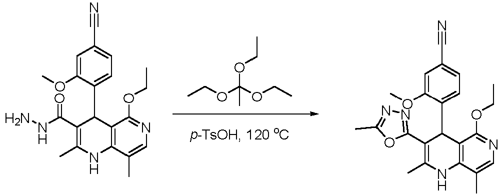

4-(4-cyano-2-methoxyphenyl)-5-ethoxy-2,8-dimethyl-1,4-dihydro-1,6-naphthyridine-3-carbohydrazid e (20 mg, 0.0510 mmol), triethyl orthoacetate (2 mL) and p-toluenesulfonic acid (30 mg, 0.174 mmol) were sequentially added into an 8 mL bottle at room temperature, stirred at 120°C for 1 hour after nitrogen replacement, cooled to room temperature, and then concentrated under reduced pressure. A residue obtained was purified by a preparative chromatoplate (petroleum ether/ethyl acetate (v/v) = 2: 1) to obtain a white solid (1.9 mg, 8.93%).
MS (ESI) M/Z: 418.45 [M+H]⁺

¹H NMR (300 MHz, Chloroform-*d*) δ7.71 (s, 1H), 7.39 (d, *J* = 7.5 Hz, 1H), 7.12 (dd, *J₁* = 7.8 Hz, *J₂* = 1.5 Hz, 1H), 7.00 (d, *J* = 1.5 Hz, 1H), 6.05 (s, 1H), 5.62 (s, 1H), 4.28 - 4.16 (m, 2H), 3.79 (s, 3H), 2.47 (s, 3H), 2.45 (s, 3H), 2.18 (s, 3H), 1.22 (t, *J* = 9.6 Hz, 3H).

### Example 4

### 4-(4-cyano-2-methoxyphenyl)-5-ethoxy-2-methyl-4,7,8,9-tetrahydrocyclopentane-1H-cyclopenta mide

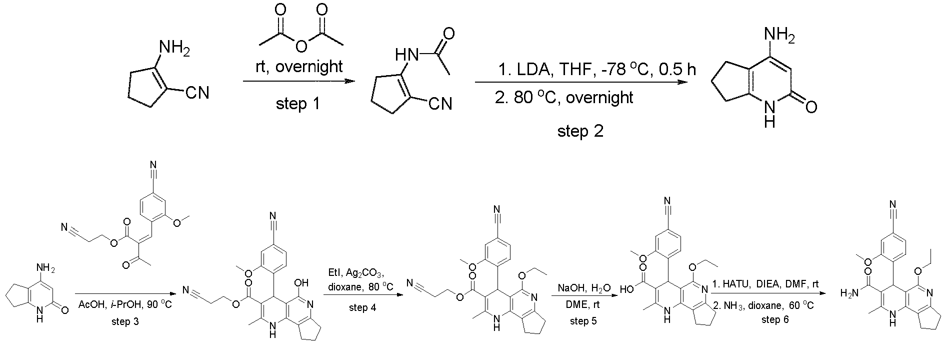

### Step 1) N-(2-cyanocyclopent-1-ene-1-yl)acetamide

1-amino-2-cyano-1-cyclopentene (5.00 g, 46.2 mmol) and acetic anhydride (32 mL) were added into a 100 mL single-mouth bottle at room temperature to react at room temperature overnight, and then concentrated under reduced pressure. A residue was pulped with petroleum ether (10 mL × 3), and then sucked and filtered to obtain a white needle-like crystal (4.80 g, yield 69.1%).
MS (ESI) M/Z: 151.10 [M+H]⁺

### Step 2) 4-amino-1,5,6,7-tetrahydro-2H-cyclopentylpyridine-2-one

*N*-(2-cyanocyclopent-1-ene-1-yl)acetamide (2.40 g, 14.4 mmol) and tetrahydrofuran (50 mL) were added into a 250 mL three-mouth bottle at room temperature, slowly dropwise added with lithium diisopropylamide (12 mL, 88.6 mmol) at -78°C after nitrogen replacement to react at -78°C for a half hour, then heated to 80°C to react overnight, cooled to room temperature, added with saturated ammonium chloride aqueous solution (30 mL) for quenching, and then extracted with ethyl acetate (30 mL × 3). Organic phases were combined, washed with a saturated saline solution (30 mL), dried with anhydrous sodium sulfate, and then sucked and filtered. The filtrate was concentrated under reduced pressure. A residue was purified by silica gel column chromatography (dichloromethane/methanol (v/v) = 5/1) to obtain a pale yellow solid (1.25 g, yield 52.1%).
MS (ESI) M/Z: 151.10 [M+H]⁺

### Step 3) 2-cyanoethyl 4-(4-cyano-2-methoxyphenyl)-5-hydroxy-2-methyl-1H,4H,7H,8H,9H-cyclopentane[h]1,6-naphthyridi ne-3-carboxylate

4-amino-1,5,6,7-tetrahydro-2*H*-cyclopentylpyridine-2-one (1.00 g, 6.66 mmol), 2-cyanoethyl-2-[(4-cyano-2-methoxyphenyl)methylene]-3-oxobutyrate (2.18 g, 7.33 mmol), isopropanol (20 mL) and acetic acid (19 µL) were added into a 50 mL single-mouth bottle at room temperature to react at 90°C overnight after nitrogen replacement, cooled to room temperature, and then sucked and filtered. A solid obtained was pulped with methyl tert-butyl ether (5 mL × 3), and then sucked and filtered to obtain a yellow solid (1.70 g, 60.4%).

### Step 4) 2-cyanoethyl 4-(4-cyano-2-methoxyphenyl)-5-ethoxy-2-methyl-1H,4H,7H,8H,9H-cyclopentane[h]1,6-naphthyridin e-3-carboxylate

2-cyanoethyl 4-(4-cyano-2-methoxyphenyl)-5-hydroxy-2-methyl-1*H*,4*H*,7*H*,8*H*,9*H*-cyclopentane[*h*]1,6-naphthyridine-3-carboxylate (1.70 g, 4.02 mmol), methyl iodide (940 mg, 6.03 mmol), silver carbonate (1.11 g, 4.02 mmol) and 1,4-dioxane (15 mL) were added into a 50 mL single-mouth bottle at room temperature to react at 80°C for 1 hour after nitrogen replacement, cooled to room temperature, added with water (50 mL) for quenching, and then extracted with ethyl acetate (50 mL × 3 times). Organic phases were combined, washed with a saturated saline solution (50 mL), dried with anhydrous sodium sulfate, sucked and filtered, and then concentrated under reduced pressure. A residue obtained was purified by preparative high-performance liquid chromatography under the following purification conditions: chromatographic column: C18 silica gel column; mobile phase A: water (containing 0.1% formic acid) and mobile phase B: acetonitrile; flow rate: 60 mL/min; gradient: increase of acetonitrile from 30% to 50% within 15 minutes; and detection wavelength: 254 nm, to obtain a yellow solid (460 mg, yield 25.0%).

### Step 5) 4-(4-cyano-2-methoxyphenyl)-5-ethoxy-2-methyl-4,7,8,9-tetrahydrocyclopentane[h][1,6]naphthyridin e-3-carboxylic acid

2-cyanoethyl 4-(4-cyano-2-methoxyphenyl)-5-ethoxy-2-methyl-1*H*,4*H*,7*H*,8*H*,9*H*-cyclopentane [*h*]1,6-naphthyridine-3-carboxylate (440 mg, 0.960 mmol), ethylene glycol dimethyl ether (4.5 mL) and an aqueous solution (1.5 mL) of sodium hydroxide (77 mg, 1.92 mmol) were added into a 50 mL single-mouth bottle at room temperature to react at room temperature for 1 hour, added with a hydrochloric acid solution (1.0 mol/L) under ice bath to quench and adjust a PH value to be 5, and then extracted with ethyl acetate (15 mL × 3 times). Organic phases were combined, washed with a saturated saline solution (15 mL × 3 times), dried with anhydrous sodium sulfate, sucked and filtered, and then concentrated under reduced pressure. A residue obtained was purified by preparative high-performance liquid chromatography under the following purification conditions: chromatographic column: C18 silica gel column; mobile phase A: water (containing 0.04% ammonia water) and mobile phase B: acetonitrile; flow rate: 60 mL/min; gradient: increase of acetonitrile from 15% to 40% within 20 minutes; and detection wavelength: 254 nm. The product was collected, and freeze-dried under reduced pressure to obtain a pale yellow solid (40 mg, yield 10.3%).

### Step 6) 4-(4-cyano-2-methoxyphenyl)-5-hydroxy-2-methyl-1H,4H,7H,8H,9H-cyclopentane[h]1,6-naphthyridi ne-3-carbonitrile

4-(4-cyano-2-methoxyphenyl)-5-ethoxy-2-methyl-4,7,8,9-tetrahydrocyclopentane[*h*][1,6]naphthyridin e-3-carboxylic acid (40 mg, 0.100 mmol), *N*,*N*-diisopropylethylamine (38 mg, 0.297 mmol), *N*,*N*-dimethylformamide (1.5 mL) and 2-(7-azobenzotriazole)-*N,N,N',N'*-tetramethylurea hexafluorophosphate (19 mg, 0.119 mmol) were added into an 8 mL bottle at room temperature to react at room temperature for 1 hour. The reaction solution was added with a 1,4-dioxane (1.0 M, 0.5 mL) solution of ammonia at room temperature to react at 60°C for 3 hours, cooled to room temperature, added with water (15 mL) for quenching, and then extracted with ethyl acetate (15 mL × 3 times). Organic phases were combined, washed with a saturated saline solution (15 mL × 3 times), dried with anhydrous sodium sulfate, sucked and filtered, and then concentrated under reduced pressure. A residue obtained was purified by preparative high-performance liquid chromatography under the following purification conditions: chromatographic column: Sunfire prep C18; mobile phase A: water (containing 0.1% formic acid) and mobile phase B: acetonitrile; flow rate: 60 mL/min; gradient: increase of acetonitrile from 20% to 42% within 7 minutes; and detection wavelength: 254 nm. The product was collected, and freeze-dried under reduced pressure to obtain a white solid (9.0 mg, yield 22.6%).
MS (ESI) M/Z: 405.45 [M+H]⁺

¹H NMR (400 MHz, DMSO-*d6*) δ8.05 (s, 1H), 7.36 (s, 1H),7.28 (d, *J* = 7.6 Hz, 1H), 7.15 (d, *J* = 7.6 Hz, 1H), 6.65 (s, 1H), 5.33 (s, 1H), 4.03 - 3.97 (m, 2H), 3.83 (s, 3H), 2.76 (t, *J* = 7.2 Hz, 1H), 2.68 (t, *J* = 7.2 Hz, 1H), 2.17 (s, 3H), 2.08 - 1.90 (m, 2H), 1.04 (t, *J* = 7.2 Hz, 3H).

### Example 5 4-(4-cyano-2-methoxyphenyl)-2-methyl-4,7,8,9-tetrahydrocyclopentane-3-carbonitrile

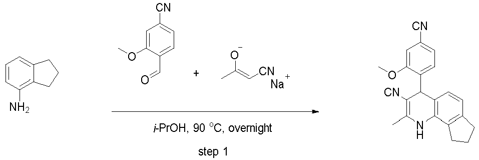

2,3-dihydro-4-indanamine (12 mg, 0.0890 mmol), 4-cyano-2-methoxybenzaldehyde (24 mg, 0.150 mmol), 1-cyanopropene-2-sodium valerate (16 mg, 0.150 mmol) and an isopropanol (0.5 mL) solution of glacial acetic acid (9 µL) were added into an 8 mL bottle at room temperature, heated to 90°C to react overnight after nitrogen replacement, cooled to room temperature, and then concentrated under reduced pressure. A residue obtained was purified by C18 reversed-phase column chromatography under the following conditions (C18 BIOTAGE 40 g reversed-phase column, mobile phase A (0.1% formic acid aqueous solution) and mobile phase B (acetonitrile), increase from 15% B to 50% B for 15 minutes, and detection wavelength 254 nm) to obtain a pale yellow solid (2.3 mg, yield 4.5%).
MS(ESI) M/: 340.25 [M-H]⁻

¹H NMR (400 MHz, Chloroform-*d*) δ7.18 - 7.16 (m, 1H), 7.14 - 7.09 (m, 2H), 6.78 (s, 3H), 5.90 (s, 1H), 5.49 (s, 1H), 3.93 (s, 3H), 2.90 - 2.75 (m, 4H), 2.24 (s, 3H), 2.19 - 2.12 (m, 2H).

### Example 6 4-(5-cyanored-8-yl)-5-ethoxy-2,8-dimethyl-1,4-dihydro-1,6-naphthyridine-3-formamide

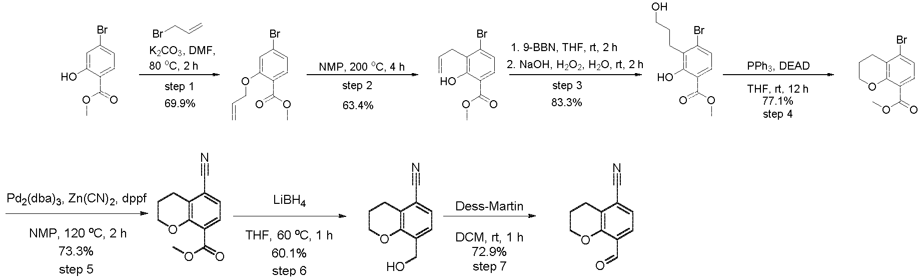

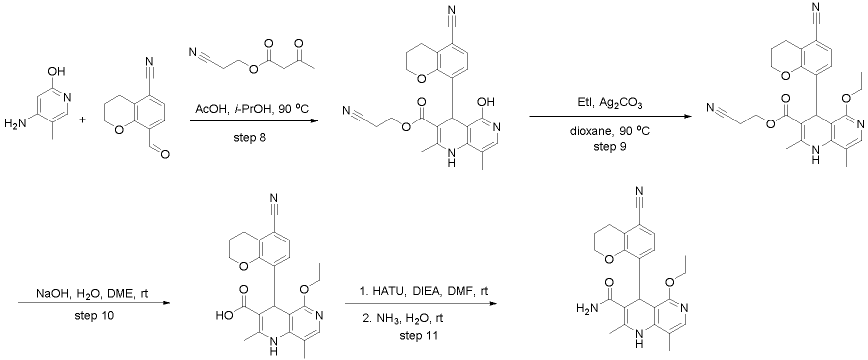

### Step 1) Methyl 2-allyloxy-4-bromobenzoate

Methyl 4-bromo-2-hydroxybenzoate (5.00 g, 21.6 mmol), potassium carbonate (5.98 g, 43.3 mmol), N,N-dimethylformamide (50 mL) and 3-bromopropene (3.75 g, 43.3 mmol) were added into a 250 ml single-mouth bottle at room temperature, heated to 80°C to react for 2 hours, cooled to room temperature, and then added with water (300 mL) for quenching, and extracted with ethyl acetate (500 mL × 3). Organic phases were combined, washed with a saturated saline solution (500 mL), dried with anhydrous sodium sulfate, sucked and filtered, and then concentrated under reduced pressure. A residue obtained was purified by silica gel column chromatography (petroleum ether/ethyl acetate (v/v) = 5: 1) to obtain a yellow solid (4.32 g, yield 69.9%).
LCMS (ESI, m/z): 271.0 [M+H]⁺

### Step 2) Methyl 3-allyl-4-bromo-2-hydroxybenzoate

Methyl 2-allyloxy-4-bromobenzoate (4.19 g, 15.5 mmol) and N-methylpyrrolidone (30 mL) were added into a 250 mL single-mouth bottle at room temperature, heated to 200°C to react for 4 hours, cooled to room temperature, and then added with water (300 mL) for quenching, and extracted with ethyl acetate (500 mL × 3). Organic phases were combined, washed with a saturated saline solution (500 mL), dried with anhydrous sodium sulfate, sucked and filtered, and then concentrated under reduced pressure. A residue obtained was purified by silica gel column chromatography (petroleum ether/ethyl acetate (v/v) = 4: 1) to obtain a colorless oil substance (2.66 g, yield 63.4%).
LCMS (ESI, m/z): 271.1 [M+H]⁺

### Step 3) Methyl 4-bromo-2-hydroxy-3-(3-hydroxypropyl)benzoate

Methyl 3-allyl-4-bromo-2-hydroxybenzoate (4.19 g, 15.5 mmol) and *N*-methylpyrrolidone (30 mL) were added into a 250 mL single-mouth bottle at room temperature, heated to 200°C to react for 4 hours, cooled to room temperature, and then added with water (300 mL) for quenching, and extracted with ethyl acetate (500 mL × 3). Organic phases were combined, washed with a saturated saline solution (500 mL), dried with anhydrous sodium sulfate, sucked and filtered, and then concentrated under reduced pressure. A residue obtained was purified by silica gel column chromatography (petroleum ether/ethyl acetate (v/v) = 4: 1) to obtain a colorless oil substance (2.31 g, yield 83.3%).
LCMS (ESI, m/z): 289.1 [M+H]⁺

### Step 4) Methyl 5-bromochrome-8-formate

Methyl 4-bromo-2-hydroxy-3-(3-hydroxypropyl)benzoate (2.31 g, 7.99 mmol), triphenylphosphine (5.03 g, 19.2 mmol) and tetrahydrofuran (40 mL) were added into a 100 mL single-mouth bottle at room temperature, slowly dropwise added with diethyl azodicarboxylate (3.34 g, 19.2 mmol) under ice bath after nitrogen replacement to react at room temperature overnight, cooled to room temperature, added with water (100 mL) for quenching, and then extracted with ethyl acetate (100 mL × 3). Organic phases were combined, washed with a saturated saline solution (100 mL), dried with anhydrous sodium sulfate, sucked and filtered, and then concentrated under reduced pressure. A residue obtained was purified by silica gel column chromatography (petroleum ether/ethyl acetate (v/v) = 5: 1) to obtain a yellow oil substance (1.67 g, yield 77.1%).
LCMS (ESI, *m*/*z*): 271.1 [M+H]⁺

### Step 5) Methyl 5-cyanochrome-8-formate

Methyl 5-bromochrome-8-formate (1.62 g, 5.96 mmol), zinc cyanide (3.51 g, 29.9 mmol), tris(dibenzylideneacetone)dipalladium (1.09 g, 1.20 mmol), 1,1'-bis(diphenylphosphino)ferrocene (1.32 g, 2.40 mmol) and *N*-methyl pyrrolidone (30 mL) were added into a 50 mL single-mouth bottle at room temperature, heated to 120°C to react for 2 hours after nitrogen replacement, cooled to room temperature, added with an aqueous solution (100 mL) for quenching, and then extracted with ethyl acetate (100 mL × 3). Organic phases were combined, washed with a saturated saline solution (100 mL), dried with anhydrous sodium sulfate, sucked and filtered, and then concentrated under reduced pressure. A residue was purified by column chromatography (petroleum ether/ethyl acetate (v/v) = 4: 1) to obtain a white solid (952 mg, yield 73.3%).
LCMS (ESI, m/z): 218.1 [M+H]⁺

### Step 6) 8-hydroxymethyl-5-chromenitrile

Methyl 5-cyanochrome-8-formate (860 mg, 3.96 mmol), tetrahydrofuran (10 mL) and a tetrahydrofuran solution (3.96 mL, 2.0 mol/L, 7.92 mmol) of lithium borohydride were added into a 50 mL single-mouth bottle at room temperature, heated to 60°C to react for 15 minutes after nitrogen replacement, cooled to room temperature, added with water (30 mL) for quenching, and then extracted with ethyl acetate (30 mL × 3). Organic phases were combined, washed with a saturated saline solution (30 mL), dried with anhydrous sodium sulfate, sucked and filtered, and then concentrated under reduced pressure. A residue was purified by column chromatography (petroleum ether/ethyl acetate (v/v) = 3: 1) to obtain a yellow oil substance (450 mg, yield 60.1%).
LCMS (ESI, m/z): 190.1 [M+H]⁺

### Step 7) 8-formylchromane-5-carbonitrile

8-hydroxymethyl-5-chromonitrile (385 mg, 1.75 mmol), dichloromethane (5 mL) and Dess-Martin periodinane (890 mg, 2.10 mmol) were added into a 25 mL single-mouth bottle at room temperature to react at room temperature for 2 hours, and then concentrated under reduced pressure. A residue was purified by column chromatography (petroleum ether/ethyl acetate (v/v) = 6: 1) to obtain a yellow solid (280 mg, yield 72.9%).
LCMS (ESI, m/z): 188.1 [M+H]⁺

### Step 8) 2-cyanoethyl 4-(5-cyano-8-hydroxy-2,8-dimethyl-1,4-dihydro-1,6-naphthyridine-3-carboxylate)

8-formylchromane-5-carbonitrile (200 mg, 1.07 mmol), 4-amino-5-methylpyridine-2-ol (200 mg, 1.61 mmol), 2-cyanoethyl 3-oxobutanoate (200 mg, 1.29 mmol), isopropanol (5 mL) and acetic acid (90 µL) were added into an 8 mL bottle at room temperature to react at 90°C overnight after nitrogen replacement, cooled to room temperature, and then concentrated under reduced pressure. A residue obtained was purified by C18 reversed-phase column chromatography under the following conditions (C18 BIOTAGE 120 g reversed-phase column, mobile phase A (0.1% formic acid aqueous solution) and mobile phase B (acetonitrile), increase from 20% B to 50% B for 15 minutes, and detection wavelength 254 nm) to obtain a yellow solid (175 mg, yield 36.7%).
LCMS (ESI, m/z): 431.2 [M+H]⁺

### Step 9) 2-cyanoethyl 4-(5-cyano-8-cyano)-5-ethoxy-2,8-dimethyl-1,4-dihydro-1,6-naphthyridine-3-carboxylate

2-cyanoethyl 4-(5-cyano-8-hydroxy-2,8-dimethyl-1,4-dihydro-1,6-naphthyridine-3-carboxylate) (175 mg, 0.407 mmol), silver carbonate (112 mg, 0.407 mmol), iodoethane (95 mg, 0.610 mmol) and 1,4-dioxane (2 mL) were added into an 8 mL bottle at room temperature to react at 90°C for 2 hours after nitrogen replacement, cooled to room temperature, and then filtered. The filter cake was rinsed with ethyl acetate (50 mL), and the filtrate was collected and concentrated under reduced pressure. A residue obtained was purified by C18 reversed-phase column chromatography under the following conditions (C18 BIOTAGE 120 g reversed-phase chromatographic column, mobile phase A (0.1% ammonium bicarbonate aqueous solution) and mobile phase B (acetonitrile), gradient: increase from 15% B to 60% B for 10 minutes, and 254 nm ultraviolet detector) to obtain a pale yellow oil substance (134 mg, yield 71.9%).
LCMS (ESI, m/z):459.2 [M+H]⁺

### Step 10) 4-(5-cyanored-8-yl)-5-ethoxy-2,8-dimethyl-1,4-dihydro-1,6-naphthyridine-3-carboxylic acid

2-cyanoethyl 4-(5-cyano-8-cyano)-5-ethoxy-2,8-dimethyl-1,4-dihydro-1,6-naphthyridine-3-carboxylate (134 mg, 0.292 mmol), ethylene glycol dimethyl ether (1 mL) and an aqueous solution (0.5 mL) of sodium hydroxide (24 mg, 0.600 mmol) were added into an 8 mL single-mouth bottle at room temperature to react at room temperature for 1 hour, added with a hydrochloric acid solution (1.0 mol/L) under ice bath to quench and adjust a PH value to be 5, and then extracted with ethyl acetate (20 mL × 3 times). Organic phases were combined, washed with a saturated saline solution (20 mL), dried with anhydrous sodium sulfate, sucked and filtered, and then concentrated under reduced pressure to obtain a pale yellow solid (83 mg, 70.1%), which was directly used in the reaction of the next step without further purification.
LCMS (ESI, m/z): 406.2 [M+H]⁺

### Step 11) 4-(5-cyanored-8-yl)-5-ethoxy-2,8-dimethyl-1,4-dihydro-1,6-naphthyridine-3-formamide

4-(5-cyanored-8-yl)-5-ethoxy-2,8-dimethyl-1,4-dihydro-1,6-naphthyridine-3-carboxylic acid (80 mg, 0.197 mmol), *N*,*N*-diisopropylethylamine (77 mg, 0.591 mmol), *N*,*N*-dimethylformamide (2 mL) and 2-(7-azobenzotriazole)-N,N,N',N'-tetramethylurea hexafluorophosphate (225 mg, 0.591 mmol) were added into an 8 mL bottle at room temperature to react at room temperature for 1 hour. The reaction solution was added with an aqueous solution (1.0 M, 0.7 mL) of ammonia at room temperature to react at room temperature for 2 hours, and then sucked and filtered. The filtrate obtained was purified by preparative high-performance liquid chromatography under the following purification conditions: chromatographic column: YMC-Actus Triart C18; mobile phase A: water (containing 0.1% formic acid) and mobile phase B: acetonitrile; flow rate: 60 mL/min; gradient: increase of acetonitrile from 25% to 45% within 7 minutes; and detection wavelength: 254/220 nm. The product was collected, and freeze-dried under reduced pressure to obtain a yellow solid (59.0 mg, yield 73.9%).
LCMS (ESI, m/z): 405.25 [M+H]⁺

¹H NMR (400 MHz, DMSO-*d*6) δ 7.70 (s, 1H), 7.56 (s, 1H), 7.18 (d, *J* = 8.0 Hz, 1H), 6.94 (d, *J* = 8.0 Hz, 1H), 6.68 (s, 2H), 5.32 (s, 1H), 4.28 - 4.20 (m, 2H), 4.05 (q, *J* = 7.2 Hz, 1H), 2.87 (t, *J* = 6.4 Hz, 2H), 2.21 (s, 3H), 2.12 (s, 3H), 2.03 - 1.94 (m, 2H), 1.10 (t, *J* = 7.2 Hz, 3H).

### Example 7 10-(4-cyano-2-methoxyphenyl)-6,8-dimethyl-7H,10H-pyrazolo[3,2-f]1,6-naphthyridine-9-carboni trile

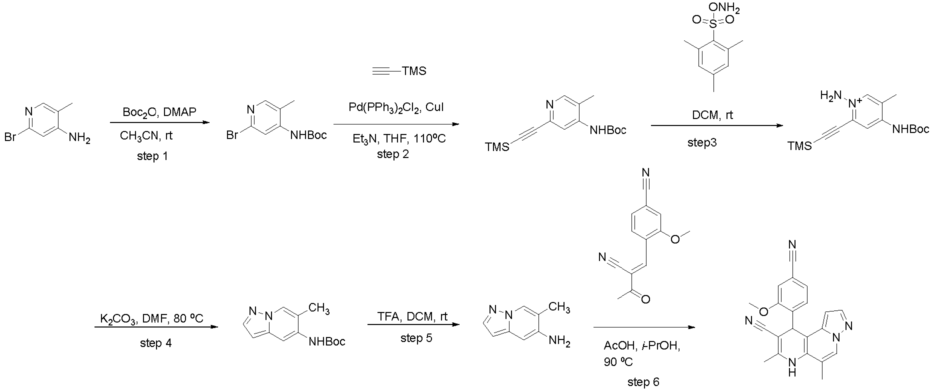

### Step 1) N-(2-bromo-5-methylpyridine-4-yl)tert-butyl carbamate

2-bromo-5-methylpyridine-4-amine (1.00 g, 5.35 mmol), *N*,*N*-dimethylpyridine-4-amine (66 mL, 0.540 mmol), di-tert-butyl dicarbonate (1.4 g, 6.42 mmol) and acetonitrile (12 mL) were added into a 50 mL single-mouth bottle at room temperature to react at room temperature for 3 hours, cooled to room temperature, added with water (30 mL) for quenching, and then extracted with ethyl acetate (30 mL × 3). Organic phases were combined, washed with a saturated saline solution (30 mL), dried with anhydrous sodium sulfate, sucked and filtered, and then concentrated under reduced pressure. A residue was purified by silica gel column chromatography (eluent: petroleum ether/ethyl acetate (v/v) = 3/1) to obtain a yellow solid (1.05 g, yield 28.3%).
MS (ESI) M/Z: 288 [M-H]⁻

### Step 2) N-{5-methyl-2-[2-(trimethylsilyl)ethynyl]pyridine-4-yl }tert-butyl carbamate

*N*-(2-bromo-5-methylpyridine-4-yl)tert-butyl carbamate (1.0 g, 3.48 mmol), bis(triphenylphosphine)palladium dichloride (733 mg, 1.05 mmol), cuprous iodide (3.99 g, 2.10 mmol), trimethylethynylsilane (4.51 mL, 34.8 mmol) and tetrahydrofuran (30 mL) were added into a 100 mL single-mouth bottle at room temperature, heated to 110°C to react for 2 hours after nitrogen replacement, cooled to room temperature, and then concentrated under reduced pressure. A residue was purified by silica gel column chromatography (eluent: petroleum ether/ethyl acetate (v/v) = 5/1) to obtain a grey solid (640 mg, yield 56.1%).
MS (ESI) M/Z: 303.20 [M-H]⁻

### Step 3) 1-amino-4-[tert-butoxycarbonylamino]-5-methyl-2-[2-(trimethylsilyl)ethynyl]pyridine-1-salt

N-{ 5-methyl-2-[2-(trimethylsilyl)ethynyl]pyridine-4-yl }tert-butyl carbamate (130 mg, 0427 mmol), dichloromethane (2.5 mL) and amino-2,4,6-trimethylbenzenesulfonate (276 mg, 1.28 mmol) were added into an 8 mL bottle at room temperature to react at room temperature for 2 hours after nitrogen replacement, cooled to room temperature, and then concentrated under reduced pressure. A solid was treated with methyl tert-butyl ether (5 mL × 3) to obtain a grey solid (120 mg, yield 87.7%).
MS (ESI) M/Z: 319.90 [M-H]⁻

### Step 4) N-{6-methylpyrazolo[1,5-a]pyridine-5-yl}tert-butyl carbamate

1-amino-4-[tert-butoxycarbonylamino]-5-methyl-2-[2-(trimethylsilyl)ethynyl]pyridine-1-salt (400 mg, 1.25 mmol), potassium carbonate (345 mg, 2.45 mmol) and N,N-dimethylformamide (4 mL) were added into an 8 mL bottle at room temperature, heated to 80°C to react for 5 hours after nitrogen replacement, cooled to room temperature, and then sucked and filtered. The filtrate was purified by C18 reversed-phase column chromatography under the following conditions (C18 BIOTAGE 40 g reversed-phase column, mobile phase A (0.1% formic acid aqueous solution) and mobile phase B (acetonitrile, Filtrate increase from 40% B to 90% B for 15 minutes, and detection wavelength 254 nm) to obtain a grey solid (100 mg, yield 28.2%).
MS (ESI) M/Z: 248.25 [M+H]⁺

### Step 5) 6-methylpyrazolo[1,5-a]pyridine-5-amine

*N*-{6-methylpyrazolo[1,5-*a*]pyridine-5-yl}tert-butyl carbamate (200 mg, 0.81 mmol), dichloromethane (4 mL) and trifluoroacetic acid (1 mL) were added into a 25 mL single-mouth bottle at room temperature to react at room temperature for 2 hours, and then concentrated under reduced pressure. A residue was dissolved in ethyl acetate (10 mL), washed with saturated sodium bicarbonate aqueous solution (10 mL) and saturated saline solution (10 mL) in sequence, dried with anhydrous sodium sulfate, and then concentrated under reduced pressure to obtain a grey solid (100 mg, yield 84.1%).

### Step 6) 10-(4-cyano-2-methoxyphenyl)-6,8-dimethyl-7H,10H-pyrazolo[3,2-f]1,6-naphthyridine-9-carbonitrile

6-methylpyrazolo[1,5-*a*]pyridine-5-amine (85 mg, 0.578 mmol), 4-(2-cyano-3-oxobut-1-en-1-yl)-3-methoxybenzonitrile (145 mg, 0.639 mmol) and an isopropanol (2 mL) solution of glacial acetic acid (36 µL) were added into an 8 mL bottle at room temperature, heated to 90°C to react overnight after nitrogen replacement, cooled to room temperature, and then concentrated under reduced pressure. A residue obtained was purified by C18 reversed-phase column chromatography under the following conditions (C18 BIOTAGE 40 g reversed-phase column, mobile phase A (0.1% formic acid aqueous solution) and mobile phase B (acetonitrile), increase from 15% B to 45% B for 15 minutes, and detection wavelength 254 nm) to obtain a white solid (7 mg, yield 3.4%).
LCMS (ESI, m/z): 356.15 [M+H]⁺

¹H NMR (400 MHz, DMSO-*d*6) δ 8.70 (s, 1H), 8.41 (s, 1H), 7.68 (s, 1H), 7.52 (s, 1H), 7.33 (d, *J* = 8.0 Hz, 1H), 7.26 (d, *J* = 8.0 Hz, 1H), 5.92 (s, 1H), 5.46 (s, 1H), 3.95 (s, 3H), 2.30 (s, 3H), 2.20 (s, 3H).

### Example 8 4-(4-cyano-2-methoxyphenyl)-2-methyl-1,4-dihydrobenzo[4,5]thieno[2,3-b]pyridine-3-carbonitril

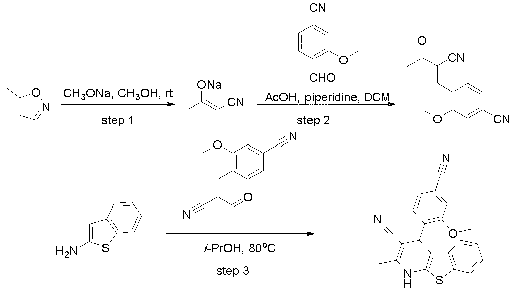

### Step 1) 1-cyanopropene-2-sodium acid

Sodium methoxide (5.00 g, 60.0 mmol) and anhydrous methanol (30 mL) were added into a 100 mL single-mouth bottle at room temperature, and then dropwise added with 5-methylisoxazole (3.25 g, 60.0 mmol) under ice water bath to react at room temperature overnight. A precipitate was generated, and then sucked and filtered. A solid obtained was pulped with methanol (15 mL), and then sucked and filtered to obtain a yellow solid (3.10 g, yield 84.7%).

### Step 2) 4-(2-cyano-3-oxobut-1-en-1-yl)-3-methoxybenzonitrile

1-cyanopropene-2-sodium acid (3.00 g, 28.6 mmol), 4-formyl-3-methoxybenzonitrile (4.59 g, 28.6 mmol), dichloromethane (50 mL), piperidine (240 mg, 2.85 mmol) and glacial acetic acid (2.28 g, 57.1 mmol) were added into a 100 mL single-mouth bottle at room temperature, heated to 60°C to react overnight after nitrogen replacement, cooled to room temperature, and then concentrated under reduced pressure. A residue was pulped with ethyl acetate (10 mL × 5), and then sucked and filtered to obtain a yellow solid (2.50 g, yield 38.8%).

### MS (ESI) M/Z: 227.95 [M+H]⁺

### Step 3) 4-(4-cyano-2-methoxyphenyl)-2-methyl-1,4-dihydrobenzo[ 4,5 ]thieno[2,3-b]pyridine-3-carbonitrile

1-benzothiophene-2-amine (50 mg, 0.335 mmol), 4-(2-cyano-3-oxobut-1-en-1-yl)-3-methoxybenzonitrile (76 mg, 0.335 mmol) and an isopropanol (0.5 mL) solution of glacial acetic acid (9 µL) were added into an 8 mL bottle at room temperature, heated to 80°C to react for 1 hour after nitrogen replacement, cooled to room temperature, and then concentrated under reduced pressure. A residue obtained was purified by preparative high-performance liquid chromatography (column model: Sunfire prep C18 silica gel column, 30*150 mm, 5 µm; mobile phase A: water (0.1% formic acid), mobile phase B: acetonitrile; flow rate: 60 mL/min; gradient: increase from 48% B to 68% B for 8 minutes; wavelength: 254/220 nm; and peak appearance time (min): 6.90) to obtain a white solid (22.3 mg, yield 18.1%).
MS (ESI) M/Z: 356.05 [M-H]⁻

¹H NMR (400 MHz, DMSO-*d*6) δ 10.25 (s, 1H), 7.81 - 7.75 (m, 1H), 7.50 (S, 1H), 7.38 - 7.22 (m, 2H), 7.20 - 7.10 (m, 2H), 7.08 - 7.02 (m, 1H), 5.60 (s, 1H), 3.97 (s, 3H), 2.13 (s, 3H).

### Example 9 4-(4-cyano-2-methoxyphenyl)-3-ethoxy-1,6-dimethyl-4,7-dihydropyrazolo[3,4-b]pyridine-5-carb onitrile

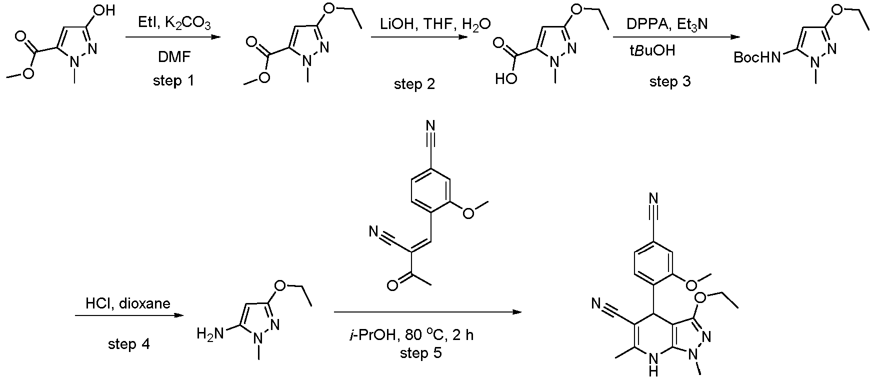

### Step 1) N-(2-cyanocyclopent-1-ene-1-yl)acetamide

Methyl 3-hydroxy-1-methylpyrazole-5-carboxylate (2.30 g, 14.7 mmol), ethyl iodide (3.40 g, 22.1 mmol), potassium carbonate (2.00 g, 14.7 mmol) and *N*,*N*-dimethylformamide (25 mL) were added into a 100 mL single-mouth bottle at room temperature, heated to 60°C to react for 5 hours after nitrogen replacement, cooled to room temperature, added with water (30 mL) for quenching, and then extracted with ethyl acetate (30 mL × 3). Organic phases were combined, washed with a saturated saline solution (30 mL), dried with anhydrous sodium sulfate, sucked and filtered, and then concentrated under reduced pressure to obtain a pale yellow oil substance (2.30 g, yield 84.7%).
LCMS (ESI, m/z): 185.10 [M+H]⁺

### Step 2) 3-ethoxy-1-methylpyrazole-5-carboxylic acid

*N*-(2-cyanocyclopent-1-ene-1-yl)acetamide (2.30 g, 12.5 mmol), tetrahydrofuran (15 mL) and a saturated aqueous solution (15 mL) of lithium hydroxide were added into a 100 mL single-mouth bottle at room temperature to react at room temperature for 3 hours, added with a hydrochloric acid solution (1.0 mol/L) to quench and adjust a PH value to be 5, and then extracted with ethyl acetate (30 mL × 3 times). Organic phases were combined, washed with a saturated saline solution (30 mL), dried with anhydrous sodium sulfate, sucked and filtered, and then concentrated under reduced pressure to obtain a white solid (2.00 g, yield 94.1%).
LCMS (ESI, m/z): 171.10 [M+H]⁺

### Step 3) Tert-butyl(3-ethoxy-1-methylpyrazole-5-yl)carbamate

3-ethoxy-1-methylpyrazole-5-carboxylic acid (2.00 g, 11.7 mmol), triethylamine (3.57 g, 35.3 mmol) and tert-butanol (15 mL) were added into a 100 mL single-mouth bottle at room temperature, slowly dropwise added with diphenyl azide phosphate (4.85 g, 17.6 mmol) after nitrogen replacement, heated to 80°C to react overnight, cooled to room temperature, added with water (20 mL) for quenching, and then extracted with ethyl acetate (30 mL × 3 times). Organic phases were combined, washed with a saturated saline solution (30 mL), dried with anhydrous sodium sulfate, sucked and filtered, and then concentrated under reduced pressure. A residue obtained was purified by C18 reversed-phase column chromatography under the following conditions (C18 BIOTAGE 40 g reversed-phase column, mobile phase A (0.1% formic acid aqueous solution) and mobile phase B (acetonitrile increase from 30% B to 50% B for 10 minutes, and detection wavelength 254 nm) to obtain a white solid (1.60 g, yield 56.4%).
LCMS (ESI, m/z): 242.20 [M+H]⁺

### Step 4) 3-ethoxy-1-methylpyrazole-5-amine

Tert-butyl(3-ethoxy-1-methylpyrazole-5-yl)carbamate (800 mg, 3.32 mmol) and a 1,4-dioxane solution (4.0 M, 5 mL) of hydrogen chloride were added into a 25 mL single-mouth bottle at room temperature, stirred at room temperature overnight, added with a saturated sodium bicarbonate aqueous solution to quench and adjust a pH value to be 7, and then extracted with ethyl acetate (50 mL × 3 times). Organic phases were combined, washed with a saturated saline solution (30 mL), dried with anhydrous sodium sulfate, sucked and filtered, and then concentrated under reduced pressure to obtain a yellow solid (405 mg, yield 86.5%).
LCMS (ESI, m/z): 142.10 [M+H]⁺

### Step 5) 4-(4-cyano-2-methoxyphenyl)-3-ethoxy-1,6-dimethyl-4,7-dihydropyrazolo[3,4-b]pyridine-5-carbonitri le

3-ethoxy-1-methylpyrazole-5-amine (50 mg, 0.344 mmol), 4-(2-cyano-3-oxobut-1-en-1-yl)-3-methoxybenzonitrile (78 mg, 0.344 mmol) and an isopropanol (1 mL) solution of glacial acetic acid (18 µL) were added into an 8 mL bottle at room temperature, heated to 80°C to react for 2 hours under nitrogen protection, cooled to room temperature, added with water (5 mL) for quenching, and then extracted with ethyl acetate (10 mL × 3 times). Organic phases were combined, washed with a saturated saline solution (10 mL), dried with anhydrous sodium sulfate, sucked and filtered, and then concentrated under reduced pressure. A residue obtained was purified by C18 reversed-phase column chromatography under the following conditions (C18 BIOTAGE 40 g reversed-phase column, mobile phase A (10 mmol/L ammonium bicarbonate aqueous solution) and mobile phase B (acetonitrile), increase from 30% B to 50% B for 10 minutes, and detection wavelength 254 nm) to obtain a white solid (8.5 mg, yield 7.73%).
MS (ESI) M/Z: 350.20 [M+H]⁺.

¹H NMR (300 MHz, DMSO-*d*6) δ 9.74 (s, 1H), 7.47 (s, 1H), 7.38 (d, *J* = 7.9 Hz, 1H), 7.17 (d, *J* = 7.9 Hz, 1H), 5.16 (s, 1H), 3.99 - 3.78 (m, 5H), 3.80 (s, 3H), 2.13 (s, 3H), 1.03 (t, *J* = 7.0 Hz, 3H).

### Example 10 9-(4-cyano-2-methoxyphenyl)-5,7-dimethyl-6,9-dihydro[1,2,4]triazolo[4,3-a][1,5]naphthyridine-8 -carbonitrile

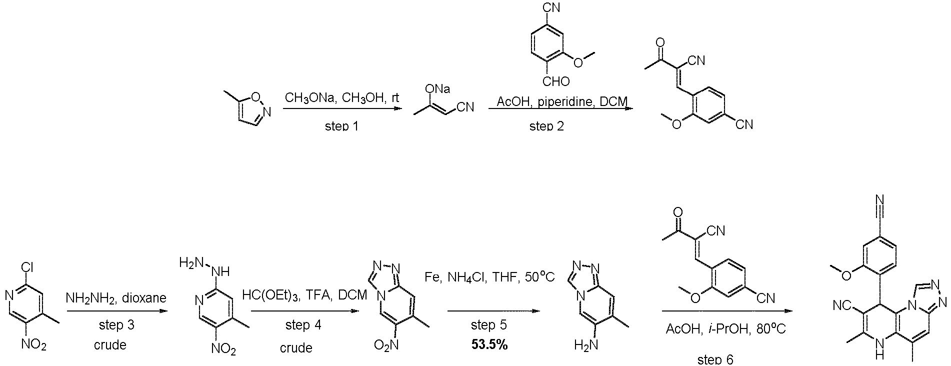

### Step 1) 1-cyanopropene-2-sodium acid

Sodium methoxide (5.00 g, 60.0 mmol) and anhydrous methanol (30 mL) were added into a 100 mL single-mouth bottle at room temperature, and then dropwise added with 5-methylisoxazole (3.25 g, 60.0 mmol) under ice water bath to react at room temperature overnight. A precipitate was generated, and then sucked and filtered. A solid obtained was pulped with methanol (15 mL), and then sucked and filtered to obtain a yellow solid (3.10 g, yield 84.7%).

### Step 2) 4-(2-cyano-3-oxobut-1-en-1-yl)-3-methoxybenzonitrile

1-cyanopropene-2-sodium acid (3.00 g, 28.6 mmol), 4-formyl-3-methoxybenzonitrile (4.59 g, 28.6 mmol), dichloromethane (50 mL), piperidine (240 mg, 2.85 mmol) and glacial acetic acid (2.28 g, 57.1 mmol) were added into a 100 mL single-mouth bottle at room temperature, heated to 60°C to react overnight after nitrogen replacement, cooled to room temperature, and then concentrated under reduced pressure. A residue was pulped with ethyl acetate (10 mL × 5), and then sucked and filtered to obtain a yellow solid (2.50 g, yield 38.8%).

### Step 3) 2-hydrazino-4-methyl-5-nitropyridine

6-chloro-2-methyl-3-nitropyridine (1.73 g, 10.0 mmol) and 1,4-dioxane (15 mL) were added into a 100 mL single-mouth bottle at room temperature, added with hydrazine hydrate (2.00 g, 40 mmol) under ice water bath to react at room temperature overnight, and then concentrated under reduced pressure. A residue was pulped with water (5 mL), and then sucked and filtered to obtain a pale yellow solid (1.30 g, crude product), which was directly used in the reaction of the next step without further purification.

### Step 4) 7-methyl-6-nitro[1,2,4]triazolo[4,3-a]pyridine

2-hydrazino-4-methyl-5-nitropyridine (1.00 g, 5.95 mmol), triethyl orthoformate (2.52 g, 23.8 mmol) and dichloromethane (50 mL) were added into a 100 mL single-mouth bottle at room temperature, added with trifluoroacetic acid (140 mg, 1.19 mmol) under ice water bath to react at room temperature overnight, and then concentrated under reduced pressure to obtain a pale yellow solid (900 mg, crude product).

### Step 5) 7-methyl-[1,2,4]triazolo[4,3-a]pyridine-6-amine

7-methyl-6-nitro[1,2,4]triazolo[4,3-*a*]pyridine (900 mg, 5.01 mmol), iron powder (1.13 g, 20.2 mmol), ammonium chloride (1.07 g, 20.2 mmol) and tetrahydrofuran (50 mL) were added into a 100 mL single-mouth bottle at room temperature, heated to 50°C to react overnight, cooled to room temperature, sucked and filtered through diatomite, and then concentrated under reduced pressure. A residue was purified by column chromatography (petroleum ether/ethyl acetate (v/v) = 2/1) to obtain a pale yellow solid (400 mg, yield 53.5%).

### Step 6) 9-(4-cyano-2-methoxyphenyl)-5,7-dimethyl-6,9-dihydro[1,2,4]triazolo[4,3-a][1,5]naphthyridine-8-car bonitrile

7-methyl-[1,2,4]triazolo[4,3-*a*]pyridine-6-amine (50 mg, 0.337 mmol), 4-(2-cyano-3-oxobut-1-en-1-yl)-3-methoxybenzonitrile (76 mg, 0.337 mmol) and an isopropanol (3 ml L) solution of glacial acetic acid (54 µL) were added into an 8 mL bottle at room temperature, heated to 80°C to react overnight after nitrogen replacement, cooled to room temperature, and then concentrated under reduced pressure. A residue obtained was purified by C18 reversed-phase column chromatography under the following conditions (C18 BIOTAGE 40 g reversed-phase column, mobile phase A (0.1% formic acid aqueous solution) and mobile phase B (acetonitrile increase from 30% B to 50% B for 10 minutes, and detection wavelength 254 nm) to obtain a white solid (20.0 mg, yield 16.7%).
MS (ESI) M/Z: 357.15 [M+H]

¹H NMR (400 MHz, DMSO-*d*6) δ 8.80 (s, 1H), 8.18 (s, 1H), 7.82-7.52 (m, 2H) , 7.28 (s, 1H), 7.04 (s, 1H), 5.89 (s, 1H), 3.88 (s, 3H), 2.19 (s, 3H).

### Example 11 4-(4-cyano-2,3-dihydrobenzofuran-7-yl)-5-ethoxy-2,8-dimethyl-1,4-dihydro-1,6-naphthyridine-3-formamide

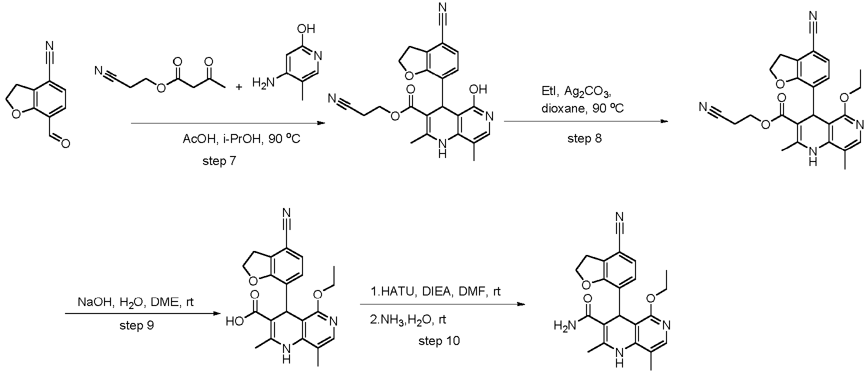

### Step 1) Methyl 4-amino-5-chloro-2,3-dihydro-1-benzofuran-7-carboxylate

4-amino-5-chloro-2,3-dihydro-1-benzofuran-7-carboxylic acid (10.0 g, 46.9 mmol) and methanol (200 mL) were added into a 500 mL single-mouth bottle at room temperature, slowly dropwise added with thionyl chloride (8.35 g, 61.9 mmol) under ice water bath, heated to 70°C to react for 1.5 hours, cooled to room temperature, added with a saturated sodium bicarbonate aqueous solution (300 mL) for quenching, and then extracted with ethyl acetate (500 mL × 3). Organic phases were combined, washed with a saturated saline solution (500 mL), dried with anhydrous sodium sulfate, sucked and filtered, and then concentrated under reduced pressure to obtain a yellow solid (10.5 g, yield 98.5%).
LCMS (ESI, m/z): 228.6 [M+H]⁺

### Step 2) Methyl 4-amino-2,3-dihydro-1-benzofuran-7-carboxylate

Methyl 4-amino-5-chloro-2,3-dihydro-1-benzofuran-7-carboxylate (10.5 g, 46.2 mmol), 10% palladium carbon (5.35 g, 4.99 mmol), sodium hydroxide (3.15 g, 78.8 mmol) and methanol (200 mL) were added into a 500 mL sealed tube inner container at room temperature, heated to 30°C to react overnight under 3 atmospheric hydrogen pressures, cooled to room temperature, and then sucked and filtered through diatomite. The filter cake was rinsed with ethyl acetate (500 mL), and the filtrates were combined, washed with a saturated saline solution (500 mL), dried with anhydrous sodium sulfate, sucked and filtered, and then concentrated under reduced pressure to obtain a yellow solid (4.80 g, yield 53.6%).
LCMS (ESI, m/z): 194.2 [M+H]⁺

### Step 3) Methyl 4-bromo-2,3-dihydro-1-benzofuran-7-carboxylate

Methyl 4-amino-2,3-dihydro-1-benzofuran-7-carboxylate (4.20 g, 21.8 mmol), tert-butyl nitrite (3.15 g, 30.7 mmol), cuprous bromide (4.20 g, 29.4 mmol) and acetonitrile (20 mL) were added into a 50 mL single-mouth bottle at room temperature, heated to 70°C to react for a half hour after nitrogen replacement, cooled to room temperature, and then concentrated under reduced pressure. A residue was purified by column chromatography (petroleum ether/ethyl acetate (v/v) = 5: 1) to obtain a yellow solid (3.50 g, yield 62.8%).

### Step 4) Methyl 4-cyano-2,3-dihydro-1-benzofuran-7-carboxylate

Methyl 4-bromo-2,3-dihydro-1-benzofuran-7-carboxylate (3.20 g, 12.5 mmol), zinc cyanide (9.54 g, 81.6 mmol), tris(dibenzylideneacetone)dipalladium (1.13 g, 1.25 mmol), 1,1'-bis(diphenylphosphanyl)ferrocene (1.37 g, 2.50 mmol) and *N*-methyl pyrrolidone (20 mL) were added into a 50 mL single-mouth bottle at room temperature, heated to 120°C to react for 2 hours after nitrogen replacement, cooled to room temperature, added with an aqueous solution (100 mL) for quenching, and then extracted with ethyl acetate (100 mL × 3). Organic phases were combined, washed with a saturated saline solution (100 mL), dried with anhydrous sodium sulfate, sucked and filtered, and then concentrated under reduced pressure. A residue was purified by column chromatography (petroleum ether/ethyl acetate (v/v) = 6: 1) to obtain a white solid (2.20 g, yield 86.6%).

### Step 5) 7-hydroxymethyl-2,3-dihydro-1-benzofuran-4-carbonitrile

Methyl 4-cyano-2,3-dihydro-1-benzofuran-7-carboxylate (1.80 g, 8.86 mmol), tetrahydrofuran (20 mL) and a tetrahydrofuran solution (6.54 mL, 2.0 mol/L, 13.3 mmol) of lithium borohydride were added into a 50 mL single-mouth bottle at room temperature, heated to 60°C to react for 20 minutes after nitrogen replacement, cooled to room temperature, added with water (50 mL) for quenching, and then extracted with ethyl acetate (50 mL × 3). Organic phases were combined, washed with a saturated saline solution (50 mL), dried with anhydrous sodium sulfate, sucked and filtered, and then concentrated under reduced pressure. A residue was purified by column chromatography (petroleum ether/ethyl acetate (v/v) = 3: 1) to obtain a white solid (960 mg, yield 61.9%).

### Step 6) 7-formyl-2,3-dihydro-1-benzofuran-4-carbonitrile

7-hydroxymethyl-2,3-dihydro-1-benzofuran-4-carbonitrile (920 mg, 6.58 mmol), dichloromethane (10 mL) and Dess-Martin Periodinane (3.24 g, 7.04 mmol) were added into a 25 mL single-mouth bottle at room temperature to react at room temperature for 2 hours after nitrogen replacement, and then concentrated under reduced pressure. A residue was purified by column chromatography (petroleum ether/ethyl acetate (v/v) = 6: 1) to obtain a yellow solid (800 mg, yield 70.2%).

### Step 7) 2-cyanoethyl-4-(4-cyano-2,3-dihydro-1-benzofuran-7-yl)-5-hydroxy-2,8-dimethyl-1,4-dihydro-1,6-nap hthyridine-3-carboxylate

7-formyl-2,3-dihydro-1-benzofuran-4-carbonitrile (100 mg, 0.577 mmol), 4-amino-5-methylpyridine-2-ol (100 mg, 0.806 mmol), 2-cyanoethyl 3-oxobutanoate (100 mg, 0.645 mmol), isopropanol (2.5 mL) and acetic acid (45 µL) were added into an 8 mL bottle at room temperature to react at 90°C overnight after nitrogen replacement, cooled to room temperature, and then concentrated under reduced pressure. A residue obtained was purified by C18 reversed-phase column chromatography under the following conditions (C18 BIOTAGE 40 g reversed-phase column, mobile phase A (10 mmol/L ammonium bicarbonate aqueous solution) and mobile phase B (acetonitrile), increase from 30% B to 50% B for 15 minutes, and detection wavelength 254 nm) to obtain a yellow solid (110 mg, yield 45.7%).
LCMS (ESI, m/z): 417.4 [M+H]⁺

### Step 8) 2-cyanoethyl 4-(4-cyano-2,3-dihydrobenzofuran-7-yl)-5-ethoxy-2,8-dimethyl-1,4-dihydro-1,6-naphthyridine-3-carb oxylate

2-cyanoethyl-4-(4-cyano-2,3-dihydro-1-benzofuran-7-yl)-5-hydroxy-2,8-dimethyl-1,4-dihydro-1,6-na phthyridine-3-carboxylate (100 mg, 0.240 mmol), silver carbonate (73 mg, 0.264 mmol), iodoethane (75 mg, 0.480 mmol) and 1,4-dioxane (3 mL) were added into an 8 mL bottle at room temperature to react at 90°C for 1 hour after nitrogen replacement, cooled to room temperature, and then filtered. The filter cake was rinsed with ethyl acetate (50 mL), and the filtrate was collected and concentrated under reduced pressure to obtain a yellow solid (110 mg, yield 45.7%), which was directly used in the reaction of the next step without further purification.
LCMS (ESI, m/z):445.20 [M+H]⁺

### Step 9) 4-(4-cyano-2,3-dihydrobenzofuran-7-yl)-5-ethoxy-2,8-dimethyl-1,4-dihydro-1,6-naphthyridine-3-carb oxylic acid

2-cyanoethyl 4-(4-cyano-2,3-dihydrobenzofuran-7-yl)-5-ethoxy-2,8-dimethyl-1,4-dihydro-1,6-naphthyridine-3-carboxyla te (100 mg, 0.225 mmol), ethylene glycol dimethyl ether (0.6 mL) and an aqueous solution (0.3 mL) of sodium hydroxide (18 mg, 0.450 mmol) were added into an 8 mL single-mouth bottle at room temperature to react at room temperature for 1 hour, added with a hydrochloric acid solution (1.0 mol/L) under ice bath to quench and adjust a PH value to be 5, and then extracted with ethyl acetate (20 mL × 3 times). Organic phases were combined, washed with a saturated saline solution (20 mL), dried with anhydrous sodium sulfate, sucked and filtered, and then concentrated under reduced pressure to obtain a pale yellow solid (80 mg, 90.7%), which was directly used in the reaction of the next step without further purification.
LCMS (ESI, m/z): 392.4 [M+H]+

### Step 10) 4-(4-cyano-2,3-dihydrobenzofuran-7-yl)-5-ethoxy-2,8-dimethyl-1,4-dihydro-1,6-naphthyridine-3-for mamide

4-(4-cyano-2,3-dihydrobenzofuran-7-yl)-5-ethoxy-2,8-dimethyl-1,4-dihydro-1,6-naphthyridine-3-carb oxylic acid (80 mg, 0.204 mmol), *N*,*N*-diisopropylethylamine (91 mg, 0.712 mmol), *N*,*N*-dimethylformamide (1 mL) and 2-(7-azobenzotriazole)-N,N,N',N'-tetramethylurea hexafluorophosphate (39 mg, 0.245 mmol) were added into an 8 mL bottle at room temperature to react at room temperature for 1 hour. The reaction solution was added with an aqueous solution (1.0 M, 0.5 mL) of ammonia at room temperature to react at room temperature for 1 hour, and then sucked and filtered. The filtrate obtained was purified by preparative high-performance liquid chromatography under the following purification conditions: chromatographic column: YMC-Actus Triart C18; mobile phase A: water (containing 0.1% formic acid) and mobile phase B: acetonitrile; flow rate: 60 mL/min; gradient: increase of acetonitrile from 20% to 40% within 7 minutes; and detection wavelength: 254/220 nm. The product was collected, and freeze-dried under reduced pressure to obtain a yellow solid (45.0 mg, yield 56.1%).
LCMS (ESI, m/z): 391.10 [M+H]⁺

¹H NMR (400 MHz, DMSO-*d*6) δ 7.70 (s, 1H), 7.55 (s, 1H), 7.11 (d, *J* = 8.0 Hz, 1H), 6.98 (d, *J* = 8.0 Hz, 1H), 6.71 (s, 2H), 5.18 (s, 1H), 4.63 (t, *J* = 8.8 Hz, 2H), 4.04 (q, *J* = 6.8 Hz, 2H), 3.40 (t, *J* = 8.8 Hz, 2H), 2.11 (s, 3H), 2.07 (s, 3H), 1.10 (t, *J* = 6.8 Hz, 3H).

### Example 12 4-(4-cyano-2-methoxyphenyl)-5-ethoxy-2-methyl-1H,4H-benzo[h]-1,6-naphthyridine-3-carbonitr ile

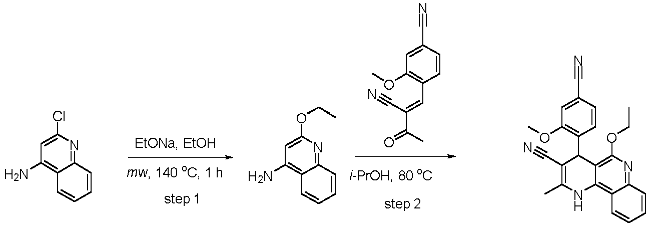

### Step 1) 2-ethoxyquinoline-4-amine

2-chloroquinoline-4-amine (200 mg, 1.12 mmol), sodium ethoxide (305 mg, 4.48 mmol) and ethanol (2.5 mL) were added into a 10 mL microwave tube at room temperature, microwave-heated to 140°C to react for 1 hour, cooled to room temperature, and then concentrated under reduced pressure. A residue was dissolved in ethyl acetate (100 mL), washed with a saturated saline solution (100 mL), dried with anhydrous sodium sulfate, sucked and filtered, and then concentrated under reduced pressure. A residue obtained was purified by C18 reversed-phase column chromatography under the following conditions (C18 BIOTAGE 40 g reversed-phase column, mobile phase A (0.1% ammonia water) and mobile phase B (acetonitrile), increase from 25% B to 35% B for 10 minutes, and detection wavelength 254 nm) to obtain a white solid (75 mg, yield 35.6%).
MS (ESI) M/Z: 189 [M+H]⁺

### Step 2) 4-(4-cyano-2-methoxyphenyl)-5-ethoxy-2-methyl-1H,4H-benzo[h]-1,6-naphthyridine-3-carbonitrile

2-ethoxyquinoline-4-amine (75 mg, 0.398 mmol), 4-(2-cyano-3-oxobut-1-en-1-yl)-3-methoxybenzonitrile (90 mg, 0.398 mmol) and an isopropanol (2 mL) solution of glacial acetic acid (36 µL) were added into an 8 mL bottle at room temperature, heated to 80°C to react overnight after nitrogen replacement, cooled to room temperature, and then concentrated under reduced pressure. A residue obtained was purified by high-performance liquid chromatography (column model: YMC-Actus Triart C18, 30*150 mm, 5 µm; mobile phase A: water (0.1% formic acid), mobile phase B: acetonitrile; flow rate: 60 mL/min; gradient: increase from 55% B to 85% B for 7 minutes; wavelength: 254/220 nm; and peak appearance time (min): 6.15), and a fraction was collected and freeze-dried under a reduced presure to obtain a white solid (48.8 mg, yield 30.7%).
MS (ESI) M/Z: 397.15 [M+H]⁺

¹H NMR (400 MHz, DMSO-*d*6) δ 9.60 (s, 1H), 8.36 (d, *J* = 8.4 Hz, 1H), 7.63 (d, *J* = 3.9 Hz, 2H), 7.52 - 7.41 (m, 2H), 7.29 - 7.33 (m, 1H), 7.12 (d, *J* = 7.9 Hz, 1H), 5.37 (s, 1H), 4.23-4.13 (m, 2H), 3.87 (s, 3H), 2.23 (m, 3H), 1.04 (t, *J* = 7.0 Hz, 3H).

### Example 13 6-(4-cyano-2-methoxyphenyl)-5-ethoxy-8-methyl-6,9-dihydrothieno [3,2-h][1,6]naphthyridine-7-f ormamide

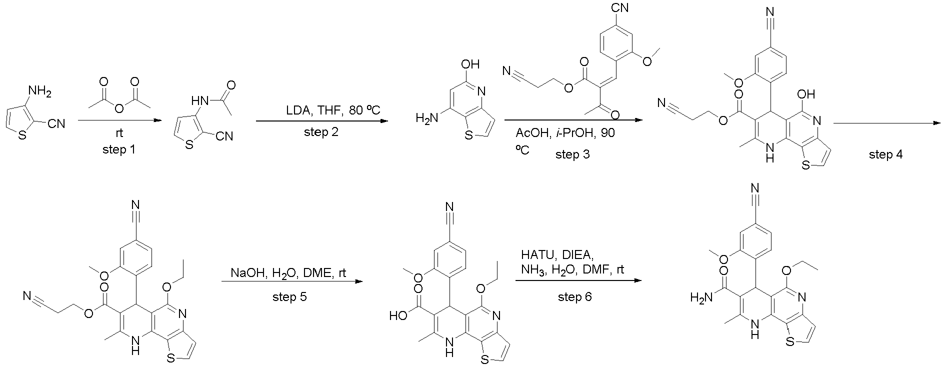

### Step 1) N-(2-cyanothiophene-3-yl)acetamide

3-amino-2-cyano-thiophene (1.00 g, 8.05 mmol) and acetic anhydride (10 mL) were added into a 50 mL single-mouth bottle at room temperature to react at room temperature overnight, and then concentrated under reduced pressure. A residue was pulped with petroleum ether (30 mL), and then sucked and filtered to obtain a white solid (1.20 g, yield 89.6%).
LCMS (ESI, m/z): 167.0 [M+H]⁺

### Step 2) 7-aminothieno[3,2-b]pyridine-5-ol

*N*-(2-cyanothiophene-3-yl)acetamide (1.20 g, 7.22 mmol) and tetrahydrofuran (24 mL) were added into a 100 mL three-mouth bottle at room temperature, slowly dropwise added with lithium diisopropylamide (6 mL, 44.3 mmol) at -78°C after nitrogen replacement to react at -78°C for a half hour, then heated to 80°C to react for 1 hour, cooled to room temperature, added with saturated ammonium chloride aqueous solution (10 mL) for quenching, and then extracted with ethyl acetate (15 mL × 3). Organic phases were combined, washed with a saturated saline solution (15 mL), dried with anhydrous sodium sulfate, and then sucked and filtered. The filtrate was concentrated under reduced pressure. A residue was purified by silica gel column chromatography (dichloromethane/methanol (v/v) = 5/1) to obtain a pale yellow solid (650 mg, yield 54.2%).
LCMS (ESI, m/z): 167.0 [M+H]⁺

### Step 3) 2-cyanoethyl-6-(4-cyano-2-methoxyphenyl)-5-hydroxy-8-methyl-6,9-dihydrothieno[3,2-h][1,6]naphth yridine-7-carboxylate

7-aminothieno[3,2-b]pyridine-5-ol (907 mg, 5.42 mmol), 2-cyanoethyl-2-(4-cyano-2-methoxybenzylidene)-3-oxobutyric acid (359 mg, 1.20 mmol), isopropanol (25 mL) and acetic acid (450 µL) were added into a 50 mL single-mouth bottle at room temperature to react at 90°C overnight after nitrogen replacement, cooled to room temperature, and then sucked and filtered. A solid obtained was pulped with ethyl acetate (15 mL × 3), and a crude product obtained by suction and filtration was purified by C18 reversed-phase column chromatography under the following conditions (C18 BIOTAGE 330 g chromatographic column, mobile phase A (0.1% FA aqueous solution) and mobile phase B (acetonitrile), gradient: increase from 15% B to 50% B for 15 minutes, and detection wavelength 254 nm) to obtain a pale yellow solid (1.09 g, yield 45.4%).
LCMS (ESI, m/z): 447.1 [M+H]⁺

### Step 4) 2-cyanoethyl-6-(4-cyano-2-methoxyphenyl)-5-ethoxy-8-methyl-6,9-dihydrothieno[3,2-h][1,6]naphthyr idine-7-carboxylate

2-cyanoethyl-6-(4-cyano-2-methoxyphenyl)-5-hydroxy-8-methyl-6,9-dihydrothieno[3,2-*h*][1,6]naphth yridine-7-carboxylate (195 mg, 0.437 mmol), methyl iodide (102 mg, 0.655 mmol), silver carbonate (120 mg, 0.437 mmol) and 1,4-dioxane (2 mL) were added into a 50 mL single-mouth bottle at room temperature to react at 90°C for 2 hours after nitrogen replacement, cooled to room temperature, added with saturated ammonium chloride aqueous solution (5 mL) for quenching, and then extracted with ethyl acetate (10 mL × 3 times). Organic phases were combined, washed with a saturated saline solution (10 mL), dried with anhydrous sodium sulfate, sucked and filtered, and then concentrated under reduced pressure. A residue obtained was purified by C18 reversed-phase column chromatography under the following purification conditions: chromatographic column: BIOTAGE C18 reversed-phase column 120 g; mobile phase A: water (containing 0.1% ammonium bicarbonate aqueous solution) and mobile phase B: acetonitrile; flow rate: 60 mL/min; gradient: increase of acetonitrile from 20% to 60% within 15 minutes; and detection wavelength: 254 nm to obtain a white solid (120 mg, yield 57.9%).
LCMS (ESI, m/z): 475.1 [M+H]⁺

### Step 5) 6-(4-cyano-2-methoxyphenyl)-5-ethoxy-8-methyl-6,9-dihydrothieno[3,2-h][1,6]naphthyridine-7-carbo xylic acid

2-cyanoethyl-6-(4-cyano-2-methoxyphenyl)-5-ethoxy-8-methyl-6,9-dihydrothieno[3,2-*h*][1,6]naphthy ridine-7-carboxylate (155 mg, 0.327 mmol), ethylene glycol dimethyl ether (1.5 mL) and an aqueous solution (0.5 mL) of sodium hydroxide (26 mg, 0.655 mmol) were added into a 50 mL single-mouth bottle at room temperature to react at room temperature for 1 hour, added with a hydrochloric acid solution (1.0 mol/L) under ice bath to quench and adjust a PH value to be 5, and then extracted with ethyl acetate (5 mL × 3 times). Organic phases were combined, washed with saturated saline (15 mL × 3 times), dried with anhydrous sodium sulfate, sucked and filtered, and then concentrated under reduced pressure. A residue obtained was purified by preparative high-performance liquid chromatography under the following purification conditions: chromatographic column: C18 silica gel column; mobile phase A: water (containing 0.1% ammonia water) and mobile phase B: acetonitrile; flow rate: 60 mL/min; gradient: increase of acetonitrile from 10% to 40% within 10 minutes; and detection wavelength: 254 nm. The product was collected, and freeze-dried under reduced pressure to obtain a white solid (111 mg, yield 80.6%)
LCMS (ESI, m/z): 422.1 [M+H]⁺

### Step 6) 6-(4-cyano-2-methoxyphenyl)-5-ethoxy-8-methyl-6,9-dihydrothieno[3,2-h][1,6]naphthyridine-7-form amide

6-(4-cyano-2-methoxyphenyl)-5-ethoxy-8-methyl-6,9-dihydrothieno[3,2-*h*][1,6]naphthyridine-7-carbo xylic acid (105 mg, 0.249 mmol), *N*,*N*-diisopropylethylamine (97 mg, 0.747 mmol), *N*,*N*-dimethylformamide (2 mL) and 2-(7-azobenzotriazole)-*N,N,N',N'*-tetramethylurea hexafluorophosphate (284 mg, 0.747 mmol) were added into an 8 mL bottle at room temperature to react at room temperature for a half hour. The reaction solution was added with an ammonia water (0.8 mL) solution at room temperature to react at room temperature for 3 hours, added with water (10 mL) for quenching, and then extracted with ethyl acetate (10 mL × 3 times). Organic phases were combined, washed with a saturated saline solution (10 mL), dried with anhydrous sodium sulfate, sucked and filtered, and then concentrated under reduced pressure. A residue obtained was purified by preparative high-performance liquid chromatography under the following purification conditions: chromatographic column: Sunfire prep C18; mobile phase A: water (containing 0.1% ammonium bicarbonate aqueous solution) and mobile phase B: acetonitrile; flow rate: 60 mL/min; gradient: increase of acetonitrile from 18% to 50% within 7 minutes; and detection wavelength: 254 nm. The product was collected, and freeze-dried under reduced pressure to obtain a white solid (47.0 mg, yield 44.9%).
LCMS (ESI, m/z): 421.0 [M+H]⁺

¹H NMR (400 MHz, DMSO-d6) δ 8.83 (s, 1H), 7.88 *(d, J=* 5.4 Hz, 1H), 7.38 (d, *J* = 1.5 Hz, 1H), 7.29 - 7.22 (m, 2H), 7.18 *(d, J=* 7.9 Hz, 1H), 6.88 - 6.73 (m, 2H), 5.48 (s, 1H), 4.18 - 4.04 (m, 2H), 3.82 (s, 3H), 2.19 (s, 3H), 1.10 (t, J = 7.0 Hz, 3H).

### Example 14 4-(7-cyanobenzo[d][1,3]dioxacyclopenten-4-yl)-5-ethoxy-2,8-dimethyl-1,4-dihydro-1,6-naphthyri dine-3-formamide

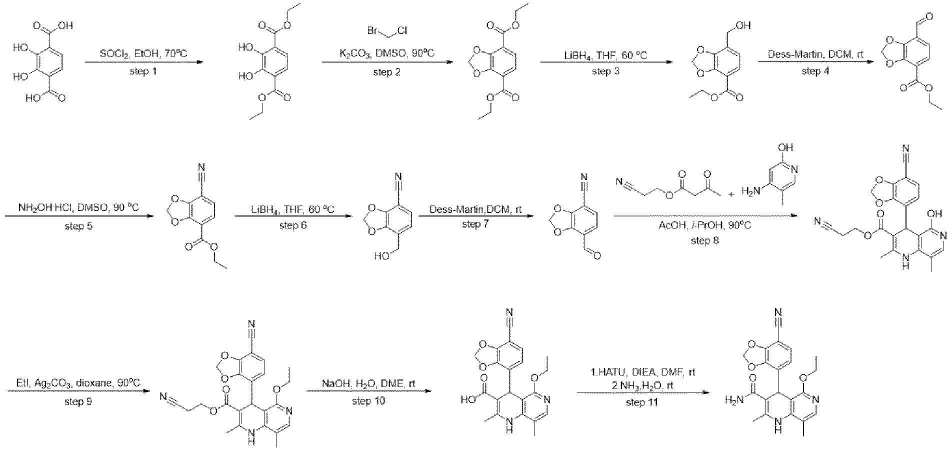

### Step 1) Diethyl 2,3-dihydroxyterephthalate

2,3-dihydroxyterephthalic acid (260 g, 1.31 mol) and ethanol (2.60 L) were added into a 10 L four-mouth bottle at room temperature, slowly dropwise added with dichlorosulfoxide (908 g, 7.63 mol) at 0°C to react at 70°C overnight, and then cooled to room temperature. The reaction solution was slowly dropwise added with a saturated sodium bicarbonate aqueous solution (5.20 L) for quenching, and then extracted with ethyl acetate (5.00 L × 3). Organic phases were combined, washed with a saturated saline solution (3.00 L), dried with anhydrous sodium sulfate, sucked and filtered, and then concentrated under reduced pressure to obtain a greyish white solid (312 g, yield 93.5%).
MS (ESI) M/Z: 255.1 [M+H]⁺

### Step 2) Diethyl benzo[d][1,3]dioxacyclopentene-4,7-dicarboxylate

Diethyl 2,3-dihydroxyterephthalate (312 g, 1.23 mol), bromochloromethane (175 g, 1.35 mol), potassium carbonate (340 g, 2.46 mol) and DMSO (1.90 L) were added into a 10 L four-mouth bottle at room temperature to react at 90°C overnight after nitrogen replacement, cooled to room temperature, added with a saturated saline solution (19.0 L) for quenching, and then extracted with ethyl acetate (12.0 L × 3). Organic phases were combined, washed with a saturated saline solution (12.0 L), dried with anhydrous sodium sulfate, and then sucked and filtered. The filtrate was concentrated under reduced pressure to obtain a greyish white solid (289 g, yield 88.4%).
MS (ESI) M/Z: 267.1 [M+H]⁺

### Step 3) Ethyl 7-hydroxymethylbenzo[d][1,3]dioxacyclopentene-4-carboxylate

Diethyl benzo[d][1,3]dioxacyclopentene-4,7-dicarboxylate (289 g, 1.09 mol), tetrahydrofuran (2.90 L) and a tetrahydrofuran solution (545 mL, 2.0 mol/L, 1.09 mol) of lithium borohydride were added into a 10 L four-mouth bottle at room temperature to react at 60°C for 2 hours after nitrogen replacement, and then cooled to 0°C. The reaction solution was added with a saturated ammonium chloride aqueous solution (6.00 L) for quenching, and then extracted with ethyl acetate (4.50 L × 3). Organic phases were combined, washed with a saturated saline solution (4.50 L), dried with anhydrous sodium sulfate, and then sucked and filtered. The filtrate was concentrated under reduced pressure. A residue was purified by column chromatography (petroleum ether/ethyl acetate (v/v) = 7: 3) to obtain a white solid (168 g, yield 69.0%).
MS (ESI) M/Z: 225.0 [M+H]⁺

### Step 4) Ethyl 7-formylbenzo[d][1,3]dioxacyclopentene-4-carboxylate

Ethyl 7-hydroxymethylbenzo[*d*][1,3]dioxacyclopentene-4-carboxylate (168 g, 750 mmol), dichloromethane (1.70 L) and Dess-Martin periodinane (397 g, 937 mmol) were added into a 3 L three-mouth bottle at room temperature to react at room temperature overnight after nitrogen replacement, and then concentrated under reduced pressure. A residue was purified by column chromatography (petroleum ether/ethyl acetate (v/v) = 1: 1) to obtain a yellow solid (165 g, yield 99.1%).
MS (ESI) M/Z: 223.1 [M+H]⁺

### Step 5) Ethyl 7-cyanobenzo[d][1,3]dioxol-4-carboxylate

Ethyl 7-formylbenzo[*d*][1,3]dioxacyclopentene-4-carboxylate (165 g, 757 mmol), hydroxylamine hydrochloride (158 g, 2.27 mmol) and DMSO (1.00 L) were added into a 3 L three-mouth bottle at room temperature, heated to 90°C to react for 1 hour after nitrogen replacement, cooled to room temperature, added with a saturated saline solution (10.0 L) for quenching, and then extracted with ethyl acetate (6.00 L × 3). Organic phases were combined, washed with a saturated saline solution (6.00 L), dried with anhydrous sodium sulfate, and then sucked and filtered. The filtrate was concentrated under reduced pressure. A residue was purified by column chromatography (petroleum ether/ethyl acetate (v/v) = 4: 1) to obtain a yellow solid (120 g, yield 73.7%).

### Step 6) 7-hydroxymethylbenzo[d][1,3]dioxol-4-carbonitrile

Ethyl 7-cyanobenzo[*d*][1,3]dioxol-4-carboxylate (120 g, 548 mmol), tetrahydrofuran (1.20 L) and a tetrahydrofuran solution (411 mL, 2.0 mol/L, 822 mmol) of lithium borohydride were added into a 3 L three-mouth bottle at room temperature to react at 60°C for 2 hours after nitrogen replacement, and then cooled to 0°C. The reaction solution was added with a saturated ammonium chloride aqueous solution (3.20 L) for quenching, and then extracted with ethyl acetate (2.50 L × 3). Organic phases were combined, washed with a saturated saline solution (2.50 L), dried with anhydrous sodium sulfate, and then sucked and filtered. The filtrate was concentrated under reduced pressure. A residue was purified by column chromatography (petroleum ether/ethyl acetate (v/v) = 5: 1) to obtain a white solid (75.0 g, yield 77.3%).

### Step 7) 7-formylbenzo[1,3]dioxol-4-carbonitrile

7-hydroxymethylbenzo[d][1,3]dioxol-4-carbonitrile (75.0 g, 424 mmol), dichloromethane (1.50 L) and Dess-Martin periodinane (270 g, 636 mmol) were added into a 3 L three-mouth bottle at room temperature to react at room temperature overnight after nitrogen replacement, and then concentrated under reduced pressure. A residue was purified by column chromatography (petroleum ether/ethyl acetate (v/v) = 3: 1) to obtain a yellow solid (67.0 g, yield 90.4%).

### Step 8) 2-cyanoethyl 4-(7-cyanobenzo[d][1,3]dioxol-4-yl)-5-hydroxy-2,8-dimethyl-1,4-dihydro-1,6-naphthyridine-3-carbox ylate

7-formylbenzo[1,3]dioxol-4-carbonitrile (65.0 g, 371 mmol), 2-cyanoethyl-3-oxobutanoate (63.3 g, 408 mmol), 4-amino-5-methylpyridine-2-ol (50.7 g, 408 mmol), isopropanol (1.30 L) and acetic acid (23.4 mL) were added into a 3 L three-mouth bottle at room temperature to react at 90°C overnight after nitrogen replacement, cooled to room temperature, and then sucked and filtered. A solid obtained was pulped with methyl tert-butyl ether (500 mL × 3), and then sucked and filtered to obtain a pale yellow solid (88.0 g, 56.7%).
MS (ESI) M/Z: 419.2 [M+H]⁺

### Step 9) 2-cyanoethyl 4-(7-cyanobenzo[d][1,3]dioxol-4-yl)-5-ethoxy-2,8-dimethyl-1,4-dihydro-1,6-naphthyridine-3-carboxyl ate

2-cyanoethyl 4-(7-cyanobenzo[*d*][1,3]dioxol-4-yl)-5-hydroxy-2,8-dimethyl-1,4-dihydro-1,6-naphthyridine-3-carboxylate (88.0 g, 210 mmol), ethyl iodide (49.3 g, 316 mmol), silver carbonate (58.1 g, 210 mmol) and 1,4-dioxane (880 mL) were added into a 2 L three-mouth bottle at room temperature to react at 90°C for 2 hours after nitrogen replacement, cooled to room temperature, and then sucked and filtered. The filtrate was concentrated under reduced pressure to obtain a yellow solid (86.5 g, 92.1%).
MS (ESI) M/Z: 447.1 [M+H]⁺

### Step 10) 4-(7-cyanobenzo[d][1,3]dioxol-4-yl)-5-ethoxy-2,8-dimethyl-1,4-dihydro-1,6-naphthyridine-3-carboxyli c acid

2-cyanoethyl 4-(7-cyanobenzo[*d*][1,3]dioxol-4-yl)-5-ethoxy-2,8-dimethyl-1,4-dihydro-1,6-naphthyridine-3-carboxylate (85.0 g, 190 mmol), ethylene glycol dimethyl ether (1275 mL), water (425 mL) and an aqueous solution (1.00 mol/L, 380 mL) of sodium hydroxide were added into a 3 L three-mouth bottle at room temperature to react at room temperature for 1 hour, added with water (1.50 L) for dilution, and then extracted with ethyl acetate (1.00 L × 1 time). A water phase was retained, added with a hydrochloric acid solution (1.0 mol/L) under ice bath to quench and adjust a PH value to be 5, and then extracted with ethyl acetate (1.00 L × 3 times). Organic phases were combined, washed with a saturated saline solution (500 mL × 1 time), dried with anhydrous sodium sulfate, and then sucked and filtered. The filtrate was concentrated under reduced pressure to obtain a pale yellow solid (70.0 g, 91.8%), which was directly used in the reaction of the next step.
MS (ESI) M/Z: 394.2 [M+H] ⁺

### Step 11) 4-(7-cyanobenzo[d][1,3]dioxol-4-yl)-5-ethoxy-2,8-dimethyl-1,4-dihydro-1,6-naphthyridine-3-formami de

4-(7-cyanobenzo[*d*][1,3]dioxol-4-yl)-5-ethoxy-2,8-dimethyl-1,4-dihydro-1,6-naphthyridine-3-carboxy lic acid (57.0 g, 145 mmol), *N*,*N*-diisopropylethylamine (37.4 g, 290 mmol), *N*,*N*-dimethylformamide (570 mL) and 2-(7-azobenzotriazole)-*N,N,N',N'*-tetramethylurea hexafluorophosphate (92.4 g, 218 mmol) were added into a 2 L three-mouth bottle at room temperature. The reaction solution was added with an aqueous solution (25%) (120 mL) of ammonia at room temperature to react at room temperature for 1.5 hours. The reaction solution was purified by preparative high-performance liquid chromatography under the following purification conditions: chromatographic column: DAC prep C18; mobile phase A: water (containing 0.1% ammonium bicarbonate) and mobile phase B: acetonitrile; flow rate: 1 L/min; gradient: increase of acetonitrile from 20% to 42% within 30 minutes; and detection wavelength: 254 nm. The product was collected, and freeze-dried under reduced pressure to obtain a white solid (38.0 g, 66.8%).
MS (ESI) M/Z: 393.3 [M+H] ⁺

¹H NMR (400 MHz, Chloroform-d) δ 7.68 (s, 1H), 6.91 (d, *J =* 8.4 Hz, 1H), 6.78 (d, *J =* 8.4 Hz, 1H), 6.14 - 6.12 (m, 2H), 5.79 (s, 1H), 5.43 (s, 2H), 5.16 (s, 1H), 4.26 - 4.16 (m, 2H), 2.44 (s, 3H), 2.14 (s, 3H), 1.26 (t, *J =* 7.0 Hz, 3H).

### Example 15 4-(4-cyano-2,3-dihydrobenzofuran-7-yl)-5-cyclopentoxy-2,8-dimethyl-1,4-dihydro-1,6-naphthyri dine-3-formamide

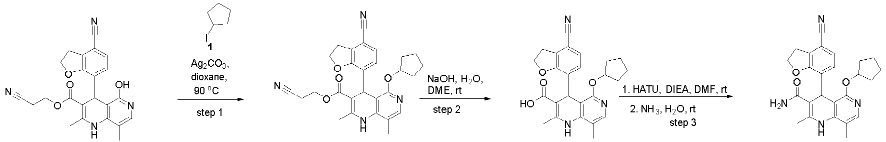

### Step 1) 2-cyanoethyl-4-(4-cyano-2,3-dihydro-1-benzofuran-7-yl)-5-cyclopentoxy-2,8-dimethyl-1,4-dihydro-1,6 -naphthyridine-3-carboxylate

2-cyanoethyl-4-(4-cyano-2,3-dihydro-1-benzofuran-7-yl)-5-hydroxy-2,8-dimethyl-1,4-dihydro-1,6-na phthyridine-3-carboxylate (100 mg, 0.240 mmol), iodocyclopentane (71 mg, 0.360 mmol), silver carbonate (66 mg, 0.240 mmol) and 1,4-dioxane (3 mL) were added into an 8 mL single-mouth bottle at room temperature to react at 90°C for 2 hours after nitrogen replacement, and then cooled to room temperature. The reaction solution was filtered, the fitrate was collected, and the filter cake was washed with ethyl acetate (3× 10 mL). The filtrate was concentrated under reduced pressure to obtain a yellow solid (110.0 mg, yield 94.5%).
MS (ESI) M/Z: 485.5 [M+H]⁺

### Step 2) 4-(4-cyano-2,3-dihydrobenzofuran-7-yl)-5-cyclopentoxy-2,8-dimethyl-1,4-dihydro-1,6-naphthyridine-3-carboxylic acid

2-cyanoethyl-4-(4-cyano-2,3-dihydro-1-benzofuran-7-yl)-5-cyclopentoxy-2,8-dimethyl-1,4-dihydro-1, 6-naphthyridine-3-carboxylate (110 mg, 0.227 mmol), ethylene glycol dimethyl ether (3 mL) and an aqueous solution (1 mL) of sodium hydroxide (18 mg, 0.454 mmol) were added into an 8 mL single-mouth bottle at room temperature to react at room temperature for 1 hour, added with a hydrochloric acid solution (1.0 mol/L) under ice bath to quench and adjust a PH value to be 5, and then extracted with ethyl acetate (5 mL × 3 times). Organic phases were combined, washed with saturated saline (5 mL × 3 times), dried with anhydrous sodium sulfate, sucked and filtered, and then concentrated under reduced pressure to obtain a pale yellow solid (100 mg, yield 93.6%), which was directly used in the reaction of the next step.
MS (ESI) M/Z: 432.5 [M+H]⁺

### Step 3) 4-(4-cyano-2,3-dihydrobenzofuran-7-yl)-5-cyclopentoxy-2,8-dimethyl-1,4-dihydro-1,6-naphthyridine-3-formamide

4-(4-cyano-2,3-dihydrobenzofuran-7-yl)-5-cyclopentoxy-2,8-dimethyl-1,4-dihydro-1,6-naphthyridine-3-carboxylic acid (90 mg, 0.209 mol), *N*,*N*-diisopropylethylamine (54 mg, 0.418 mmol), *N*,*N*-dimethylformamide (1.5 mL) and 2-(7-azobenzotriazole)-*N,N,N',N'*-tetramethylurea hexafluorophosphate (127 mg, 0.334 mmol) were added into an 8 mL bottle at room temperature to react at room temperature for a half hour. The reaction solution was added with an aqueous solution (25%) (1.5 mL) of ammonia at room temperature to react at room temperature for a half hour, and then filtered. The filtrate was purified by preparative high-performance liquid chromatography under the following purification conditions: chromatographic column: YMC-Actus Triart C18; 30*150 mm, 5 µm; mobile phase A: water (containing 0.1% formic acid) and mobile phase B: acetonitrile; flow rate: 60 mL/min; gradient: increase of acetonitrile from 38% to 55% within 7 minutes; and detection wavelength: 254/220 nm. The product was collected, and freeze-dried under reduced pressure to obtain a white solid (50.0 mg, yield 55.7%).
LCMS (ESI, m/z): 431.1 [M+H]⁺

¹H NMR (400 MHz, DMSO-*d*₆) δ 7.67 (s, 1H), 7.56 (s, 1H), 7.12 (d, *J =* 8.0 Hz, 1H), 6.95 (d, *J =* 8.0 Hz, 1H), 6.72 (s, 2H), 5.17 (s, 1H), 5.12 (s, 1H), 4.62-4.52 (m, 2H), 3.50-3.36 (m, 2H), 2.17 (s, 3H), 2.11 (s, 3H), 1.80-1.18 (m, 8H).

### Example 16 4-(4-cyano-2,3-dihydrobenzofuran-7-yl)-5-(3,3-difluorocyclobutyl)methoxy)-2,8-dimethyl-1,4-dih ydro-1,6-naphthyridine-3-formamide

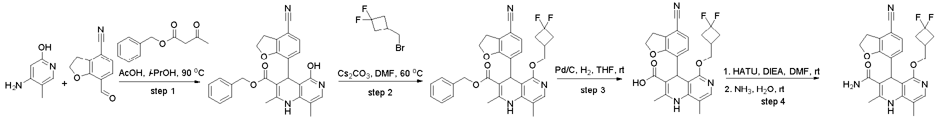

### Step 1) 4-(4-cyano-2,3-dihydro-1-benzofuran-7-yl)-5-hydroxy-2,8-dimethyl-1,4-dihydro-1,6-naphthyridine-3-carboxylate

7-formyl-2,3-dihydro-1-benzofuran-4-carbonitrile (300 mg, 1.73 mmol), benzyl acetoacetate (333 mg, 1.73 mmol), 4-amino-5-methylpyridine-2-ol (215 mg, 1.73 mmol), isopropanol (8.5 mL) and acetic acid (0.15 mL) were added into an 8 mL single-mouth bottle at room temperature to react at 90°C overnight after nitrogen replacement, cooled to room temperature, and then concentrated under reduced pressure. A residue obtained was purified by silica gel column chromatography (with 100% ethyl acetate) to obtain a white solid (185 mg, yield 23.6%).
MS (ESI) M/Z: 454.5 [M+H]⁺

### Step 2) 4-(4-cyano-2,3-dihydro-1-benzofuran-7-yl)-5-[(3,3-difluorocyclobutyl)methoxy]-2,8-dimethyl-1,4-dihy dro-1,6-naphthyridine-3-carboxylate

4-(4-cyano-2,3-dihydro-1-benzofuran-7-yl)-5-hydroxy-2,8-dimethyl-1,4-dihydro-1,6-naphthyridine-3-carboxylate (110 mg, 0.240 mmol), 3-bromomethyl-1,1-difluorocyclobutane (67 mg, 0.360 mmol), cesium carbonate (158 mg, 0.490 mmol) and N,N-dimethylformamide (1 mL) were added into an 8 mL single-mouth bottle at room temperature to react at 60°C for 2 hours, cooled to room temperature, added with water (10 mL) for quenching, and then extracted with ethyl acetate (10 mL × 3 times). Organic phases were combined, washed with a saturated saline solution (10 mL), dried with anhydrous sodium sulfate, sucked and filtered, and then concentrated under reduced pressure. A residue obtained was purified (with petroleum ether/ethyl acetate = 5/1) by a preparative chromatoplate (pre-TLC) to obtain a white solid (40 mg, yield 29.6%).
MS (ESI) M/Z: 558.6 [M+H]⁺

### Step 3) 4-(4-cyano-2,3-dihydro-1-benzofuran-7-yl)-5-[(3,3-difluorocyclobutyl)methoxy]-2,8-dimethyl-1,4-dihy dro-1,6-naphthyridine-3-carboxylic acid

4-(4-cyano-2,3-dihydro-1-benzofuran-7-yl)-5-[(3,3-difluorocyclobutyl)methoxy]-2,8-dimethyl-1,4-dih ydro-1,6-naphthyridine-3-carboxylate (100 mg, 0.180 mmol), palladium carbon (10%, 100 mg) and tetrahydrofuran (2 mL) were added into a 30 mL high-pressure kettle inner container at room temperature, introduced with hydrogen at 3 standard atmospheric pressures after nitrogen replacement to react at room temperature overnight, and then sucked and filtered to obtain a filtrate. The filtrate was concentrated under reduced pressure to obtain a pale yellow solid (30 mg, yield 35.8%), which was directly used in the reaction of the next step.
MS (ESI) M/Z: 468.5 [M+H]⁺

### Step 4) 4-(4-cyano-2,3-dihydrobenzofuran-7-yl)-5-(3,3-difluorocyclobutyl)methoxy)-2,8-dimethyl-1,4-dihydro -1,6-naphthyridine-3-formamide

4-(4-cyano-2,3-dihydro-1-benzofuran-7-yl)-5-[(3,3-difluorocyclobutyl)methoxy]-2,8-dimethyl-1,4-dih ydro-1,6-naphthyridine-3-carboxylic acid (30 mg, 0.064 mmol), *N*,*N*-diisopropylethylamine (17 mg, 0.130 mmol), *N*,*N*-dimethylformamide (0.5 mL) and 2-(7-azobenzotriazole)-*N,N,N',N'*-tetramethylurea hexafluorophosphate (37 mg, 0.096 mmol) were added into an 8 mL bottle at room temperature to react at room temperature for a half hour. The reaction solution was added with an aqueous solution (25%) (0.2 mL) of ammonia at room temperature to react at room temperature for 1 hour, and then sucked and filtered. The filtrate obtained was purified by preparative high-performance liquid chromatography under the following purification conditions: column model: XBridge Shield RP18 OBD Column, 30*150 mm, 5 µm; mobile phase A: water (containing 0.1% ammonia water), and mobile phase B: acetonitrile; flow rate: 60 mL/min; gradient: increase from 25% B to 60% B to elute for 7 minutes; detection wavelength: 254/220 nm; and peak appearance time (min): 6.58. A fraction was collected, and freeze-dried under reduced pressure to obtain a white solid (17.0 mg, yield 56.8%).
LCMS (ESI, m/z): 467.5 [M+H]⁺

¹H NMR (400 MHz, CDCl₃) δ 7.73 (s, 1H), 7.56 (s, 1H), 7.12 (d, *J =* 8.0 Hz, 1H), 6.96 (d, *J =* 8.0 Hz, 1H), 5.17 (s, 1H), 4.66 - 4.50 (m, 2H), 4.17 - 4.04 (m, 2H), 2.47 - 2.12 (m, 13H).

¹⁹F NMR (376 MHz, CDCl₃) δ -83.5, -94.4.

### Example 17 4-(4-cyano-2,3-dihydrobenzofuran-7-yl)-5-(difluoromethoxy)-2,8-dimethyl-1,4-dihydro-1,6-naph thyridine-3-formamide

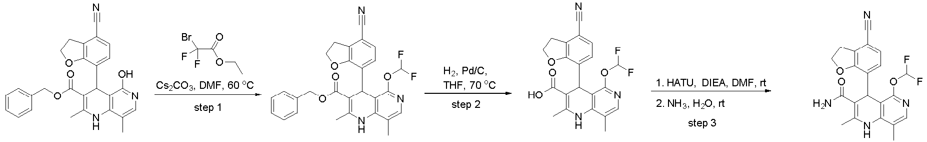

### Step 1) 4-(4-cyano-2,3-dihydrobenzofuran-7-yl)-5-(difluoromethoxy)-2,8-dimethyl-1,4-dihydro-1,6-naphthyri dine-3-carboxylate

4-(4-cyano-2,3-dihydro-1-benzofuran-7-yl)-5-hydroxy-2,8-dimethyl-1,4-dihydro-1,6-naphthyridine-3-carboxylate (100 mg, 0.221 mmol), 2-bromo-2,2-difluoroethyl acetate (67 mg, 0.332 mmol), cesium carbonate (144 mg, 0.442 mmol and *N*,*N*-dimethylformamide (2 mL) were added into an 8 mL single-mouth bottle at room temperature to react at 60°C for 2 hours, cooled to room temperature, added with water (20 mL) for quenching, and then extracted with ethyl acetate (20 mL × 3 times). Organic phases were combined, washed with a saturated saline solution (20 mL), dried with anhydrous sodium sulfate, sucked and filtered, and then concentrated under reduced pressure. A residue obtained was purified (with petroleum ether/ethyl acetate = 5/1) by a preparative chromatoplate (pre-TLC) to to obtain a yellow solid (60 mg, yield 54.6%).
MS (ESI) M/Z: 504.6 [M+H]⁺

### Step 2) 4-cyano-2,3-dihydrobenzofuran-7-yl-5-(difluoromethoxy)-2,8-dimethyl-1,4-dihydro-1,6-naphthyridin e-3-carboxylic acid

4-(4-cyano-2,3-dihydrobenzofuran-7-yl)-5-(difluoromethoxy)-2,8-dimethyl-1,4-dihydro-1,6-naphthyri dine-3-carboxylate (60 mg, 0.119 mmol), palladium carbon (10%, 60 mg) and tetrahydrofuran (2 mL) were added into a 30 mL high-pressure kettle inner container at room temperature, introduced with hydrogen at 3 standard atmospheric pressures after nitrogen replacement to react at 70°C overnight, and then cooled to room temperature. The reaction solution was sucked and filtered to obtain a filtrate. The filtrate was concentrated under reduced pressure to obtain a pale yellow solid (30 mg, yield 60.9%), which was directly used in the reaction of the next step.
MS (ESI) M/Z: 414.4 [M+H]⁺

### Step 3) 4-(4-cyano-2,3-dihydrobenzofuran-7-yl)-5-(difluoromethoxy)-2,8-dimethyl-1,4-dihydro-1,6-naphthyri dine-3-formamide

4-cyano-2,3-dihydrobenzofuran-7-yl-5-(difluoromethoxy)-2,8-dimethyl-1,4-dihydro-1,6-naphthyridin e-3-carboxylic acid (20 mg, 0.048 mmol), *N*,*N*-diisopropylethylamine (13 mg, 0.096 mmol), *N*,*N*-dimethylformamide (0.3 mL) and 2-(7-azobenzotriazole)-N,N,N',N'-tetramethylurea hexafluorophosphate (28 mg, 0.072 mmol) were added into an 8 mL bottle at room temperature to react at room temperature for a half hour. The reaction solution was added with an aqueous solution (25%) (0.2 mL) of ammonia at room temperature to react at room temperature for 1 hour. and then sucked and filtered. The filtrate obtained was purified by preparative high-performance liquid chromatography under the following purification conditions: column model: XBridge Shield RP18 OBD Column, 30*150 mm, 5 µm; mobile phase A: water (containing 0.1% ammonia water), and mobile phase B: acetonitrile; flow rate: 60 mL/min; gradient: increase from 30% B to 60% B to elute for 7 minutes; and detection wavelength: 254/220 nm. A fraction was collected, and freeze-dried under reduced pressure to obtain a white solid (1.1 mg, yield 5.51%).
LCMS (ESI, m/z): 413.05 [M+H]⁺

¹H NMR (300 MHz, CDCl₃) δ 7.68 (s, 1H), 7.05 - 6.99 (m, 2H), 5.87 (s, 1H), 5.23 (s, 1H), 4.75 (t, *J =* 8.7 Hz, 2H), 3.43 (t, *J =* 8.7 Hz, 2H), 2.51 (s, 3H), 2.19 (s, 3H).

¹⁹F NMR (282 MHz, CDCl₃) δ -87.2, -90.2.

### Example 18 4-(4-cyano-2,3-dihydrobenzofuran-7-yl)-5-cyclopropoxy-2,8-dimethyl-1,4-dihydro-1,6-naphthyri dine-3-formamide

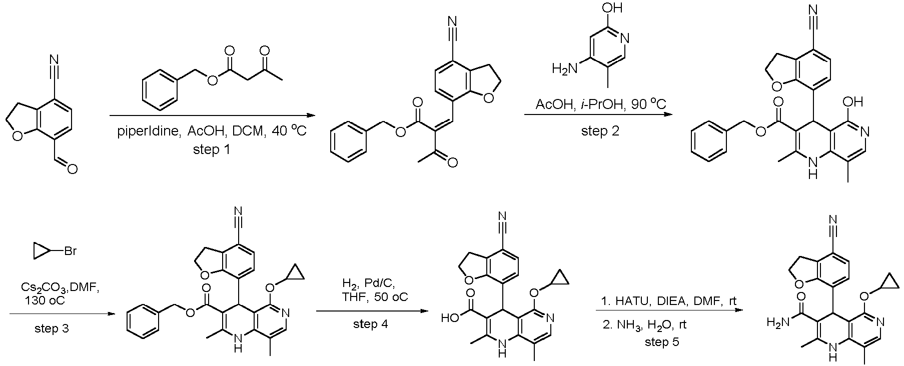

### Step 1) (Z)-2-(4-cyano-2,3-dihydro-1-benzofuran-7-yl)methylene)-3-hydroxybenzyl butyrate

7-formyl-2,3-dihydro-1-benzofuran-4-carbonitrile (566 mg, 3.27 mmol), piperidine (28 mg, 0.327 mmol), acetic acid (0.22 mL), benzyl acetoacetate (628 mg, 3.27 mmol) and dichloromethane (12.0 mL) were added into a 40 mL single-mouth bottle at room temperature to react at 40°C overnight after nitrogen replacement, cooled to room temperature, and then concentrated under reduced pressure. A residue obtained was purified by silica gel column chromatography (petroleum ether/ethyl acetate (v/v) = 3: 1) to obtain a yellow oil substance (600 mg, yield 52.9%).

LCMS (ESI, m/z): 348.10 [M+H]⁺

### Step 2) 4-(4-cyano-2,3-dihydro-1-benzofuran-7-yl)-5-hydroxy-2,8-dimethyl-1,4-dihydro-1,6-naphthyridine-3-carboxylate

(*Z*)-2-(4-cyano-2,3-dihydro-1-benzofuran-7-yl)methylene)-3-hydroxybenzyl butyrate (100 mg, 0.288 mmol), 4-amino-5-methylpyridine-2-ol (35.7 mg, 0.288 mmol), acetic acid (0.02 mL) and dichloromethane (2.0 mL) were added into a 40 mL single-mouth bottle at room temperature to react at 90°C overnight after nitrogen replacement, cooled to room temperature, and then sucked and filtered. A solid obtained was pulped with isopropanol (2.0 mL × 3), and then sucked and filtered to obtain a pale yellow solid (105 mg, yield 80.8%).

LCMS (ESI, m/z): 454.25 [M+H]⁺

### Step 3) 4-(4-cyano-2,3-dihydrobenzofuran-7-yl)-5-cyclopropoxy-2,8-dimethyl-1,4-dihydro-1,6-naphthyridine-3-carboxylate

4-(4-cyano-2,3-dihydro-1-benzofuran-7-yl)-5-hydroxy-2,8-dimethyl-1,4-dihydro-1,6-naphthyridine-3-carboxylate (200 mg, 0.441 mmol), bromocyclopropane (534 mg, 4.41 mmol), cesium carbonate (719 mg, 2.21 mmol) and *N*,*N*-dimethylformamide (2 mL) were added into an 8 mL single-mouth bottle at room temperature to react during stirring at 130°C overnight under nitrogen protection, cooled to room temperature, and then filtered. The filtrate obtained was purified by a C18 column (mobile phase A: 0.1% formic acid aqueous solution, and mobile phase B: acetonitrile; flow rate: 60 mL/min; increase from 10% to 50% within 10 minutes; and detection wavelength: 254 nm). A fraction was collected and freeze-dried under reduced pressure to obtain a yellow solid (35 mg, yield 16.1%).
MS (ESI) M/Z: 494.2 [M+H]⁺

### Step 4) 4-(4-cyano-2,3-dihydrobenzofuran-7-yl)-5-cyclopropoxy-2,8-dimethyl-1,4-dihydro-1,6-naphthyridine-3-carboxylic acid

4-(4-cyano-2,3-dihydrobenzofuran-7-yl)-5-cyclopropoxy-2,8-dimethyl-1,4-dihydro-1,6-naphthyridine-3-carboxylate (35 mg, 0.071 mmol), palladium carbon (10%, 35 mg) and tetrahydrofuran (2 mL) were added into a 30 mL high-pressure kettle inner container at room temperature, introduced with hydrogen at 3 standard atmospheric pressures after nitrogen replacement to react at 50°C for 3 hours, and then cooled to room temperature. The reaction solution was sucked and filtered to obtain a filtrate. The filtrate was concentrated under reduced pressure to obtain a pale yellow solid (28 mg, yield 97.8%), which was directly used in the reaction of the next step.
MS (ESI) M/Z: 404.2 [M+H]⁺

### Step 5) 4-(4-cyano-2,3-dihydrobenzofuran-7-yl)-5-cyclopropoxy-2,8-dimethyl-1,4-dihydro-1,6-naphthyridine-3-formamide

4-(4-cyano-2,3-dihydrobenzofuran-7-yl)-5-cyclopropoxy-2,8-dimethyl-1,4-dihydro-1,6-naphthyridine-3-carboxylic acid (28 mg, 0.069 mmol), *N*,*N*-diisopropylethylamine (27 mg, 0.207 mmol), *N*,*N*-dimethylformamide (0.5 mL) and 2-(7-azobenzotriazole)-N,N,N',N'-tetramethylurea hexafluorophosphate (42 mg, 0.110 mmol) were added into an 8 mL bottle at room temperature to react at room temperature for a half hour. The reaction solution was added with an aqueous solution (25%) (0.5 mL) of ammonia at room temperature to react at room temperature for 1 hour. The reaction solution was sucked and filtered. The filtrate obtained was purified by a C18 column (mobile phase A: 0.1% ammonium bicarbonate aqueous solution, and mobile phase B: acetonitrile; flow rate: 60 mL/min; increase from 20% to 50% within 10 minutes; and detection wavelength: 254 nm). A fraction was collected and freeze-dried under reduced pressure to obtain a white solid (20.2 mg, yield 72.3%).
LCMS (ESI, m/z): 403.05 [M+H]⁺

¹H NMR (400 MHz, Chloroform-d) δ7.74 (s, 1H), 7.02 - 6.98 (m, 2H), 5.82 (s, 1H), 5.08 (s, 1H), 4.79 - 4.65 (m, 2H), 4.42 - 4.18 (m, 2H), 3.43 (t, *J =* 8.4 Hz, 2H), 2.47 (s, 3H), 2.16 (s, 3H), 0.74 - 0.54 (m, 3H), 0.20 - 0.15 (m, 1H).

### Example 19 4-cyano-2,3-dihydrobenzofuran-7-yl-5-cyclopropylmethoxy-2,8-dimethyl-1,4-dihydro-1,6-napht hyridine-3-formamide

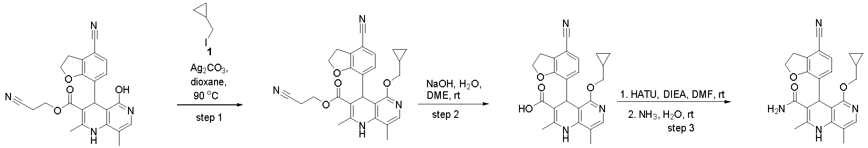

### Step 1) 2-cyanoethyl 4-(4-cyano-2,3-dihydrobenzofuran-7-yl)-5-cyclopropylmethoxy-2,8-dimethyl-1,4-dihydro-1,6-naphth yridine-3-carboxylate

2-cyanoethyl-4-(4-cyano-2,3-dihydro-1-benzofuran-7-yl)-5-hydroxy-2,8-dimethyl-1,4-dihydro-1,6-na phthyridine-3-carboxylate (40 mg, 0.096 mmol), (iodomethyl)cyclopropane (71 mg, 0.360 mmol), silver carbonate (27 mg, 0.096 mmol) and 1,4-dioxane (1 mL) were added into an 8 mL single-mouth bottle at room temperature to react at 90°C for 2 hours after nitrogen replacement, cooled to room temperature, and then concentrated under reduced pressure. A residue was purified by a C18 column (mobile phase A: 0.1% formic acid aqueous solution, and mobile phase B: acetonitrile; flow rate: 60 mL/min; increase from 10% B to 50% B within 10 minutes; and detection wavelength: 254 nm). A fraction was collected and freeze-dried under reduced pressure to obtain a yellow solid (22.5 mg, yield 49.8%).
MS (ESI) M/Z: 471.2 [M+H]⁺

### Step 2) 4-cyano-2,3-dihydrobenzofuran-7-yl-5-cyclopropylmethoxy-2,8-dimethyl-1,4-dihydro-1,6-naphthyrid ine-3-carboxylic acid

2-cyanoethyl 4-(4-cyano-2,3-dihydrobenzofuran-7-yl)-5-cyclopropylmethoxy-2,8-dimethyl-1,4-dihydro-1,6-naphthyridin e-3-carboxylate (20 mg, 0.042 mmol), ethylene glycol dimethyl ether (0.6 mL) and an aqueous solution (0.2 mL) of sodium hydroxide (4 mg, 0.084 mmol) were added into an 8 mL single-mouth bottle at room temperature to react at room temperature for 1 hour, added with a hydrochloric acid solution (1.0 mol/L) under ice bath to quench and adjust a PH value to be 5, and then extracted with ethyl acetate (5 mL × 3 times). Organic phases were combined, washed with saturated saline (5 mL × 3 times), dried with anhydrous sodium sulfate, sucked and filtered, and then concentrated under reduced pressure to obtain a pale yellow solid (17 mg, yield 96.9%), which was directly used in the reaction of the next step.
MS (ESI) M/Z: 418.5 [M+H]⁺

### Step 3) 4-cyano-2,3-dihydrobenzofuran-7-yl-5-cyclopropylmethoxy-2,8-dimethyl-1,4-dihydro-1,6-naphthyrid ine-3-formamide

4-cyano-2,3-dihydrobenzofuran-7-yl-5-cyclopropylmethoxy-2,8-dimethyl-1,4-dihydro-1,6-naphthyridi ne-3-carboxylic acid (16 mg, 0.038 mmol), N,N-diisopropylethylamine (15 mg, 0.115 mmol), N,N-dimethylformamide (0.3 mL) and 2-(7-azobenzotriazole)-*N,N,N',N'*-tetramethylurea hexafluorophosphate (23 mg, 0.062 mmol) were added into an 8 mL bottle at room temperature to react at room temperature for a half hour. The reaction solution was added with an aqueous solution (25%) (0.1 mL) of ammonia at room temperature to react at room temperature for a half hour. The reaction solution was filtered. The filtrate was purified by a C18 column (mobile phase A: 0.1% ammonium bicarbonate aqueous solution; mobile phase B: acetonitrile; flow rate: 60 mL/min; increase from 20% B to 50% B within 10 minutes; and detection wavelength: 254 nm). The fraction was collected, and freeze-dried under reduced pressure. The product was collected, and freeze-dried under reduced pressure to obtain a white solid (2.2 mg, yield 12.9%).
LCMS (ESI, m/z): 417.0 [M+H]⁺

¹H NMR (400 MHz, CDCl₃) δ 7.85 (s, 1H), 7.06 - 7.00 (m, 2H), 6.69 (s, 2H), 6.23 (s, 1H), 5.20 (s, 1H), 4.73 (t, *J =* 8.8 Hz, 2H), 4.42 - 4.03 (m, 2H), 3.45 (t, *J =* 8.8 Hz, 2H), 2.50 (s, 3H), 1.13 - 1.08 (m, 1H), 0.61 - 0.50 (m, 2H), 0.31 - 0.15 (m, 2H).

### Example 20 4-(4-cyano-2,3-dihydro-1-benzofuran-7-yl)-5-isopropoxy-2,8-dimethyl-1,4-dihydro-1,6-naphthyri dine-3-formamide

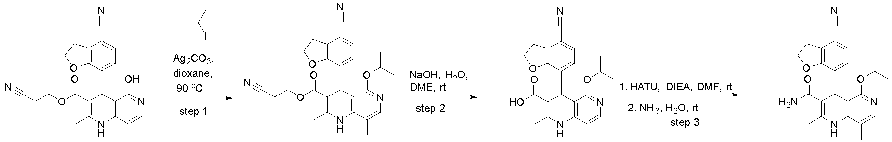

### Step 1) 2-cyanoethyl 4-(4-cyano-2,3-dihydro-1-benzofuran-7-yl)-5-isopropoxy-2,8-dimethyl-1,4-dihydro-1,6-naphthyridine -3-carboxylate

2-cyanoethyl-4-(4-cyano-2,3-dihydro-1-benzofuran-7-yl)-5-hydroxy-2,8-dimethyl-1,4-dihydro-1,6-na phthyridine-3-carboxylate (50 mg, 0.120 mmol), 2-iodopropane (33 mg, 0.192 mmol), silver carbonate (33 mg, 0.120 mmol) and 1,4-dioxane (1 mL) were added into an 8 mL single-mouth bottle at room temperature to react at 90°C for 2 hours after nitrogen replacement, and then cooled to room temperature. The reaction solution was filtered, the filter cake (3 × 5 mL) was rinsed with ethyl acetate, and the filtrate was collected and concentrated and dried under reduced pressure to obtain a white solid (40 mg, yield 72.7%).
MS (ESI) M/Z: 459.2 [M-H]⁻

### Step 2) 4-cyano-2,3-dihydrobenzofuran-7-yl-5-isopropoxy-2,8-dimethyl-1,4-dihydro-1,6-naphthyridine-3-car boxylic acid

2-cyanoethyl 4-(4-cyano-2,3-dihydro-1-benzofuran-7-yl)-5-isopropoxy-2,8-dimethyl-1,4-dihydro-1,6-naphthyridine-3-ca rboxylate (40 mg, 0.087 mmol), ethylene glycol dimethyl ether (0.9 mL) and an aqueous solution (0.3 mL) of sodium hydroxide (7 mg, 0.174 mmol) were added into an 8 mL single-mouth bottle at room temperature to react at room temperature for 1 hour, added with a hydrochloric acid solution (1.0 mol/L) under ice bath to quench and adjust a PH value to be 5, and then extracted with ethyl acetate (3 mL × 3 times). Organic phases were combined, washed with saturated saline (3 mL × 3 times), dried with anhydrous sodium sulfate, sucked and filtered, and then concentrated under reduced pressure to obtain a pale yellow solid (30 mg, yield 84.6%), which was directly used in the reaction of the next step.
MS (ESI) M/Z: 406.3 [M+H]⁺

### Step 3) 4-(4-cyano-2,3-dihydro-1-benzofuran-7-yl)-5-isopropoxy-2,8-dimethyl-1,4-dihydro-1,6-naphthyridine -3-formamide

4-cyano-2,3-dihydrobenzofuran-7-yl-5-isopropoxy-2,8-dimethyl-1,4-dihydro-1,6-naphthyridine-3-car boxylic acid (30 mg, 0.074 mmol), *N*,*N*-diisopropylethylamine (0.05 mL), *N*,*N*-dimethylformamide (0.5 mL) and 2-(7-azobenzotriazole)-*N,N,N',N'*-tetramethylurea hexafluorophosphate (42 mg, 0.111 mmol) were added into an 8 mL bottle at room temperature to react at room temperature for a half hour. The reaction solution was added with an aqueous solution (25%) (0.2 mL) of ammonia at room temperature to react at room temperature for a half hour. The reaction solution was filtered. The filtrate was purified by a C18 column (mobile phase A: an aqueous solution containing 0.05% ammonia water; mobile phase B: acetonitrile; flow rate: 60 mL/min; increase from 15% B to 40% B within 15 minutes; and detection wavelength: 254 nm). The product was collected, and freeze-dried under reduced pressure to obtain a white solid (14.4 mg, yield 48.1%).
LCMS (ESI, m/z): 404.10 [M+H]⁺

¹H NMR (400 MHz, CDCl₃) δ 7.68 (s, 1H), 7.13-6.83 (m, 2H), 6.20 (s, 1H), 5.78 (s, 1H), 5.33- 5.03 (m, 3H), 4.92- 4.53 (m, 2H), 3.43 (t, *J =* 9.2 Hz, 2H), 2.49 (s, 3H), 2.15 (s, 3H), 1.25 (d, *J =* 6.1 Hz, 3H), 0.94 *(d, J=* 6.1 Hz, 3H).

### Example 21 4-(4-cyano-2,3-dihydrobenzofuran-7-yl)-5-cyclobutoxy-2,8-dimethyl-1,4-dihydro-1,6-naphthyrid ine-3-formamide

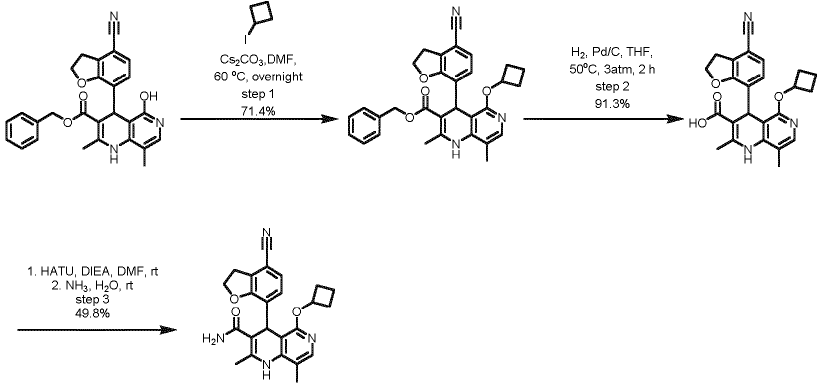

### Step 1) 4-(4-cyano-2,3-dihydrobenzofuran-7-yl)-5-cyclobutoxy-2,8-dimethyl-1,4-dihydro-1,6-naphthyridine-3 -carboxylate

4-(4-cyano-2,3-dihydro-1-benzofuran-7-yl)-5-hydroxy-2,8-dimethyl-1,4-dihydro-1,6-naphthyridine-3-carboxylate (50 mg, 0.110 mmol), iodocyclobutane (60 mg, 0.331 mmol), cesium carbonate (72 mg, 0.221 mmol) and N,N-dimethylformamide (1 mL) were added into an 8 mL single-mouth bottle at room temperature to react during stirring at 60°C overnight under nitrogen protection, The reaction solution was cooled to room temperature, added with water (10 mL) for dilution, and then extracted with ethyl acetate (3 × 10 mL). Organic phases were combined, washed with a saturated saline solution (3× 10 mL), dried with anhydrous sodium sulfate, and then sucked and filtered to obtain a filtrate. The filtrate was concentrated and dried under reduced pressure. A residue obtained was purified (with petroleum ether/ethyl acetate = 4/1) by a preparative chromatoplate (pre-TLC) to obtain a white solid (40 mg, yield 71.4%).
MS (ESI) M/Z: 508.2 [M+H]⁺

### Step 2) 4-(4-cyano-2,3-dihydrobenzofuran-7-yl)-5-cyclobutoxy-2,8-dimethyl-1,4-dihydro-1,6-naphthyridine-3 -carboxylic acid

4-(4-cyano-2,3-dihydrobenzofuran-7-yl)-5-cyclobutoxy-2,8-dimethyl-1,4-dihydro-1,6-naphthyridine-3 -carboxylate (40 mg, 0.085 mmol), palladium carbon (10%, 40 mg) and tetrahydrofuran (2 mL) were added into a 30 mL high-pressure kettle inner container at room temperature, introduced with hydrogen at 3 standard atmospheric pressures after nitrogen replacement to react at 50°C for 2 hours, and then cooled to room temperature. The reaction solution was sucked and filtered to obtain a filtrate. The filtrate was concentrated under reduced pressure to obtain a white solid (30 mg, yield 91.3%), which was directly used in the reaction of the next step.
MS (ESI) M/Z: 418.2 [M+H]⁺

### Step 3) 4-(4-cyano-2,3-dihydrobenzofuran-7-yl)-5-cyclobutoxy-2,8-dimethyl-1,4-dihydro-1,6-naphthyridine-3 -formamide

4-(4-cyano-2,3-dihydrobenzofuran-7-yl)-5-cyclobutoxy-2,8-dimethyl-1,4-dihydro-1,6-naphthyridine-3 -carboxylic acid (30 mg, 0.072 mmol), *N*,*N*-diisopropylethylamine (19 mg, 0.145 mmol), N,N-dimethylformamide (1 mL) and 2-(7-azobenzotriazole)-*N,N,N',N'*-tetramethylurea hexafluorophosphate (41 mg, 0.109 mmol) were added into an 8 mL bottle at room temperature to react at room temperature for a half hour. The reaction solution was added with an aqueous solution (25%) (0.5 mL) of ammonia at room temperature to react at room temperature for 1 hour. The reaction solution was sucked and filtered. The filtrate obtained was purified by preparative high-performance liquid chromatography under the following purification conditions: chromatographic column: Xselect CSH C18 OBD; mobile phase A: water (containing 5 mmol/L ammonium bicarbonate) and mobile phase B: acetonitrile; flow rate: 50 L/min; gradient: increase of acetonitrile from 40% to 50% within 7 minutes; and detection wavelength: 254/220 nm. The product was collected and freeze-dried under reduced pressure to obtain a white solid (15.0 mg, yield 49.8%).
LCMS (ESI, m/z): 417.2 [M+H]⁺

¹H NMR (400 MHz, Chloroform-d) δ 7.66 (s, 1H), 7.10-6.96 (m, 2H), 6.28 (s, 1H), 5.77 (s, 1H), 5.20 (s, 1H), 5.12-5.00 (m, 1H), 4.83-4.66 (m, 2H), 3.52-3.36 (m, 2H), 2.49 (s, 3H), 2.46-2.38 (m, 1H), 2.28 - 2.18 (m, 1H), 2.14 (s, 3H), 2.06-1.95 (m, 1H), 1.76 - 1.64 (m, 3H).

### Example 22 4-cyano-2,3-dihydrobenzofuran-7-yl-5-cyclobutyl-2,8-dimethyl-1,4-dihydro-1,6-naphthyridine-3-formamide

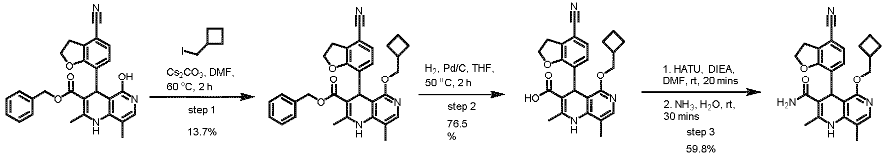

### Step 1) 4-cyano-2,3-dihydrobenzofuran-7-yl-5-cyclobutyl-2,8-dimethyl-1,4-dihydro-1,6-naphthyridine-3-carb oxylate

4-(4-cyano-2,3-dihydro-1-benzofuran-7-yl)-5-hydroxy-2,8-dimethyl-1,4-dihydro-1,6-naphthyridine-3-carboxylate (300 mg, 0.663 mmol), (iodomethyl)cyclobutane (194 mg, 0.996 mmol), cesium carbonate (431 mg, 1.27 mmol) and *N*,*N*-dimethylformamide (3 mL) were added into an 8 mL single-mouth bottle at room temperature to react during stirring at 60°C for 2 hours under nitrogen protection. The reaction solution was cooled to room temperature and filtered. The filtrate was purified by a C18 reversed-phase column (under the following conditions: mobile phase A: water (containing 0.1% formic acid), and mobile phase B: acetonitrile; flow rate: 60 mL/min; gradient: increase from 30% B to 60% B to elute for 15 minutes; detection wavelength: 254 nm; and reference wavelength: 220 nm). The product was collected and concentrated under reduced pressure to obtain a white solid (47 mg, yield 13.7%).
MS (ESI) M/Z: 522.2 [M+H]⁺

### Step 2) 4-cyano-2,3-dihydrobenzofuran-7-yl-5-cyclobutyl-2,8-dimethyl-1,4-dihydro-1,6-naphthyridine-3-carb oxylic acid

4-cyano-2,3-dihydrobenzofuran-7-yl-5-cyclobutyl-2,8-dimethyl-1,4-dihydro-1,6-naphthyridine-3-carb oxylate (47 mg, 0.090 mmol), palladium carbon (10%, 47 mg) and tetrahydrofuran (2 mL) were added into a 30 mL high-pressure kettle inner container at room temperature, introduced with hydrogen at 3 standard atmospheric pressures after nitrogen replacement to react at 50°C for 2 hours, and then cooled to room temperature. The reaction solution was sucked and filtered to obtain a filtrate. The filtrate was concentrated under reduced pressure to obtain a pale yellow solid (30 mg, yield 77.2%), which was directly used in the reaction of the next step.
MS (ESI) M/Z: 432.2 [M+H]⁺

### Step 3) 4-cyano-2,3-dihydrobenzofuran-7-yl-5-cyclobutyl-2,8-dimethyl-1,4-dihydro-1,6-naphthyridine-3-for mamide

4-cyano-2,3-dihydrobenzofuran-7-yl-5-cyclobutyl-2,8-dimethyl-1,4-dihydro-1,6-naphthyridine-3-carb oxylic acid (30 mg, 0.070 mmol), *N*,*N*-diisopropylethylamine (19 mg, 0.145 mmol), *N*,*N*-dimethylformamide (0.5 mL) and 2-(7-azobenzotriazole)-N,N,N',N'-tetramethylurea hexafluorophosphate (42 mg, 0.110 mmol) were added into an 8 mL bottle at room temperature to react at room temperature for a half hour. The reaction solution was added with an aqueous solution (25%) (0.2 mL) of ammonia at room temperature to react at room temperature for 1 hour. The reaction solution was sucked and filtered. The filtrate obtained was purified by preparative high-performance liquid chromatography under the following purification conditions: chromatographic column: Xselect CSH C18 OBD; mobile phase A: water (containing 5 mmol/L ammonium bicarbonate) and mobile phase B: acetonitrile; flow rate: 50 L/min; gradient: increase of acetonitrile from 35% to 50% within 7 minutes; and detection wavelength: 254/220 nm. The product was collected and freeze-dried under reduced pressure to obtain a white solid (17.9 mg, yield 59.8%).
LCMS (ESI, m/z): 431.2 [M+H]⁺

¹H NMR (400 MHz, Chloroform-*d*₃) δ 7.68 (s, 1H), 7.01 (d, *J =* 8.1 Hz, 2H), 6.68 (s, 2H), 5.82 (s, 1H), 5.17 (s, 1H), 4.72 (t, *J =* 8.6 Hz, 2H), 4.17-4.08 (m, 2H), 3.42 (t, *J =* 8.6 Hz, 2H), 2.62-2.52 (m, 1H), 2.48 (s, 3H), 2.16 (s, 3H), 2.06-1.61 (m, 6H).

### Example 23 4-cyano-2,3-dihydrobenzofuran-7-yl-2,8-dimethyl-5-trifluoromethoxy-1,4-dihydro-1,6-naphthyri dine-3-formamide

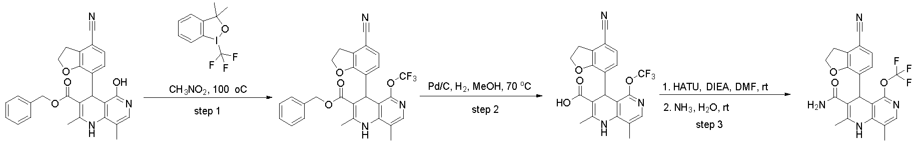

### Step 1) 4-cyano-2,3-dihydrobenzofuran-7-yl-2,8-dimethyl-5-trifluoromethoxy-1,4-dihydro-1,6-naphthyridine -3-carboxylate

4-(4-cyano-2,3-dihydro-1-benzofuran-7-yl)-5-hydroxy-2,8-dimethyl-1,4-dihydro-1,6-naphthyridine-3-carboxylate (100 mg, 0.220 mmol), 3,3-dimethyl-1-(trifluoromethyl)-1,2-benziodoxol (218 mg, 0.663 mmol) and nitromethane (4 mL) were added into an 8 mL single-mouth bottle at room temperature to react during stirring at 100°C for 5 hours under nitrogen protection. The reaction solution was cooled to room temperature, added with water (10 mL) for dilution, and then extracted with ethyl acetate (3 × 10 mL). Organic phases were combined, washed with a saturated saline solution (3× 10 mL), dried with anhydrous sodium sulfate, and then sucked and filtered to obtain a filtrate. The filtrate was concentrated and dried under reduced pressure. A residue obtained was purified (with petroleum ether/ethyl acetate = 1/1) by a preparative chromatoplate (pre-TLC) to obtain a yellow solid (40 mg, yield 34.8%).
MS (ESI) M/Z: 522.2 [M+H]⁺

### Step 2) 4-cyano-2,3-dihydrobenzofuran-7-yl-2,8-dimethyl-5-trifluoromethoxy-1,4-dihydro-1,6-naphthyridine -3-carboxylic acid

4-cyano-2,3-dihydrobenzofuran-7-yl-2,8-dimethyl-5-trifluoromethoxy-1,4-dihydro-1,6-naphthyridine-3-carboxylate (20 mg, 0.038 mmol), palladium carbon (10%, 20 mg) and methanol (2 mL) were added into a 30 mL high-pressure kettle inner container at room temperature, introduced with hydrogen at 3 standard atmospheric pressures after nitrogen replacement to react at 70°C for 4 hours, and then cooled to room temperature. The reaction solution was sucked and filtered to obtain a filtrate. The filtrate was purified by preparative high-performance liquid chromatography under the following purification conditions: chromatographic column: C18 reversed-phase column; mobile phases: water (containing 0.1% formic acid) and acetonitrile; flow rate: 40 mL/min; gradient: increase from 30% B to 50% B within 15 minutes; and detection wavelength: 254 nm. The product was collected, and freeze-dried and concentrated under reduced pressure to obtain a white solid (12 mg, yield 72.5%), which was directly used in the reaction of the next step.
MS (ESI) M/Z: 432.2 [M+H]⁺

### Step 3) 4-(4-cyano-2,3-dihydrobenzofuran-7-yl)-5-cyclobutoxy-2,8-dimethyl-1,4-dihydro-1,6-naphthyridine-3 -formamide

4-cyano-2,3-dihydrobenzofuran-7-yl-2,8-dimethyl-5-trifluoromethoxy-1,4-dihydro-1,6-naphthyridine-3-carboxylic acid (12 mg, 0.028 mmol), *N*,*N*-diisopropylethylamine (7 mg, 0.054 mmol), *N*,*N*-dimethylformamide (0.3 mL) and 2-(7-azobenzotriazole)-*N,N,N',N'*-tetramethylurea hexafluorophosphate (16 mg, 0.042 mmol) were added into an 8 mL bottle at room temperature to react at room temperature for a half hour. The reaction solution was added with an aqueous solution (25%) (0.1 mL) of ammonia at room temperature to react at room temperature for 1 hour. The reaction solution was sucked and filtered. The filtrate obtained was purified by preparative high-performance liquid chromatography under the following purification conditions: chromatographic column: Xselect CSH C18 OBD; mobile phase A: water (containing 0.1% trifluoroformic acid), and mobile phase B: acetonitrile; flow rate: 50 L/min; gradient: increase of acetonitrile from 20% to 50% within 7 minutes; and detection wavelength: 254/220 nm. The product was collected and freeze-dried under reduced pressure to obtain a white solid (2.9 mg, yield 24.2%).
LCMS (ESI, m/z): 431.2 [M+H]⁺

¹H NMR (400 MHz, CDCl₃) δ 7.82 (s, 1H), 7.06-6.98 (m, 2H), 5.97 (s, 1H), 5.22 (s, 1H), 4.78 (t, *J* = 8.7 Hz, 2H), 3.47 (t, *J=* 8.7 Hz, 2H), 2.55 (s, 3H), 2.26 (s, 3H).

¹⁹F NMR (377 MHz, CDCl₃) δ -56.0.

### Example 24 4-cyano-2,3-dihydrobenzofuran-7-yl-2,8-dimethyl-5-(2,2,2-trifluoroethoxy)-1,4-dihydro-1,6-naph thyridine-3-formamide

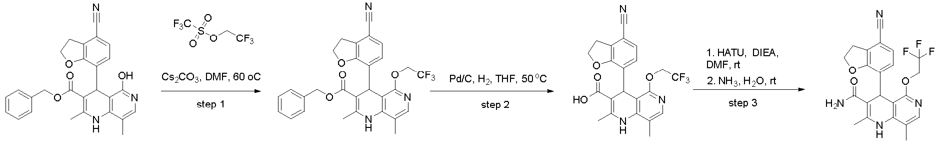

### Step 1) 4-cyano-2,3-dihydrobenzofuran-7-yl-2,8-dimethyl-5-(2,2,2-trifluoroeth oxy)-1,4-dihydro-1,6-naphthyr idine-3-carboxylate

4-(4-cyano-2,3-dihydro-1-benzofuran-7-yl)-5-hydroxy-2,8-dimethyl-1,4-dihydro-1,6-naphthyridine-3-carboxylate (100 mg, 0.221 mmol), 2,2,2-trifluoroethyl trifluoromethanesulfonate (92 mg, 0.354 mmol), cesium carbonate (144 mg, 0.442 mmol) and *N*,*N*-dimethylformamide (2 mL) were added into an 8 mL single-mouth bottle at room temperature to react during stirring at room temperature for 2 hours, added with water (20 mL) for dilution, and then extracted with ethyl acetate (3 × 20 mL). Organic phases were combined, washed with a saturated saline solution (20 mL), dried with anhydrous sodium sulfate, and then sucked and filtered to obtain a filtrate. The filtrate was concentrated and dried under reduced pressure. A residue obtained was purified (with petroleum ether/ethyl acetate = 2/1) by a preparative chromatoplate (pre-TLC) to obtain a yellow solid (36 mg, yield 36.1%).
MS (ESI) M/Z: 536.2 [M+H]⁺

### Step 2) 4-cyano-2,3-dihydrobenzofuran-7-yl-2,8-dimethyl -5-(2,2,2-trifluoroethoxy)-1,4-dihydro-1,6-naphthyridine-3-carboxylic acid

4-cyano-2,3-dihydrobenzofuran-7-yl-2,8-dimethyl-5-(2,2,2-trifluoroethoxy)-1,4-dihydro-1,6-naphthyri dine-3-carboxylate (36.0 mg, 0.067 mmol), palladium carbon (10%, 36 mg) and tetrahydrofuran (2 mL) were added into a 30 mL high-pressure kettle inner container at room temperature, introduced with hydrogen at 3 standard atmospheric pressures after nitrogen replacement to react at 70°C for 3 hours, and then cooled to room temperature. The reaction solution was sucked and filtered to obtain a filtrate. The filtrate was concentrated under reduced pressure to obtain a white solid (24 mg, yield 80.1%), which was directly used in the reaction of the next step.
MS (ESI) M/Z: 446.2 [M+H]⁺

### Step 3) 4-cyano-2,3-dihydrobenzofuran-7-yl-2,8-dimethyl-5-(2,2,2-trifluoroethoxy)-1,4-dihydro-1,6-naphthyr idine-3-formamide

4-cyano-2,3-dihydrobenzofuran-7-yl-2,8-dimethyl-5-(2,2,2-trifluoroethoxy)-1,4-dihydro-1,6-naphthyri dine-3-carboxylic acid (24 mg, 0.054 mmol), *N*,*N*-diisopropylethylamine (19 mg, 0.145 mmol), *N*,*N*-dimethylformamide (0.5 mL) and 2-(7-azobenzotriazole)-*N,N,N',N'*-tetramethylurea hexafluorophosphate (30 mg, 0.081 mmol) were added into an 8 mL bottle at room temperature to react at room temperature for a half hour. The reaction solution was added with an aqueous solution (25%) (0.2 mL) of ammonia at room temperature to react at room temperature for 1 hour. The reaction solution was sucked and filtered. The filtrate obtained was purified by preparative high-performance liquid chromatography under the following purification conditions: chromatographic column: Xselect CSH C18 OBD; mobile phase A: water (containing 5 mmol/L ammonium bicarbonate) and mobile phase B: acetonitrile; flow rate: 50 L/min; gradient: increase of acetonitrile from 40% to 50% within 7 minutes; and detection wavelength: 254/220 nm. The product was collected and freeze-dried under reduced pressure to obtain a white solid (6.3 mg, yield 26.3%).
MS (ESI) M/Z: 445.15 [M+H]⁺

¹H NMR (400 MHz, CDCl₃) δ 7.66 (s, 1H), 7.05- 6.92 (m, 2H), 5.83 (s, 1H), 5.22 (s, 1H), 4.80-4.40 (m, 4H), 3.42 (t, *J =* 8.7 Hz, 2H), 2.52 (s, 3H), 2.23 (s, 3H).
¹⁹F NMR (282 MHz, CDCl₃) δ -74.1.

### Example 25 4-(8-cyano-2,3-dihydrobenzo[b][1,4]dioxin-5-yl)-5-ethoxy-2,8-dimethyl-1,4-dihydro-1,6-naphthy ridine-3-formamide

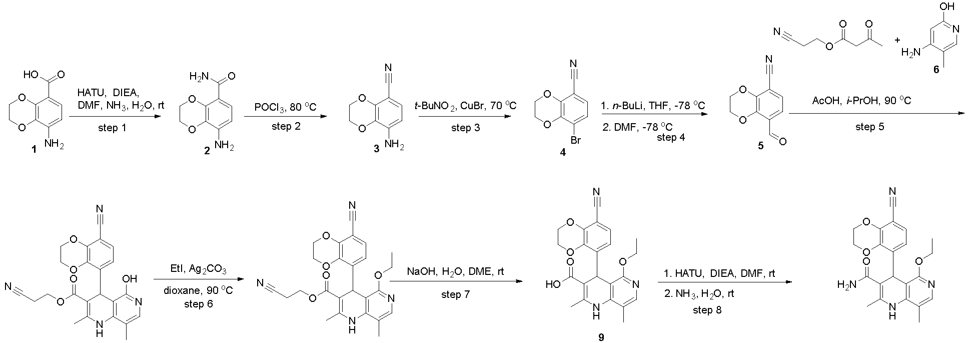

### Step 1) 8-amino-2,3-dihydro-1,4-benzodioxazine-5-formamide

8-amino-2,3-dihydro-1,4-benzodioxazine-5-carboxylic acid (1.00 g, 5.12 mmol), N,N-dimethylformamide (25 mL), N,N-diisopropylethylamine (1.32 g, 10.2 mmol) and 2-(7-azabenzotriazole)-N,N,N',N'-tetramethylurea hexafluorophosphate (2.92 g, 7.69 mmol) were added into a 100 mL single-mouth bottle at room temperature to react at room temperature for 0.5 hour, The reaction solution was added with an aqueous solution (1.0 M, 6 mL) of ammonia at room temperature to react at room temperature for 1 hour, added with water (250 mL) for dilution, and then extracted with ethyl acetate (250 mL × 3). Organic phases were combined, washed with a saturated saline solution (250 mL), dried with anhydrous sodium sulfate, sucked and filtered, and then concentrated under reduced pressure. A residue was purified by silica gel column chromatography (eluent: pure ethyl acetate) to obtain an orange oil substance (800 mg, yield 80.4%).
LCMS (ESI, m/z): 195.1 [M+H]⁺

### Step 2) 8-amino-2,3-dihydro-1,4-benzodioxazine-5-carbonitrile

8-amino-2,3-dihydro-1,4-benzodioxazine-5-formamide (766 mg, 3.95 mmol) and phosphorus oxychloride (15 mL) were added into a 50 mL single-mouth bottle at room temperature, heated to 80°C to react for 1 hour, cooled to room temperature, added with a sodium hydroxide solution (1.0 mol/L) to quench and adjust a pH value to be about 8, and then extracted with ethyl acetate (50 mL × 3). Organic phases were combined, washed with a saturated saline solution (50 mL), dried with anhydrous sodium sulfate, sucked and filtered, and then concentrated under reduced pressure. A residue was purified by column chromatography (dichloromethane/methanol (v/v) = 20/1) to obtain a white solid (310 mg, yield 44.6%).
LCMS (ESI, m/z): 177.1 [M+H]⁺

### Step 3) 8-bromo-2,3-dihydro-1,4-benzodioxazine-5-carbonitrile

8-amino-2,3-dihydro-1,4-benzodioxazine-5-carbonitrile (310 mg, 1.76 mmol), tert-butyl nitrite (181 mg, 1.76 mmol), cuprous bromide (366 mg, 2.55 mmol) and acetonitrile (8 mL) were added into a 40 mL sample bottle at room temperature react at 70°C for 1.5 hours. The reaction solution was cooled to room temperature, sucked and filtered, and rinsed with ethyl acetate (8 mL) for 3 times to obtain a filtrate. The filtrate was concentrated under reduced pressure. A residue was purified by a preparative chromatoplate (petroleum ether/ethyl acetate (v/v) = 5/1) to obtain a white solid (110 mg, yield 26.0%).

### Step 4) 8-formyl-2,3-dihydro-1,4-benzodioxazine-5-carbonitrile

8-bromo-2,3-dihydro-1,4-benzodioxazine-5-carbonitrile (110 mg, 0.458 mmol) and tetrahydrofuran (0.5 mL) were added into an 8 mL sample bottle at room temperature. The reaction solution was cooled to -78°C after nitrogen replacement, slowly dropwise added with an n-hexane solution of n-butyl lithium (0.2 mL, 2.5 mmol/mL, 0.50 mmol) to react at -78°C for a half hour, added with N,N-dimethylformamide (0.5 mL) to react at -78 °C for a half hour, added with a saturated ammonium chloride aqueous solution (5 mL) for quenching, and then extracted with dichloromethane (5 mL × 3). Organic phases were combined, washed with a saturated saline solution (5 mL), dried with anhydrous sodium sulfate, and then sucked and filtered to obtain a filtrate. The filtrate was concentrated under reduced pressure. A residue was purified by a preparative chromatoplate (petroleum ether/ethyl acetate (v/v) = 5/1) to obtain a white solid (40 mg, yield 46.2%).

### Step 5) 2-cyanoethyl 4-(8-cyano-2,3-dihydrobenzo[b][1,4]dioxin-5-yl)-5-hydroxy-2,8-dimethyl-1,4-dihydro-1,6-naphthyridi ne-3-carboxylate

8-formyl-2,3-dihydro-1,4-benzodioxazine-5-carbonitrile (35 mg, 0.185 mmol), 2-cyanoethyl-3-oxobutanoate (29 mg, 0.185 mmol), 4-amino-5-methylpyridine-2-ol (23 mg, 0.185 mmol), isopropanol (0.6 mL) and acetic acid (12 µL) were added into an 8 mL single-mouth bottle at room temperature to react at 90°C for 4 hours after nitrogen replacement, cooled to room temperature, and then concentrated under reduced pressure. A residue obtained was purified by C18 reversed-phase column chromatography under the following conditions (C18 BIOTAGE 40 g reversed-phase column, mobile phase A (0.1% ammonium bicarbonate aqueous solution) and mobile phase B (acetonitrile), gradient: increase from 25% B to 30% B for 15 minutes, and detection wavelength 254 nm) to obtain a pale yellow solid (15 mg, yield 18.8%).
MS (ESI) M/Z: 433.2 [M+H]⁺

### Step 6) 2-cyanoethyl 4-(8-cyano-2,3-dihydrobenzo[b][1,4]dioxin-5-yl)-5-ethoxy-2,8-dimethyl-1,4-dihydro-1,6-naphthyridin e-3-carboxylate

2-cyanoethyl 4-(8-cyano-2,3-dihydrobenzo[*b*][1,4]dioxin-5-yl)-5-hydroxy-2,8-dimethyl-1,4-dihydro-1,6-naphthyridine-3 -carboxylate (15 mg, 0.035 mmol), ethyl iodide (8 mg, 0.053 mmol), silver carbonate (10 mg, 0.035 mmol) and 1,4-dioxane (0.3 mL) were added into an 8 mL single-mouth bottle at room temperature to react at 90°C for 1 hour after nitrogen replacement, cooled to room temperature, sucked and filtered, and then rinsed with acetonitrile (3 mL) to obtain a filtrate. The filtrate was concentrated under reduced pressure to obtain a yellow solid (15 mg, crude product), which was directly used in the reaction of the next step without further purification.
MS (ESI) M/Z: 461.3 [M+H]⁺

### Step 7) 4-(8-cyano-2,3-dihydrobenzo[b][1,4]dioxin-5-yl)-5-ethoxy-2,8-dimethyl-1,4-dihydro-1,6-naphthyridin e-3-carboxylic acid

2-cyanoethyl 4-(8-cyano-2,3-dihydrobenzo[*b*][1,4]dioxin-5-yl)-5-ethoxy-2,8-dimethyl-1,4-dihydro-1,6-naphthyridine-3-c arboxylate (15 mg, 0.033 mmol), ethylene glycol dimethyl ether (0.45 mL) and an aqueous solution (0.15 mL) of sodium hydroxide (3 mg, 0.066 mmol) were added into an 8 mL single-mouth bottle at room temperature to react at room temperature for 1 hour, added with a hydrochloric acid solution (1.0 mol/L) under ice bath to quench and adjust a pH value to be 5, and then extracted with ethyl acetate (5 mL × 3 times). Organic phases were combined, washed with saturated saline (5 mL × 3 times), dried with anhydrous sodium sulfate, sucked and filtered, and then concentrated under reduced pressure to obtain a pale yellow solid (10 mg, crude product), which was directly used in the reaction of the next step without further purification.
LCMS (ESI, m/z): 408.1 [M+H]⁺

### Step 8) 4-(8-cyano-2,3-dihydrobenzo[b][1,4]dioxin-5-yl)-5-ethoxy-2,8-dimethyl-1,4-dihydro-1,6-naphthyridin e-3-formamide

4-(8-cyano-2,3-dihydrobenzo[*b*][1,4]dioxin-5-yl)-5-ethoxy-2,8-dimethyl-1,4-dihydro-1,6-naphthyridin e-3-carboxylic acid (10 mg, 0.025 mmol), *N*,*N*-diisopropylethylamine (6 mg, 0.049 mmol), *N*,*N*-dimethylformamide (0.3 mL) and 2-(7-azobenzotriazole)-*N,N,N',N'*-tetramethylurea hexafluorophosphate (14 mg, 0.038 mmol) were added into an 8 mL bottle at room temperature to react at room temperature for a half hour. The reaction solution was added with an aqueous solution (25%) (0.1 mL) of ammonia at room temperature to react at room temperature for 1 hour. The reaction solution was purified by preparative high-performance liquid chromatography under the following purification conditions: chromatographic column: YMC-Actus Triart C18; mobile phase A: water (containing 0.1% formic acid) and mobile phase B: acetonitrile; flow rate: 50 mL/min; gradient: increase of acetonitrile from 25% to 30% within 10 minutes; and detection wavelength: 254/220 nm. The product was collected, and freeze-dried under reduced pressure to obtain a white solid (1.1 mg, yield 11.0%).
MS (ESI) M/Z: 407.30 [M+H]⁺

¹H NMR (300 MHz, DMSO-*d*₆) δ 7.69 (s, 1H), 7.57 *(d, J=* 8.1 Hz, 1H), 7.12 (d, *J =* 8.1 Hz, 1H), 6.76 - 6.62 (m, 3H), 5.34 (s, 1H), 4.44 - 4.25 (m, 4H), 4.06 *(d, J=* 7.0 Hz, 2H), 2.18 (s, 3H), 2.12 (s, 3H), 1.11 (t, *J= 7.0* Hz, 3H).

### Preparation of chiral compounds:

### Examples 32&33

### (S)-4-(5-cyanored-8-yl)-5-ethoxy-2,8-dimethyl-1,4-dihydro-1,6-naphthyridine-3-formamide 32

### (R)-4-(5-cyanored-8-yl)-5-ethoxy-2,8-dimethyl-1,4-dihydro-1,6-naphthyridine-3-formamide 33

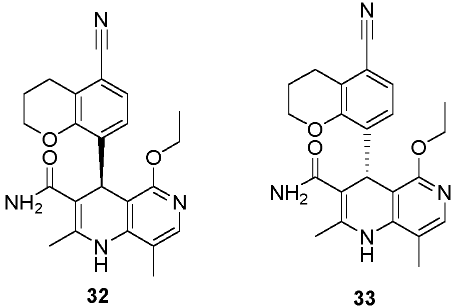

4-(5-cyanored-8-yl)-5-ethoxy-2,8-dimethyl-1,4-dihydro-1,6-naphthyridine-3-formamide (56 mg, 0.138 mmol) (compound **6)** was subjected to chiral resolution under resolution conditions: (chiral column: CHIRALPAK IG, 2*25 cm, 5 µm; mobile phase A: n-hexane (10 mmol/L methanol solution of ammonia), and mobile phase B: ethanol; flow rate: 20 mL/min; gradient: 40% B for 15 minutes; wavelength: 208/258 nm; peak appearance time of **33:** 8.80 minutes; peak appearance time of **32:** 12.0 minutes; sample solution: ethanol; injection volume: 0.8 mL; and number of syringes: 5) to obtain two enantiomer monomers, comprising a monomer **33:** white solid (20.3 mg, yield 36.2%) and a monomer **32:** white solid (16.9 mg, yield 30.1%).
**32** LCMS (ESI, m/z): 405.25 [M+H]⁺
**32** ¹H NMR (400 MHz, DMSO-*d*6) δ 7.70 (s, 1H), 7.56 (s, 1H), 7.18 (d, *J =* 8.0 Hz, 1H), 6.94 (d, *J =* 8.0 Hz, 1H), 6.68 (s, 2H), 5.32 (s, 1H), 4.28 - 4.20 (m, 2H), 4.08 - 4.00 (m, 2H), 2.89 (d, *J= 7.0* Hz, 2H), 2.12 (s, 3H), 2.07 (s, 3H), 2.03 - 1.94 (m, 2H), 1.10 *(t, J=* 7.0 Hz, 3H).
**33** LCMS (ESI, m/z): 405.25 [M+H]⁺
**33** ¹H NMR (400 MHz, DMSO-*d*6) δ 7.70 (s, 1H), 7.56 (s, 1H), 7.18 (d, *J =* 8.0 Hz, 1H), 6.94 (d, *J*= 8.0 Hz, 1H), 6.68 (s, 2H), 5.32 (s, 1H), 4.28 - 4.20 (m, 2H), 4.08 - 4.00 (m, 2H), 2.89 (d, *J= 7.0* Hz, 2H), 2.12 (s, 3H), 2.07 (s, 3H), 2.03 - 1.94 (m, 2H), 1.10 *(t, J=* 7.0 Hz, 3H).

### Examples 36&37

### (S)-4-(4-cyano-2,3-dihydrobenzofuran-7-yl)-5-ethoxy-2,8-dimethyl-1,4-dihydro-1,6-naphthyridin e-3-formamide 36

### (R)-4-(4-cyano-2,3-dihydrobenzofuran-7-yl)-5-ethoxy-2,8-dimethyl-1,4-dihydro-1,6-naphthyridin e-3-formamide 37

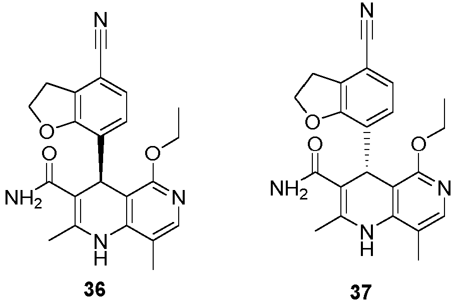

4-(4-cyano-2,3-dihydrobenzofuran-7-yl)-5-ethoxy-2,8-dimethyl-1,4-dihydro-1,6-naphthyridine-3-form amide (40 mg, 0.102 mmol) (compound **11)** was subjected to chiral resolution under resolution conditions: (chiral column: CHIRAL ART Cellulose-SB, 2*25 cm, 5 µm; mobile phase A: n-hexane (10 mmol/L methanol solution of ammonia), and mobile phase B: ethanol; flow rate: 20 mL/min; gradient: 20% B to 20% B for 15 minutes; wavelength: 218/270 nm; peak appearance time of **36:** 9.47 minutes; peak appearance time of **37:** 11.2 minutes; sample solution: ethanol; injection volume: 0.5 mL; and number of syringes: 9) to obtain two enantiomer monomers, comprising a monomer **37:** white solid (7.5 mg, yield 18.8%) and a monomer **36:** white solid (9.3 mg, yield 23.3%).
**36** LCMS (ESI, m/z): 391.10 [M+H]⁺
**36** ¹H NMR (400 MHz, DMSO-d6) δ 7.70 (s, 1H), 7.55 (s, 1H), 7.11 (d, *J =* 8.0 Hz, 1H), 6.98 (d, *J* = 8.0 Hz, 1H), 6.71 (s, 2H), 5.18 (s, 1H), 4.63 *(t, J=* 8.8 Hz, 2H), 4.04 (q, *J* = 6.8 Hz, 2H), 3.40 *(t, J=* 8.8 Hz, 2H), 2.11 (s, 3H), 2.07 (s, 3H), 1.10 *(t, J=* 6.8 Hz, 3H).
**37** LCMS (ESI, m/z): 391.10 [M+H]⁺
**37** ¹H NMR (400 MHz, DMSO-d6) δ 7.70 (s, 1H), 7.55 (s, 1H), 7.11 (d, *J =* 8.0 Hz, 1H), 6.98 (d, *J* = 8.0 Hz, 1H), 6.71 (s, 2H), 5.18 (s, 1H), 4.63 *(t, J=* 8.8 Hz, 2H), 4.04 (q, *J =* 6.8 Hz, 2H), 3.40 *(t, J=* 8.8 Hz, 2H), 2.11 (s, 3H), 2.07 (s, 3H), 1.10 *(t, J=* 6.8 Hz, 3H).

### Examples 38&39

### (S)-6-(4-cyano-2-methoxyphenyl)-5-ethoxy-8-methyl-6,9-dihydrothieno[3,2-h][1,6]naphthyridine -7-formamide 38

### (R)-6-(4-cyano-2-methoxyphenyl)-5-ethoxy-8-methyl-6,9-dihydrothieno[3,2-h] [1,6]naphthyridine -7-formamide 39

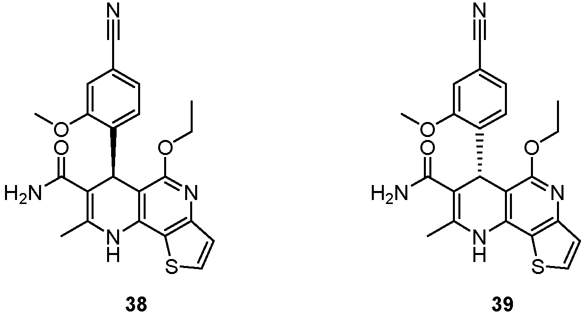

6-(4-cyano-2-methoxyphenyl)-5-ethoxy-8-methyl-6,9-dihydrothieno[3,2-*h*][1,6]naphthyridine-7-form amide (60 mg, 0.148 mmol) was subjected to chiral resolution under resolution conditions: (chiral column: CHIRALPAK IG, 2*25 cm, 5 µm; mobile phase A: n-hexane (10 mmol/L methanol solution of ammonia), and mobile phase B: ethanol; flow rate: 20 mL/min; gradient: 10% B for 34.5 minutes; wavelength: 210/220 nm; peak appearance time of **39:** 20.68 minutes; peak appearance time of **38:** 28.59 minutes; sample solution: methanol: dichloromethane = 1: 1; injection volume: 0.35 mL; and number of syringes: 12) to obtain two enantiomer monomers, comprising a monomer 0A: white solid (13.8 mg, yield 33.6%) and a monomer 0B: white solid (14.0 mg, white solid, yield 34.1%).
**38** MS (ESI) M/Z: 421.00 [M+H]⁺
**38** ¹H NMR (400 MHz, DMSO-d6) δ 8.83 (s, 1H), 7.88 (d, *J =* 5.6 Hz, 1H), 7.37 (d, *J =* 1.6 Hz, 1H), 7.29 - 7.22 (m, 2H), 7.18 (d, *J =* 7.6 Hz, 1H), 6.88 - 6.73 (m, 2H), 5.48 (s, 1H), 4.18 - 4.04 (m, 2H), 3.82 (s, 3H), 2.19 (s, 3H), 1.10 (t, J = 7.2 Hz, 3H).
**39** MS (ESI) M/Z: 421.05 [M+H]⁺
**39** ¹H NMR (400 MHz, DMSO-*d*6) δ 8.83 (s, 1H), 7.88 (d, *J =* 5.6 Hz, 1H), 7.37 (d, *J =* 1.6 Hz, 1H), 7.29 - 7.22 (m, 2H), 7.18 (d, *J =* 7.6 Hz, 1H), 6.88 - 6.73 (m, 2H), 5.48 (s, 1H), 4.18 - 4.04 (m, 2H), 3.82 (s, 3H), 2.19 (s, 3H), 1.10 (t, J = 7.2 Hz, 3H).

### Examples 40&41

### (R)-4-(8-cyano-2,3-dihydrobenzo[b][1,4]dioxin-5-yl)-5-ethoxy-2,8-dimethyl-1,4-dihydro-1,6-naph thyridine-3-formamide 41

### (S)-4-(8-cyano-2,3-dihydrobenzo[b][1,4]dioxin-5-yl)-5-ethoxy-2,8-dimethyl-1,4-dihydro-1,6-naph thyridine-3-formamide 40

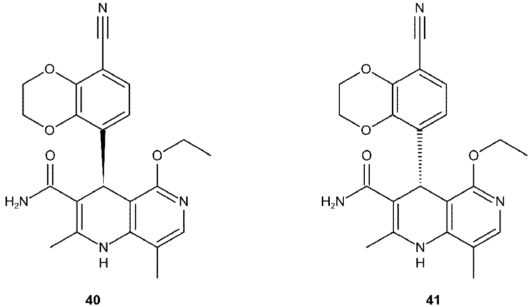

4-(8-cyano-2,3-dihydrobenzo[*b*][1,4]dioxin-5-yl)-5-ethoxy-2,8-dimethyl-1,4-dihydro-1,6-naphthyridin e-3-formamide (32.0 mg, 0.079 mmol) (compound **25)** was subjected to chiral resolution under the following resolution conditions: (chiral column: CHIRALPAK IG, 2*25 cm, 5 µm; mobile phase A: n-hexane (10 mmol/L methanol solution of ammonia), and mobile phase B: ethanol; flow rate: 20 mL/min; gradient: 15% B to 15% B for 20 minutes; wavelength: 218/260 nm; peak appearance time of **41:** 10.22 minutes; peak appearance time of **40:** 16.14 minutes; sample solution: ethanol: dichloromethane = 1: 1; injection volume: 0.5 mL; and number of syringes: 3) to obtain enantiomer monomers, comprising a monomer **41** (10.8 mg, white solid, yield 33.7%) and a monomer **40** (9.8 mg, white solid, yield 30.6%).
**40:** MS (ESI) M/Z: 407.30 [M+H]⁺

¹H NMR (300 MHz, DMSO-*d*₆) δ 7.69 (s, 1H), 7.57 *(d, J=* 8.1 Hz, 1H), 7.12 (d, *J =* 8.1 Hz, 1H), 6.76 - 6.62 (m, 3H), 5.34 (s, 1H), 4.44 - 4.25 (m, 4H), 4.06 *(d, J=* 7.0 Hz, 2H), 2.18 (s, 3H), 2.12 (s, 3H), 1.11 (t, *J= 7.0* Hz, 3H).
**41:** MS (ESI) M/Z: 407.30 [M+H]⁺

¹H NMR (300 MHz, DMSO-*d*₆) δ 7.69 (s, 1H), 7.57 *(d, J=* 8.1 Hz, 1H), 7.12 (d, *J =* 8.1 Hz, 1H), 6.76 - 6.62 (m, 3H), 5.34 (s, 1H), 4.44 - 4.25 (m, 4H), 4.06 *(d, J=* 7.0 Hz, 2H), 2.18 (s, 3H), 2.12 (s, 3H), 1.11 (t, *J= 7.0* Hz, 3H).

### Examples 46&47

### (S)-4-(4-cyano-2,3-dihydrobenzofuran-7-yl)-5-(difluoromethoxy)-2,8-dimethyl-1,4-dihydro-1,6-n aphthyridine-3-formamide 46

### (R)-4-(4-cyano-2,3-dihydrobenzofuran-7-yl)-5-(difluoromethoxy)-2,8-dimethyl-1,4-dihydro-1,6-n aphthyridine-3-formamide 47

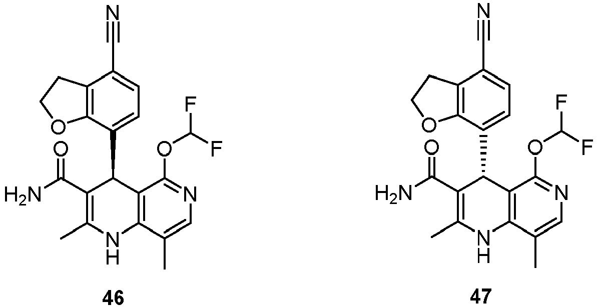

4-(4-cyano-2,3-dihydrobenzofuran-7-yl)-5-(difluoromethoxy)-2,8-dimethyl-1,4-dihydro-1,6-naphthyri dine-3-formamide (32.0 mg, 0.057 mmol) was subjected to chiral resolution under the following resolution conditions: (chiral column: CHIRAL ART Cellulose-SZ, 3*25 cm, 5 µm; mobile phase A: n-hexane (2 mmol methanol solution of ammonia), and mobile phase B: ethanol; flow rate: 40 mL/min; gradient: 20% B to 20% B for 23 minutes; wavelength: 207/236 nm; peak appearance time of **47:** 11.6 minutes; peak appearance time of **46:** 20.3 minutes; sample solution: methanol: dichloromethane = 1: 1; injection volume: 0.2 mL; and number of syringes: 3) to obtain two enantiomer monomers, comprising a monomer 0A (8.50 mg, white solid, yield 26.6%) and a monomer 0B (10.5 mg, white solid, yield 32.8%).
**46:** MS (ESI) M/Z: 413.05 [M+H]⁺

**46** ¹H NMR (300 MHz, CDCl₃) δ 7.68 (s, 1H), 7.05 - 6.99 (m, 2H), 5.87 (s, 1H), 5.23 (s, 1H), 4.75 (t, *J =* 8.7 Hz, 2H), 3.43 (t, *J =* 8.7 Hz, 2H), 2.51 (s, 3H), 2.19 (s, 3H).

19F NMR (282 MHz, CDCl₃) δ -87.2, -90.2.
**47:** MS (ESI) M/Z: 413.05 [M+H]⁺

**47** ¹H NMR (300 MHz, CDCl₃) δ 7.68 (s, 1H), 7.05 - 6.99 (m, 2H), 5.87 (s, 1H), 5.23 (s, 1H), 4.75 (t, *J =* 8.7 Hz, 2H), 3.43 (t, *J =* 8.7 Hz, 2H), 2.51 (s, 3H), 2.19 (s, 3H).

19F NMR (282 MHz, CDCl₃) δ -87.2, -90.2.

### Examples 48&49

### (S)4-(4-cyano-2,3-dihydrobenzofuran-7-yl)-5-cyclopropoxy-2,8-dimethyl-1,4-dihydro-1,6-naphth yridine-3-formamide 48

### (R)4-(4-cyano-2,3-dihydrobenzofuran-7-yl)-5-cyclopropoxy-2,8-dimethyl-1,4-dihydro-1,6-naphth yridine-3-formamide 49

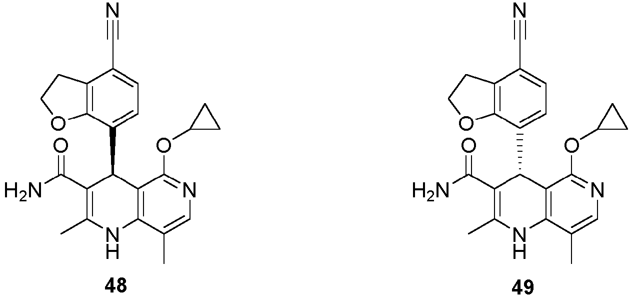

4-(4-cyano-2,3-dihydrobenzofuran-7-yl)-5-cyclopropoxy-2,8-dimethyl-1,4-dihydro-1,6-naphthyridine-3-formamide (20.2 mg, 0.050 mmol) was subjected to chiral resolution under the following resolution conditions: (chiral column: CHIRAL ART Cellulose-SZ, 3*25 cm, 5 µm; mobile phase A: n-hexane (10 mmol methanol solution of ammonia), and mobile phase B: ethanol; flow rate: 20 mL/min; gradient: 30% B to 30% B for 22 minutes; wavelength: 200/220 nm; peak appearance time of **49:** 13.4 minutes; peak appearance time of **48:** 19.8 minutes; sample solution: methanol: dichloromethane = 1: 1; injection volume: 2 mL; and number of syringes: 3) to obtain two enantiomer monomers, comprising a monomer **49** (6.20 mg, white solid, yield 22.2%) and a monomer **48** (5.40 mg, white solid, yield 19.3%).
**48:** LCMS (ESI) M/Z: 403.20 [M+H]⁺

**48** ¹H NMR (400 MHz, Chloroform-d) δ7.74 (s, 1H), 7.02 - 6.98 (m, 2H), 5.82 (s, 1H), 5.08 (s, 1H), 4.79 - 4.65 (m, 2H), 4.42 - 4.18 (m, 2H), 3.43 (t, *J =* 8.4 Hz, 2H), 2.47 (s, 3H), 2.16 (s, 3H), 0.74 - 0.54 (m, 3H), 0.20 - 0.15 (m, 1H).
**49:** LCMS (ESI) M/Z: 403.20 [M+H]⁺

**49** ¹H NMR (400 MHz, Chloroform-d) δ7.74 (s, 1H), 7.02 - 6.98 (m, 2H), 5.82 (s, 1H), 5.08 (s, 1H), 4.79 - 4.65 (m, 2H), 4.42 - 4.18 (m, 2H), 3.43 (t, *J =* 8.4 Hz, 2H), 2.47 (s, 3H), 2.16 (s, 3H), 0.74 - 0.54 (m, 3H), 0.20 - 0.15 (m, 1H).

### Examples 62&63

### (R)-4-(7-cyanobenzo[d][1,3]dioxol-4-yl)-5-ethoxy-2,8-dimethyl-1,4-dihydro-1,6-naphthyridine-3-formamide 63

### (S)-4-(7-cyanobcnzo[d][1,3]dioxol-4-yl)-5-cthoxy-2,8-dimcthyl-1,4-dihydro-1,6-naphthyridinc-3-f ormamide 62

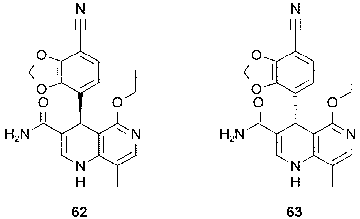

4-(7-cyanobenzo[d][1,3]dioxol-4-yl)-5-ethoxy-2,8-dimethyl-1,4-dihydro-1,6-naphthyridine-3-formami de (20 mg, 0.148 mmol) (compound 14) was subjected to chiral resolution under the following resolution conditions: (chiral column: CHIRAL ART Amylose-C NEO, 2*25 cm, 5 µm; mobile phase A: n-hexane (10 mmol methanol solution of ammonia), and mobile phase B: ethanol; flow rate: 20 mL/min; gradient: 10% B to 10% B for 28 minutes; wavelength: 214/254 nm; peak appearance time of **63:** 10.7 minutes; peak appearance time of **62:** 15.6 minutes; sample solution: hexane: ethanol = 85: 15; injection volume: 0.4 mL; and number of syringes: 2) to obtain two enantiomer monomers, comprising a monomer **63** (5.7 mg, white solid, yield 28.5%) and a monomer **62** (3.7 mg, white solid, yield 18.5%).
**62:** LCMS (ESI) M/Z: 393.1 [M+H]⁺

**62** ¹H NMR (400 MHz, Chloroform-d) δ δ 7.70 (s, 1H), 6.91 (d, *J* = 8.4 Hz, 1H), 6.77 (d, *J =* 8.4 Hz, 1H), 6.14 (s, 2H), 5.78 (s, 1H), 5.47 (s, 2H), 5.14 (s, 1H), 4.20 (q, *J* = 7.0 Hz, 2H), 2.46 (s, 3H), 2.15 (s, 3H), 1.26 (t, *J =* 7.0 Hz, 3H).
**63:** LCMS (ESI) M/Z: 393.1 [M+H]⁺

**63** ¹H NMR (400 MHz, Chloroform-d) δ δ 7.70 (s, 1H), 6.91 (d, *J =* 8.4 Hz, 1H), 6.77 (d, *J =* 8.4 Hz, 1H), 6.14 (s, 2H), 5.78 (s, 1H), 5.47 (s, 2H), 5.14 (s, 1H), 4.20 (q, *J* = 7.0 Hz, 2H), 2.46 (s, 3H), 2.15 (s, 3H), 1.26 (t, *J =* 7.0 Hz, 3H).

Other racemate embodiments in the present application were all resolved by similar means.

### Single crystal X-ray diffraction analysis

### Single crystal X-ray diffraction analysis of compound 36

About 5 mg of compound 36 was dissolved in 0.6 mL of acetone at room temperature, and then gradually added with 1.4 mL of water. The filtrate was filtered into a clean bottle, covered with a pinhole film, and slowly evaporated at room temperature. A bulk single crystal was formed after 1 day, and a single crystal obtained was used for single crystal X-ray diffraction, as shown in FIG. 9.

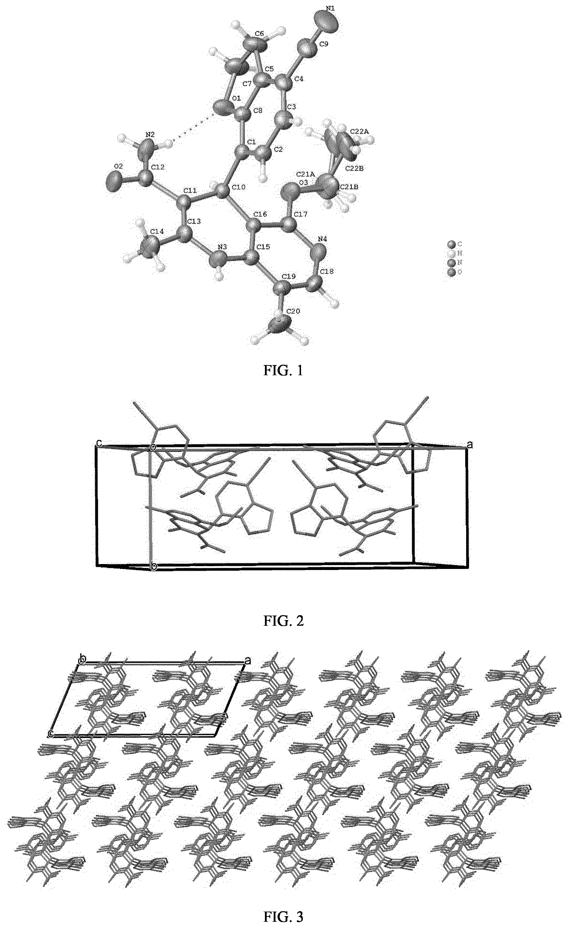

The **36** was crystallized in a monoclinic system in a C2 space group with a chemical formula of C₂₂H₂₂N₄O₃. One **36** molecule existed in each asymmetric unit, and a unit cell contained four asymmetric units. As shown in FIG. 1, chiral carbon of C10 was determined as an "S" configuration. A position of terminal ethyl was disordered.

A refined single crystal structure was shown in FIG. 2 and FIG. 3 (for clarity, a main conformation and hydrogen atoms were omitted). Crystal structure data were summarized in Table 2, and details of atomic coordinates, anisotropic displacement parameters, bond length and angle, hydrogen bond, torsion angle, and atomic occupancy were as shown in Tables 3 to 10. An XRPD pattern calculated according to the single crystal structure (above) was consistent with an experimental pattern (below) (FIG. 4).

**Table 2 Crystal data and structure refinement diagram of compound 36**

| | |
|---|---|
| Empirical formula | C₂₂H₂₂N₄O₃ |
| Formula weight | 390.43 |
| Temperature/K | 180.00(10) |
| Crystal system | Monoclinic |
| Space group | C2 |
| *a*/Å | 23.1853(2) |
| *b*/Å | 8.74723(7) |
| *c*/Å | 10.94032(10) |
| *α*/*°* | 90 |
| *β*/° | 112.7428(8) |
| *γ*/*°* | 90 |
| Volume/Å³ | 2046.27(3) |
| *Z* | 4 |
| *ρ*_{calc}g/cm³ | 1.267 |
| *µ*/mm⁻¹ | 0.703 |
| *F(000)* | 824.0 |
| Crystal size/mm³ | 0.18 × 0.11 × 0.09 |
| Radiation | Cu Kα (*λ* = 1.54184 Å) |
| 2*θ* range for data collection/° | 8.27 to 146.586 |
| Index ranges | -28 ≤*h* ≤28, -10 ≤ *k* ≤ 10,-13 ≤ *l* ≤13 |
| Reflections collected | 18390 |
| Independent reflections | 3849 [*R*ᵢₙₜ = 0.0492, *R*_{sigma} = 0.0286] |
| Data/restraints/parameters | 3849/8/283 |
| Goodness-of-fit on *F*² | 1.038 |
| Final *R* indexes [*I*>=2*σ* (*I*)] | *R*₁ = 0.0338, *wR*₂ = 0.0918 |
| Final *R* indexes [all data] | *R*₁ = 0.0343, *wR*₂ = 0.0926 |
| Largest diff. peak/hole / e Å⁻³ | 0.18/-0.14 |
| Flack parameter | 0.20(8) |

**Table 3 Fractional atomic coordinates (×10⁴) and equivalent isotropic displacement parameters (Å2×10³) of compound 36**

| Atom | *x* | *y* | *z* | U(eq) |
|---|---|---|---|---|
| O1 | 5598.3(7) | 7323.4(16) | 2415.6(16) | 40.3(4) |
| O2 | 6551.3(8) | 8603(2) | -853.4(16) | 52.5(5) |
| O3 | 7008.4(7) | 6525(2) | 5083.5(15) | 45.4(4) |
| N1 | 4786.8(13) | 1056(3) | 1548(3) | 69.4(7) |
| N2 | 6078.2(10) | 8909(3) | 550.6(19) | 47.1(5) |
| N3 | 8006.3(8) | 6131(2) | 2080.7(17) | 39.1(4) |
| N4 | 8025.3(8) | 5611(2) | 5878.4(16) | 38.5(4) |
| C1 | 6408.3(8) | 5702(2) | 2201.2(17) | 27.8(4) |
| C2 | 6546.6(9) | 4203(2) | 1959.5(19) | 32.9(4) |
| C3 | 6127.8(10) | 3011(2) | 1753(2) | 38.8(5) |
| C4 | 5538.3(10) | 3304(2) | 1772(2) | 37.4(5) |
| C5 | 5379.3(9) | 4780(2) | 1978(2) | 36.3(5) |
| C6 | 4787.7(11) | 5459(3) | 2001(3) | 53.1(6) |
| C7 | 5004.1(10) | 7049(3) | 2550(3) | 52.4(6) |
| C8 | 5814.0(9) | 5932(2) | 2197.9(18) | 31.3(4) |
| C9 | 5112.1(11) | 2057(3) | 1637(2) | 48.0(5) |
| C10 | 6904.6(8) | 6949(2) | 2508.4(17) | 27.0(4) |
| C11 | 7021.4(9) | 7387(2) | 1268.1(18) | 30.2(4) |
| C12 | 6544.6(9) | 8333(2) | 244.2(19) | 35.0(4) |
| C13 | 7535.8(9) | 6892(3) | 1091.8(19) | 35.9(4) |
| C14 | 7688.9(13) | 7122(4) | -112(2) | 57.9(7) |
| C15 | 8018.1(9) | 5964(2) | 3345.7(18) | 31.1(4) |
| C16 | 7500.8(8) | 6389(2) | 3597.2(18) | 27.6(4) |
| C17 | 7533.9(9) | 6162(2) | 4892.2(19) | 32.0(4) |
| C18 | 8523.3(10) | 5251(3) | 5591(2) | 39.8(5) |
| C19 | 8556.0(9) | 5384(2) | 4372(2) | 36.4(4) |
| C20 | 9134.7(11) | 4958(4) | 4141(3) | 58.6(7) |
| C21A | 6947(9) | 6260(30) | 6319(17) | 61(2) |
| C21B | 7011(12) | 6080(40) | 6340(20) | 61(2) |
| C22A | 6454(6) | 5055(14) | 6100(12) | 97(3) |
| C22B | 6345(8) | 5840(20) | 6176(18) | 97(3) |

| | | | | |
|---|---|---|---|---|
| U(eq) is defined as one third of the trace of the orthogonalized Uᵢⱼ tensor. | | | | |

**Table 4 Anisotropic displacement parameters (Å²×10³) of compound 36**

| Atom | U₁₁ | U₂₂ | U₃₃ | U₂₃ | U₁₃ | U₁₂ |
|---|---|---|---|---|---|---|
| O1 | 31.0(7) | 36.7(8) | 56.7(9) | -5.8(6) | 20.7(7) | 2.1(6) |
| O2 | 49.3(9) | 71.8(12) | 37.1(8) | 22.4(8) | 17.4(7) | 9.5(8) |
| O3 | 39.8(8) | 71.6(11) | 30.0(7) | 5.9(7) | 19.1(6) | 7.1(7) |
| N1 | 76.5(16) | 63.5(15) | 61.8(14) | -10.5(11) | 19.7(12) | -40.2(14) |
| N2 | 55.1(12) | 49.8(11) | 38.0(9) | 14.8(9) | 19.8(9) | 21.5(9) |
| N3 | 33.5(8) | 55.9(11) | 32.4(8) | 4.6(8) | 17.7(7) | 9.8(8) |
| N4 | 40.1(9) | 46.6(10) | 26.4(8) | 4.8(7) | 10.3(7) | 0.8(8) |
| C1 | 29.4(9) | 30.5(9) | 23.7(8) | 0.7(7) | 10.4(7) | 0.1(7) |
| C2 | 33.5(9) | 33.0(10) | 32.3(9) | -2.3(7) | 12.9(8) | 1.0(8) |
| C3 | 45.5(11) | 31.0(10) | 37.9(10) | -6.0(8) | 13.9(9) | -3.8(8) |
| C4 | 41.7(11) | 36.1(10) | 31.4(9) | -3.8(8) | 10.6(8) | -13.0(9) |
| C5 | 29.5(9) | 44.1(11) | 33.2(10) | -1.9(8) | 9.7(8) | -5.4(8) |
| C6 | 32.2(11) | 60.7(15) | 68.6(16) | -7.0(12) | 22.1(11) | -7.9(10) |
| C7 | 33.8(11) | 57.3(14) | 71.2(16) | -3.2(13) | 25.8(11) | 4.4(11) |
| C8 | 30.3(9) | 31.7(9) | 31.5(9) | -1.8(8) | 11.6(7) | 0.4(8) |
| C9 | 51.1(13) | 48.7(12) | 39.1(11) | -5.8(10) | 11.7(10) | -17.4(12) |
| C10 | 28.1(8) | 28.6(8) | 24.8(8) | -0.7(7) | 10.7(7) | 0.6(7) |
| C11 | 32.3(9) | 33.3(9) | 24.0(9) | 2.4(7) | 9.8(7) | -1.3(7) |
| C12 | 37.0(10) | 35.0(9) | 30.9(9) | 5.4(8) | 10.7(8) | -2.3(8) |
| C13 | 37.3(10) | 45.6(11) | 26.6(9) | 3.8(8) | 14.3(8) | 1.1(9) |
| C14 | 56.3(14) | 88.4(19) | 39.4(12) | 18.4(13) | 30.0(11) | 17.0(15) |
| C15 | 30.0(9) | 35.9(9) | 27.1(9) | -0.4(8) | 10.7(7) | -1.7(8) |
| C16 | 28.2(8) | 29.0(8) | 25.0(8) | -0.7(6) | 9.6(7) | -2.1(7) |
| C17 | 32.6(9) | 37.0(10) | 27.2(9) | -0.7(7) | 12.4(7) | -3.2(8) |
| C18 | 32.8(10) | 48.0(12) | 30.9(9) | 6.2(8) | 3.9(8) | 1.8(9) |
| C19 | 28.6(9) | 41.4(10) | 36.3(10) | 2.3(8) | 9.4(8) | 0.4(8) |
| C20 | 32.4(11) | 90(2) | 50.4(13) | 10.8(13) | 13.0(10) | 15.5(12) |
| C21A | 54(4) | 103(5) | 35.9(13) | 15(2) | 29(2) | 14(3) |
| C21B | 54(4) | 103(5) | 35.9(13) | 15(2) | 29(2) | 14(3) |
| C22A | 113(5) | 97(7) | 105(4) | 42(7) | 69(4) | -8(6) |
| C22B | 113(5) | 97(7) | 105(4) | 42(7) | 69(4) | -8(6) |

The anisotropic displacement factor exponent takes the form: -2π²[h²a*²U₁₁+2hka*b*U₁₂+...].

**Table 5 Bond length of compound 36**

| Atom | Atom | Length/Å | | Atom | Atom | Length/Å |
|---|---|---|---|---|---|---|
| O1 | C7 | 1.461(3) | | C4 | C5 | 1.385(3) |
| O1 | C8 | 1.371(2) | | C4 | C9 | 1.440(3) |
| O2 | C12 | 1.230(3) | | C5 | C6 | 1.504(3) |
| O3 | C17 | 1.351(2) | | C5 | C8 | 1.379(3) |
| O3 | C21A | 1.432(15) | | C6 | C7 | 1.521(4) |
| O3 | C21B | 1.43(2) | | C10 | C11 | 1.531(2) |
| N1 | C9 | 1.135(3) | | C10 | C16 | 1.516(2) |
| N2 | C12 | 1.348(3) | | C11 | C12 | 1.485(3) |
| N3 | C13 | 1.376(3) | | C11 | C13 | 1.351(3) |
| N3 | C15 | 1.381(2) | | C13 | C14 | 1.504(3) |
| N4 | C17 | 1.321(3) | | C15 | C16 | 1.381(3) |
| N4 | C18 | 1.346(3) | | C15 | C19 | 1.411(3) |
| C1 | C2 | 1.399(3) | | C16 | C17 | 1.403(2) |
| C1 | C8 | 1.391(2) | | C18 | C19 | 1.370(3) |
| C1 | C10 | 1.526(3) | | C19 | C20 | 1.505(3) |
| C2 | C3 | 1.383(3) | | C21A | C22A | 1.503(18) |
| C3 | C4 | 1.399(3) | | C21B | C22B | 1.50(2) |

**Table 6 Bond angle of compound 36**

| Atom | Atom | Atom | Angle/° | | Atom | Atom | Atom | Angle/° |
|---|---|---|---|---|---|---|---|---|
| C8 | O1 | C7 | 107.03(16) | | C12 | C11 | C10 | 118.21(16) |
| C17 | O3 | C21A | 122.2(8) | | C13 | C11 | C10 | 120.99(16) |
| C17 | O3 | C21B | 114.7(12) | | C13 | C11 | C12 | 120.78(17) |
| C13 | N3 | C15 | 122.79(16) | | O2 | C12 | N2 | 119.03(19) |
| C17 | N4 | C18 | 116.20(17) | | O2 | C12 | C11 | 123.9(2) |
| C2 | C1 | C10 | 120.22(16) | | N2 | C12 | C11 | 117.11(17) |
| C8 | C1 | C2 | 115.76(17) | | N3 | C13 | C14 | 111.69(18) |
| C8 | C1 | C10 | 123.96(17) | | C11 | C13 | N3 | 121.22(16) |
| C3 | C2 | C1 | 122.67(18) | | C11 | C13 | C14 | 127.05(19) |
| C2 | C3 | C4 | 119.21(19) | | N3 | C15 | C19 | 120.27(17) |
| C3 | C4 | C9 | 119.7(2) | | C16 | C15 | N3 | 119.28(17) |
| C5 | C4 | C3 | 119.74(18) | | C16 | C15 | C19 | 120.45(17) |
| C5 | C4 | C9 | 120.5(2) | | C15 | C16 | C10 | 121.92(16) |
| C4 | C5 | C6 | 132.24(19) | | C15 | C16 | C17 | 116.79(17) |
| C8 | C5 | C4 | 119.16(18) | | C17 | C16 | C10 | 121.10(16) |
| C8 | C5 | C6 | 108.59(19) | | O3 | C17 | C16 | 115.01(17) |
| C5 | C6 | C7 | 101.74(17) | | N4 | C17 | O3 | 120.25(17) |
| O1 | C7 | C6 | 106.91(19) | | N4 | C17 | C16 | 124.72(18) |
| O1 | C8 | C1 | 123.76(17) | | N4 | C18 | C19 | 125.65(19) |
| O1 | C8 | C5 | 112.81(17) | | C15 | C19 | C20 | 121.79(19) |
| C5 | C8 | C1 | 123.43(18) | | C18 | C19 | C15 | 116.15(18) |
| N1 | C9 | C4 | 178.5(3) | | C18 | C19 | C20 | 122.05(19) |
| C1 | C10 | C11 | 111.11(14) | | O3 | C21A | C22A | 108.1(13) |
| C16 | C10 | C1 | 108.82(15) | | O3 | C21B | C22B | 107.4(17) |
| C16 | C10 | C11 | 110.94(15) | | | | | |

**Table 7 Hydrogen bond of compound 36**

| D | H | A | d(D-H)/Å | d(H-A)/Å | d(D-A)/Å | D-H-A/° |
|---|---|---|---|---|---|---|
| N2 | H2A | O1 | 0.87(2) | 2.20(2) | 3.013(3) | 154(3) |
| N2 | H2B | N1¹ | 0.89(2) | 2.16(2) | 3.043(3) | 177(3) |
| N3 | H3 | O2² | 0.86(2) | 2.27(3) | 2.968(3) | 138(2) |

| | | | | | | |
|---|---|---|---|---|---|---|
| ¹1*-x, 1+y, -z;* ²3/2-*x, -1*/*2+y, -z* | | | | | | |

**Table 8 Torsion angle of compound 36**

| A | B | c | D | Angle/° | | A | B | c | D | Angle/° |
|---|---|---|---|---|---|---|---|---|---|---|
| N3 | C15 | C16 | C10 | 3.5(3) | | C10 | C1 | C8 | O1 | 2.9(3) |
| N3 | C15 | C16 | C17 | 178.62(17) | | C10 | C1 | C8 | C5 | -176.97(17) |
| N3 | C15 | C19 | C18 | -179.62(19) | | C10 | C11 | C12 | O2 | -171.2(2) |
| N3 | C15 | C19 | C20 | 1.2(3) | | C10 | C11 | C12 | N2 | 8.3(3) |
| N4 | C18 | C19 | C15 | 0.6(3) | | C10 | C11 | C13 | N3 | -7.6(3) |
| N4 | C18 | C19 | C20 | 179.7(2) | | C10 | C11 | C13 | C14 | 174.9(2) |
| C1 | C2 | C3 | C4 | 0.6(3) | | C10 | C16 | C17 | O3 | -1.9(3) |

| A | B | c | D | Angle/° | | A | B | C | D | Angle/° |
|---|---|---|---|---|---|---|---|---|---|---|
| C1 | C10 | C11 | C12 | 74.8(2) | | C10 | C16 | C17 | N4 | 176.72(18) |
| C1 | C10 | C11 | C13 | -103.5(2) | | C11 | C10 | C16 | C15 | -15.7(2) |
| C1 | C10 | C16 | C15 | 106.82(19) | | C11 | C10 | C16 | C17 | 169.41(17) |
| C1 | C10 | C16 | C17 | -68.1(2) | | C12 | C11 | C13 | N3 | 174.2(2) |
| C2 | C1 | C8 | O1 | -179.97(18) | | C12 | C11 | C13 | C14 | -3.3(4) |
| C2 | C1 | C8 | C5 | 0.2(3) | | C13 | N3 | C15 | C16 | 9.1(3) |
| C2 | C1 | C10 | C11 | 72.2(2) | | C13 | N3 | C15 | C19 | -170.2(2) |
| C2 | C1 | C10 | C16 | -50.2(2) | | C13 | C11 | C12 | O2 | 7.1(3) |
| C2 | C3 | C4 | C5 | 0.9(3) | | C13 | C11 | C12 | N2 | -173.5(2) |
| C2 | C3 | C4 | C9 | -176.32(19) | | C15 | N3 | C13 | C11 | -7.0(3) |
| C3 | C4 | C5 | C6 | 177.4(2) | | C15 | N3 | C13 | C14 | 170.9(2) |
| C3 | C4 | C5 | C8 | -1.8(3) | | C15 | C16 | C17 | O3 | -177.07(17) |
| C4 | C5 | C6 | C7 | 169.2(2) | | C15 | C16 | C17 | N4 | 1.6(3) |
| C4 | C5 | C8 | O1 | -178.57(18) | | C16 | C10 | C11 | C12 | -164.03(16) |
| C4 | C5 | C8 | C1 | 1.3(3) | | C16 | C10 | C11 | C13 | 17.7(3) |
| C5 | C6 | C7 | O1 | 16.5(3) | | C16 | C15 | C19 | C18 | 1.1(3) |
| C6 | C5 | C8 | O1 | 2.0(2) | | C16 | C15 | C19 | C20 | -178.0(2) |
| C6 | C5 | C8 | C1 | -178.09(19) | | C17 | O3 | C21A | C22A | -113.5(12) |
| C7 | O1 | C8 | C1 | -170.83(19) | | C17 | O3 | C21B | C22B | -152.9(16) |
| C7 | O1 | C8 | C5 | 9.0(2) | | C17 | N4 | C18 | C19 | -1.2(3) |
| C8 | O1 | C7 | C6 | -16.2(3) | | C18 | N4 | C17 | O3 | 178.60(19) |
| C8 | C1 | C2 | C3 | -1.1(3) | | C18 | N4 | C17 | C16 | 0.0(3) |
| C8 | C1 | C10 | C11 | -110.78(18) | | C19 | C15 | C16 | C10 | -177.20(18) |
| C8 | C1 | C10 | C16 | 126.79(18) | | C19 | C15 | C16 | C17 | -2.1(3) |
| C8 | C5 | C6 | C7 | -11.5(3) | | C21A | O3 | C17 | N4 | -3.4(11) |
| C9 | C4 | C5 | C6 | -5.4(4) | | C21A | O3 | C17 | C16 | 175.3(11) |
| C9 | C4 | C5 | C8 | 175.38(18) | | C21B | O3 | C17 | N4 | -7.5(15) |
| C10 | C1 | C2 | C3 | 176.12(17) | | C21B | O3 | C17 | C16 | 171.2(15) |

**Table 9 Hydrogen atomic coordinates (Å×10⁴) and isotropic displacement parameters (Å2×10³) of compound 36**

| Atom | *x* | *y* | *z* | U(eq) |
|---|---|---|---|---|
| H2A | 6018(14) | 8660(40) | 1270(30) | 57 |
| H2B | 5828(12) | 9560(30) | -40(30) | 57 |
| H3 | 8301(11) | 5720(30) | 1900(30) | 47 |
| H2 | 6944 | 3996 | 1936 | 39 |
| H3A | 6239 | 2005 | 1600 | 47 |
| H6A | 4634 | 4871 | 2588 | 64 |
| H6B | 4455 | 5507 | 1101 | 64 |
| H7A | 4691 | 7824 | 2046 | 63 |
| H7B | 5060 | 7105 | 3493 | 63 |
| H10 | 6754 | 7874 | 2834 | 32 |
| H14A | 8055 | 6506 | -27 | 87 |
| H14B | 7779 | 8205 | -187 | 87 |
| H14C | 7332 | 6807 | -905 | 87 |
| H18 | 8883 | 4871 | 6294 | 48 |
| H20A | 9029 | 4184 | 3441 | 88 |
| H20B | 9449 | 4545 | 4961 | 88 |
| H20C | 9303 | 5868 | 3872 | 88 |
| H21A | 6825 | 7214 | 6639 | 73 |
| H21B | 7351 | 5907 | 6993 | 73 |
| H21C | 7206 | 6894 | 7009 | 73 |
| H21D | 7254 | 5129 | 6652 | 73 |
| H22A | 6060 | 5401 | 5414 | 146 |
| H22B | 6395 | 4878 | 6928 | 146 |
| H22C | 6587 | 4103 | 5814 | 146 |
| H22D | 6090 | 6704 | 5683 | 146 |
| H22E | 6321 | 5774 | 7049 | 146 |
| H22F | 6188 | 4893 | 5684 | 146 |

**Table 10 Atomic occupancy of compound 36**

| Atom | *Occupancy* | | Atom | *Occupancy* | | Atom | *Occupancy* |
|---|---|---|---|---|---|---|---|
| C21A | 0.584(14) | | H21A | 0.584(14) | | H21B | 0.584(14) |
| C21B | 0.416(14) | | H21C | 0.416(14) | | H21D | 0.416(14) |
| C22A | 0.584(14) | | H22A | 0.584(14) | | H22B | 0.584(14) |
| H22C | 0.584(14) | | C22B | 0.416(14) | | H22D | 0.416(14) |
| H22E | 0.416(14) | | H22F | 0.416(14) | | | |

### Single crystal X-ray diffraction analysis of compound 62

About 5 mg of compound 62 was dissolved in 0.1 mL of ethanol at room temperature, and then gradually added with 0.35 mL of n-heptane. The filtered solution entered a clean sample bottle, and the filtrate was covered with a pinhole film, and slowly evaporated under environmental conditions. A bulk single crystal was formed after 1 day, and a single crystal obtained was used for single crystal X-ray diffraction, as shown in FIG. 10.

The **62** was crystallized in a monoclinic system in a C2 space group with a chemical formula of C₂₁H₂₀N₄O₄. Two **62** molecules existed in each asymmetric unit, and a unit cell contained four asymmetric units. As shown in FIG. 5, chiral carbon of C9 (C30) was determined as an "S" configuration.

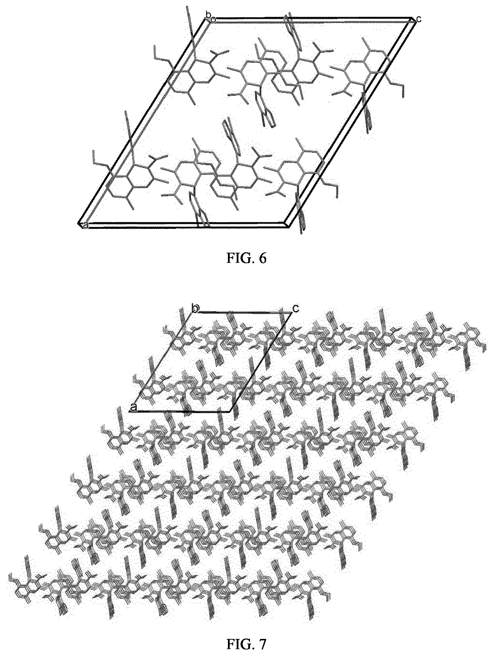

A refined single crystal structure was shown in FIG. 6 and FIG. 7 (for clarity, hydrogen atoms were omitted). Crystal structure data were summarized in Table 11, and details of atomic coordinates, anisotropic displacement parameters, bond length and angle, hydrogen bond and torsion angle were as shown in Tables 12 to 18. An XRPD pattern calculated according to the single crystal structure was consistent with an experimental pattern (FIG. 8).

**Table 11 Crystal data and structure optimization of compound 62**

| | |
|---|---|
| Empirical formula | C₂₁H₂₀N₄O₄ |
| Formula weight | 392.41 |
| Temperature/K | 180.00(10) |
| Crystal system | Monoclinic |
| Space group | C2 |
| *a*/Å | 24.92890(19) |
| *b*/Å | 8.89441(5) |
| *c*/Å | 21.18548(18) |
| α/° | 90 |
| *β*/° | 121.6750(10) |
| *γ*/° | 90 |
| Volume/Å³ | 3997.68(6) |
| *Z* | 8 |
| *ρ*_{calc}g/cm³ | 1.304 |
| *µ*/mm⁻¹ | 0.762 |
| *F(000)* | 1648.0 |
| Crystal size/mm³ | 0.28 × 0.09 × 0.08 |
| Radiation | Cu Kα (*λ* = 1.54184 Å) |
| 2*θ* range for data collection/° | 4.902 to 146.544 |
| Index ranges | -30 ≤*h* ≤ 28, -10 ≤ *k*≤ 10, -25 *≤ l* ≤ 26 |
| Reflections collected | 42385 |
| Independent reflections | 7574 [*R*ᵢₙₜ = 0.0664, *R*_{sigma} = 0.0373] |
| Data/restraints/parameters | 7574/16/548 |
| Goodness-of-fit on *F*² | 1.027 |
| Final *R* indexes [*I*>=2*σ* (*I*)] | *R*₁ = 0.0368, *wR*₂ = 0.1020 |
| Final *R* indexes [all data] | *R*₁ = 0.0380, *wR*₂ = 0.1035 |
| Largest diff. peak/hole / e Å⁻³ | 0.21/-0.18 |
| Flack parameter | 0.11(7) |

**Table 12 Fractional atomic coordinates (×10⁴) and equivalent isotropic displacement parameters (Å2×10³) of compound 62**

| Atom | *x* | *y* | *z* | U(eq) |
|---|---|---|---|---|
| O1 | 4731.7(7) | 1967(2) | -1056.4(11) | 49.8(5) |
| O2 | 5546.5(7) | 248.6(18) | -461.8(11) | 45.8(5) |
| O3 | 6488.6(8) | -269(2) | 1908.2(9) | 39.4(4) |
| O4 | 6991.4(8) | 1373(2) | -645.4(9) | 42.3(4) |
| N1 | 4465.4(11) | 6106(3) | -1206.4(14) | 52.3(6) |
| N2 | 6459.5(10) | -1685(2) | 1012.7(11) | 34.9(4) |
| N3 | 8059.4(9) | 1900(2) | 1950.6(10) | 34.0(4) |
| N4 | 8006.2(9) | 2359(2) | -34.8(10) | 31.3(4) |
| C1 | 6392.3(9) | 2111(2) | 243.3(10) | 23.4(4) |
| C2 | 6487.4(10) | 3677(2) | 354.6(12) | 30.4(4) |
| C3 | 6006.3(11) | 4699(2) | 0.2(13) | 33.8(5) |
| C4 | 5380.9(11) | 4223(2) | -502.1(12) | 30.1(4) |
| C5 | 5286.5(10) | 2693(3) | -603.0(12) | 29.9(4) |
| C6 | 5778.8(10) | 1681(2) | -241.3(11) | 28.0(4) |
| C7 | 4889.9(11) | 401(3) | -977.3(18) | 55.4(8) |
| C8 | 4871.0(11) | 5268(3) | -896.2(13) | 36.9(5) |
| C9 | 6935.9(9) | 985(2) | 611.7(10) | 22.3(4) |
| C10 | 7094.3(9) | 557(2) | 1391.7(10) | 24.4(4) |
| C11 | 7615.2(10) | 1070(3) | 2001.9(11) | 29.5(4) |
| C12 | 6659.8(9) | -480(2) | 1468.5(11) | 26.9(4) |
| C13 | 7807.7(12) | 745(4) | 2788.0(13) | 46.2(6) |
| C14 | 8041.2(9) | 2061(2) | 1294.3(11) | 26.1(4) |
| C15 | 8565.8(10) | 2662(2) | 1296.1(12) | 30.6(4) |
| C16 | 8513.1(10) | 2767(2) | 621.5(12) | 31.1(4) |
| C17 | 7524.0(10) | 1795(2) | -13.8(11) | 26.9(4) |
| C18 | 7514.6(9) | 1597(2) | 637.9(11) | 24.0(4) |
| C19 | 9150.6(12) | 3142(4) | 2000.7(14) | 49.0(6) |
| C20 | 6876.0(15) | 1888(6) | -1348.5(14) | 70.8(11) |
| C21 | 6291(3) | 2800(8) | -1719(3) | 131(2) |
| O5 | 9452.5(8) | 5517(2) | 5710.0(10) | 49.9(5) |
| O6 | 10160.1(9) | 7480(3) | 6243.4(12) | 62.9(6) |
| O7 | 8515.9(7) | 4391(2) | 3038.4(9) | 39.5(4) |
| O8 | 8054.4(9) | 6432(2) | 5619.1(9) | 44.4(4) |
| N5 | 10201.2(15) | 11686(4) | 5993.4(17) | 76.8(9) |
| N6 | 8920.9(13) | 3793(3) | 4219.4(13) | 56.9(7) |
| N7 | 6923.4(10) | 6246(3) | 3009.9(11) | 42.3(5) |
| N8 | 7013.0(10) | 7208(3) | 4987.2(13) | 44.6(5) |
| C22 | 8590.9(10) | 7012(2) | 4705.8(11) | 28.4(4) |
| C23 | 8446.4(11) | 8482(3) | 4420.6(12) | 36.5(5) |
| C24 | 8862.0(13) | 9661(3) | 4737.7(14) | 42.5(6) |
| C25 | 9467.1(12) | 9425(3) | 5373.6(14) | 41.7(5) |
| C26 | 9613.9(11) | 7984(3) | 5640.7(14) | 40.5(5) |
| C27 | 9187.2(10) | 6821(3) | 5317.4(12) | 34.3(5) |
| C28 | 10059.4(15) | 5925(4) | 6308(2) | 75.9(11) |
| C29 | 9887.6(15) | 10666(4) | 5727.2(16) | 54.3(7) |
| C30 | 8097.7(9) | 5771(2) | 4377.1(11) | 27.4(4) |
| C31 | 7965.9(10) | 5262(2) | 3617.4(11) | 28.5(4) |
| C32 | 7404.2(10) | 5526(3) | 2986.7(12) | 35.0(5) |
| C33 | 7208.5(12) | 5050(4) | 2215.9(13) | 47.2(6) |
| C34 | 8473.2(10) | 4459(3) | 3593.4(12) | 31.3(4) |
| C35 | 6951.9(11) | 6580(3) | 3661.9(13) | 35.8(5) |
| C36 | 6422.8(11) | 7183(3) | 3644.8(15) | 44.1(6) |
| C37 | 6490.4(12) | 7458(3) | 4321.1(16) | 47.8(6) |
| C38 | 7500.3(12) | 6661(3) | 4978.1(13) | 35.4(5) |
| C39 | 7502.9(10) | 6317(2) | 4332.2(12) | 30.3(4) |
| C40 | 5820.1(13) | 7517(5) | 2925.8(18) | 68.0(10) |
| C41 | 8107.3(16) | 6838(5) | 6306.2(15) | 67.3(10) |
| C42 | 8773(2) | 7054(7) | 6873.0(18) | 88.0(13) |

| | | | | |
|---|---|---|---|---|
| U(eq) is defined as one third of the trace of the orthogonalized Uᵢⱼ tensor. | | | | |

**Table 13 Anisotropic displacement parameters (Å2×10³) of compound 62**

| Atom | U₁₁ | U₂₂ | U₃₃ | U₂₃ | U₁₃ | U₁₂ |
|---|---|---|---|---|---|---|
| O1 | 18.9(7) | 40.9(9) | 64.5(11) | -8.7(8) | 4.7(7) | 3.8(7) |
| O2 | 18.8(7) | 29.3(8) | 69.3(11) | -8.3(8) | 9.4(7) | -2.3(6) |
| O3 | 34.5(8) | 53.9(10) | 39.5(8) | -5.2(7) | 26.0(7) | -7.5(7) |
| O4 | 32.8(8) | 67.5(12) | 24.9(7) | -2.5(7) | 14.0(7) | -15.9(8) |
| N1 | 49.5(13) | 49.9(12) | 62.6(14) | 18.3(11) | 33.0(12) | 24.1(11) |
| N2 | 40.3(10) | 30.0(9) | 45.0(10) | -2.4(8) | 29.7(9) | -8.9(8) |
| N3 | 26.0(9) | 48.1(11) | 23.6(8) | -6.5(8) | 10.0(7) | -12.5(8) |
| N4 | 28.1(9) | 34.8(9) | 34.8(9) | 3.4(7) | 19.1(8) | -1.4(7) |
| C1 | 21.1(9) | 26.3(9) | 23.0(8) | -0.4(7) | 11.6(7) | -0.3(7) |
| C2 | 25.6(10) | 27.7(10) | 35.3(10) | -5.2(8) | 14.3(9) | -2.8(8) |
| C3 | 36.4(11) | 24.2(10) | 44.5(12) | -1.8(8) | 23.8(10) | -0.2(8) |
| C4 | 30.2(10) | 30.6(11) | 32.2(10) | 4.7(8) | 18.2(9) | 6.9(8) |
| C5 | 21.4(9) | 34.8(11) | 29.0(10) | -2.3(8) | 10.2(8) | 1.7(8) |
| C6 | 22.5(10) | 25.9(10) | 33.3(10) | -0.7(8) | 13.1(8) | 0.3(8) |
| C7 | 21.8(11) | 39.4(13) | 74.9(19) | -12.8(13) | 4.7(11) | 1.1(10) |
| C8 | 36.4(12) | 38.3(12) | 40.9(12) | 5.8(10) | 23.6(10) | 9.4(10) |
| C9 | 18.4(9) | 24.8(9) | 22.0(9) | -0.7(7) | 9.5(7) | -2.2(7) |
| C10 | 22.1(9) | 26.4(9) | 25.3(9) | 1.3(7) | 12.8(8) | 0.7(7) |
| C11 | 24.9(10) | 37.1(10) | 26.0(10) | 0.2(8) | 13.0(8) | -2.1(8) |
| C12 | 20.8(9) | 31.3(10) | 27.7(9) | 5.4(8) | 12.0(8) | 2.1(8) |
| C13 | 33.8(12) | 73.5(18) | 27.1(11) | 1.2(11) | 13.0(9) | -12.8(12) |
| C14 | 20.9(9) | 27.3(9) | 29.2(9) | -0.3(7) | 12.6(8) | -1.4(7) |
| C15 | 21.8(9) | 32.6(10) | 35.7(11) | 0.5(8) | 13.9(9) | -2.4(8) |
| C16 | 24.2(10) | 32.7(10) | 39.2(11) | 3.0(8) | 18.6(9) | -1.8(8) |
| C17 | 22.6(9) | 28.6(9) | 27.7(9) | 1.8(8) | 12.0(8) | -1.3(8) |
| C18 | 20.6(9) | 23.9(9) | 27.5(9) | 0.5(7) | 12.6(8) | 1.3(7) |
| C19 | 28.0(11) | 71.8(18) | 40.7(13) | -5.3(12) | 13.5(10) | -19.2(12) |
| C20 | 46.8(16) | 134(3) | 25.5(12) | 4.4(16) | 14.8(12) | -29(2) |
| C21 | 158(5) | 134(5) | 70(3) | 55(3) | 38(3) | 39(4) |
| O5 | 32.8(8) | 45.4(10) | 47.6(10) | 11.3(8) | 4.8(8) | 5.8(8) |
| O6 | 33.1(10) | 73.2(14) | 52.8(11) | 2.2(10) | 2.2(8) | -9.0(9) |
| O7 | 31.9(8) | 54.5(10) | 33.5(8) | -3.8(7) | 18.1(7) | 2.7(7) |
| O8 | 41.3(9) | 62.7(11) | 32.7(8) | -4.3(8) | 21.9(7) | 3.1(8) |
| N5 | 80(2) | 81(2) | 81.1(19) | -29.6(16) | 50.2(17) | -50.6(18) |
| N6 | 68.7(16) | 65.6(15) | 42.1(12) | 14.0(11) | 32.9(12) | 40.8(13) |
| N7 | 30.6(10) | 61.3(13) | 30.4(9) | 1.0(9) | 12.8(8) | 12.5(9) |
| N8 | 37.6(11) | 55.9(13) | 47.1(11) | -14.3(10) | 27.0(10) | -4.6(9) |
| C22 | 26.2(10) | 32.9(10) | 27.1(9) | 1.2(8) | 14.7(8) | 1.1(8) |
| C23 | 35.3(11) | 34.9(11) | 32.8(11) | 4.2(9) | 13.4(9) | -1.3(9) |
| C24 | 47.9(14) | 35.6(12) | 43.6(13) | 2.8(10) | 23.6(11) | -7.5(11) |
| C25 | 41.6(13) | 46.3(13) | 41.5(12) | -7.3(11) | 24.6(11) | -15.1(11) |
| C26 | 26.7(10) | 53.4(14) | 37.1(12) | -1.3(10) | 13.7(9) | -5.5(10) |
| C27 | 26.8(10) | 38.0(11) | 35.7(11) | 2.9(9) | 14.8(9) | 1.1(9) |
| C28 | 36.5(15) | 66(2) | 78(2) | 12.2(17) | -2.5(15) | 4.2(15) |
| C29 | 59.0(16) | 59.0(17) | 55.0(16) | -12.9(13) | 37.0(14) | -24.0(15) |
| C30 | 26.4(9) | 29.4(10) | 26.3(10) | 1.9(8) | 13.7(8) | 1.8(8) |
| C31 | 28.9(10) | 29.5(10) | 29.0(10) | 0.2(8) | 16.6(8) | 0.7(8) |
| C32 | 31.0(10) | 42.8(12) | 31.3(11) | 1.7(9) | 16.3(9) | 5.4(9) |
| C33 | 37.4(12) | 72.3(18) | 28.0(11) | 1.0(11) | 14.5(10) | 13.9(12) |
| C34 | 30.4(10) | 31.3(10) | 30.9(10) | -2.4(8) | 15.1(9) | 1.8(8) |
| C35 | 30.3(11) | 41.8(12) | 35.7(11) | -4.2(9) | 17.5(9) | 1.1(9) |
| C36 | 27.5(11) | 57.1(15) | 46.8(13) | -12.0(11) | 18.8(11) | 1.1(10) |
| C37 | 31.7(12) | 62.4(17) | 54.0(15) | -20.1(12) | 25.7(11) | -3.5(11) |
| C38 | 33.5(11) | 39.5(12) | 36.3(11) | -6.2(9) | 20.5(9) | -4.6(10) |
| C39 | 27.7(10) | 30.9(10) | 35.0(11) | -2.7(8) | 18.2(9) | -3.2(8) |
| C40 | 32.4(14) | 103(3) | 56.9(18) | -15.3(17) | 15.3(13) | 18.7(15) |
| C41 | 58.3(18) | 113(3) | 37.3(14) | -13.2(17) | 29.7(14) | 2.2(19) |
| C42 | 77(2) | 136(4) | 41.2(15) | -21(2) | 23.8(16) | -2(3) |

The anisotropic displacement factor exponent takes the form: -2π²[h²a*²U₁₁+2hka*b*U₁₂+...].

**Table 14 Bond length of compound 62**

| Atom | Atom | Length/Å | | Atom | Atom | Length/Å |
|---|---|---|---|---|---|---|
| O1 | C5 | 1.363(3) | | O5 | C27 | 1.377(3) |
| O1 | C7 | 1.434(3) | | O5 | C28 | 1.419(4) |
| O2 | C6 | 1.376(3) | | O6 | C26 | 1.364(3) |
| O2 | C7 | 1.419(3) | | O6 | C28 | 1.425(5) |
| O3 | C12 | 1.226(3) | | O7 | C34 | 1.236(3) |
| O4 | C17 | 1.352(3) | | O8 | C38 | 1.350(3) |
| O4 | C20 | 1.436(3) | | O8 | C41 | 1.437(3) |
| N1 | C8 | 1.145(3) | | N5 | C29 | 1.135(4) |
| N2 | C12 | 1.351(3) | | N6 | C34 | 1.342(3) |
| N3 | C11 | 1.382(3) | | N7 | C32 | 1.382(3) |
| N3 | C14 | 1.374(3) | | N7 | C35 | 1.378(3) |
| N4 | C16 | 1.348(3) | | N8 | C37 | 1.344(4) |
| N4 | C17 | 1.325(3) | | N8 | C38 | 1.318(3) |
| C1 | C2 | 1.412(3) | | C22 | C23 | 1.406(3) |
| C1 | C6 | 1.375(3) | | C22 | C27 | 1.377(3) |
| C1 | C9 | 1.529(3) | | C22 | C30 | 1.522(3) |
| C2 | C3 | 1.372(3) | | C23 | C24 | 1.376(3) |
| C3 | C4 | 1.414(3) | | C24 | C25 | 1.414(4) |
| C4 | C5 | 1.378(3) | | C25 | C26 | 1.371(4) |
| C4 | C8 | 1.436(3) | | C25 | C29 | 1.433(4) |
| C5 | C6 | 1.384(3) | | C26 | C27 | 1.381(3) |
| C9 | C10 | 1.532(3) | | C30 | C31 | 1.532(3) |
| C9 | C18 | 1.515(3) | | C30 | C39 | 1.515(3) |
| C10 | C11 | 1.343(3) | | C31 | C32 | 1.355(3) |
| C10 | C12 | 1.495(3) | | C31 | C34 | 1.476(3) |
| C11 | C13 | 1.499(3) | | C32 | C33 | 1.499(3) |
| C14 | C15 | 1.411(3) | | C35 | C36 | 1.407(3) |
| C14 | C18 | 1.382(3) | | C35 | C39 | 1.382(3) |
| C15 | C16 | 1.368(3) | | C36 | C37 | 1.374(4) |
| C15 | C19 | 1.500(3) | | C36 | C40 | 1.504(4) |
| C17 | C18 | 1.404(3) | | C38 | C39 | 1.405(3) |
| C20 | C21 | 1.483(7) | | C41 | C42 | 1.463(5) |

**Table 15 Bond angle of compound 62**

| Atom | Atom | Atom | Angle/° | | Atom | Atom | Atom | Angle/° |
|---|---|---|---|---|---|---|---|---|
| C5 | O1 | C7 | 105.25(17) | | C27 | O5 | C28 | 105.8(2) |
| C6 | O2 | C7 | 106.44(17) | | C26 | O6 | C28 | 105.6(2) |
| C17 | O4 | C20 | 119.73(19) | | C38 | O8 | C41 | 118.8(2) |
| C14 | N3 | C11 | 122.21(17) | | C35 | N7 | C32 | 123.1(2) |
| C17 | N4 | C16 | 116.31(18) | | C38 | N8 | C37 | 116.0(2) |
| C2 | C1 | C9 | 122.32(17) | | C23 | C22 | C30 | 121.00(18) |
| C6 | C1 | C2 | 114.98(18) | | C27 | C22 | C23 | 115.2(2) |
| C6 | C1 | C9 | 122.66(17) | | C27 | C22 | C30 | 123.7(2) |
| C3 | C2 | C1 | 122.7(2) | | C24 | C23 | C22 | 122.8(2) |
| C2 | C3 | C4 | 121.0(2) | | C23 | C24 | C25 | 120.3(2) |
| C3 | C4 | C8 | 122.2(2) | | C24 | C25 | C29 | 120.3(3) |
| C5 | C4 | C3 | 116.31(19) | | C26 | C25 | C24 | 116.9(2) |
| C5 | C4 | C8 | 121.5(2) | | C26 | C25 | C29 | 122.7(3) |
| O1 | C5 | C4 | 127.2(2) | | O6 | C26 | C25 | 127.8(2) |
| O1 | C5 | C6 | 111.01(19) | | O6 | C26 | C27 | 110.4(2) |
| C4 | C5 | C6 | 121.8(2) | | C25 | C26 | C27 | 121.8(2) |
| O2 | C6 | C5 | 108.76(17) | | O5 | C27 | C26 | 109.3(2) |
| C1 | C6 | O2 | 128.01(18) | | C22 | C27 | O5 | 127.7(2) |
| C1 | C6 | C5 | 123.23(19) | | C22 | C27 | C26 | 123.0(2) |
| O2 | C7 | O1 | 108.54(19) | | O5 | C28 | O6 | 108.8(2) |
| N1 | C8 | C4 | 179.6(3) | | N5 | C29 | C25 | 177.3(4) |
| C1 | C9 | C10 | 111.77(15) | | C22 | C30 | C31 | 111.77(16) |
| C18 | C9 | C1 | 110.80(16) | | C39 | C30 | C22 | 108.96(17) |
| C18 | C9 | C10 | 110.41(15) | | C39 | C30 | C31 | 111.46(17) |
| C11 | C10 | C9 | 121.68(17) | | C32 | C31 | C30 | 121.67(19) |
| C11 | C10 | C12 | 119.64(17) | | C32 | C31 | C34 | 120.68(19) |
| C12 | C10 | C9 | 118.67(16) | | C34 | C31 | C30 | 117.65(18) |
| N3 | C11 | C13 | 112.82(18) | | N7 | C32 | C33 | 112.16(19) |
| C10 | C11 | N3 | 121.04(18) | | C31 | C32 | N7 | 120.8(2) |
| C10 | C11 | C13 | 126.00(19) | | C31 | C32 | C33 | 127.0(2) |
| O3 | C12 | N2 | 121.26(19) | | O7 | C34 | N6 | 118.4(2) |
| O3 | C12 | C10 | 123.4(2) | | O7 | C34 | C31 | 124.0(2) |
| N2 | C12 | C10 | 115.38(18) | | N6 | C34 | C31 | 117.54(19) |
| N3 | C14 | C15 | 119.90(18) | | N7 | C35 | C36 | 120.2(2) |
| N3 | C14 | C18 | 119.74(18) | | N7 | C35 | C39 | 119.6(2) |
| C18 | C14 | C15 | 120.34(18) | | C39 | C35 | C36 | 120.2(2) |
| C14 | C15 | C19 | 121.7(2) | | C35 | C36 | C40 | 121.7(2) |
| C16 | C15 | C14 | 116.27(19) | | C37 | C36 | C35 | 116.2(2) |
| C16 | C15 | C19 | 122.0(2) | | C37 | C36 | C40 | 122.1(2) |
| N4 | C16 | C15 | 125.67(19) | | N8 | C37 | C36 | 125.8(2) |
| O4 | C17 | C18 | 115.05(17) | | O8 | C38 | C39 | 114.9(2) |
| N4 | C17 | O4 | 120.58(19) | | N8 | C38 | O8 | 120.3(2) |
| N4 | C17 | C18 | 124.36(19) | | N8 | C38 | C39 | 124.7(2) |
| C14 | C18 | C9 | 121.80(17) | | C35 | C39 | C30 | 122.05(19) |
| C14 | C18 | C17 | 117.03(18) | | C35 | C39 | C38 | 117.0(2) |
| C17 | C18 | C9 | 121.07(17) | | C38 | C39 | C30 | 120.9(2) |
| O4 | C20 | C21 | 109.2(3) | | O8 | C41 | C42 | 109.0(3) |

**Table 16 Hydrogen bond of compound 62**

| D | H | A | d(D-H)/Å | d(H-A)/Å | d(D-A)/Å | D-H-A/° |
|---|---|---|---|---|---|---|
| N2 | H2A | N1¹ | 0.89(2) | 2.33(2) | 3.215(3) | 179(3) |
| N2 | H2B | N4² | 0.90(2) | 2.22(2) | 3.112(3) | 176(3) |
| N3 | H3 | O7 | 0.87(2) | 2.22(2) | 2.960(3) | 143(3) |
| N6 | H6A | N5³ | 0.86(2) | 2.24(2) | 3.089(3) | 171(4) |
| N6 | H6B | O5 | 0.87(2) | 2.42(3) | 3.112(3) | 137(3) |

| | | | | | | |
|---|---|---|---|---|---|---|
| ¹1*-x, -1+y, -z;* ²3/2*-x, -1*/*2+y, -z;* ³2-*x, -1+y,* 1-*z* | | | | | | |

**Table 17 Torsion angle of compound 62**

| A | B | c | D | Angle/° | | A | B | c | D | Angle/° |
|---|---|---|---|---|---|---|---|---|---|---|
| O1 | C5 | C6 | O2 | 0.3(3) | | O6 | C26 | C27 | O5 | -0.1(3) |
| O1 | C5 | C6 | C1 | 179.2(2) | | O6 | C26 | C27 | C22 | 178.8(2) |
| O4 | C17 | C18 | C9 | -1.3(3) | | O8 | C38 | C39 | C30 | -1.4(3) |
| O4 | C17 | C18 | C14 | -177.84(19) | | O8 | C38 | C39 | C35 | -177.9(2) |
| N3 | C14 | C15 | C16 | 179.2(2) | | N7 | C35 | C36 | C37 | 178.9(3) |
| N3 | C14 | C15 | C19 | -0.2(3) | | N7 | C35 | C36 | C40 | -1.5(4) |
| N3 | C14 | C18 | C9 | 3.3(3) | | N7 | C35 | C39 | C30 | 4.4(4) |
| N3 | C14 | C18 | C17 | 179.86(19) | | N7 | C35 | C39 | C38 | -179.1(2) |
| N4 | C17 | C18 | C9 | 178.16(19) | | N8 | C38 | C39 | C30 | 176.7(2) |
| N4 | C17 | C18 | C14 | 1.6(3) | | N8 | C38 | C39 | C35 | 0.1(4) |
| C1 | C2 | C3 | C4 | 0.1(3) | | C22 | C23 | C24 | C25 | 0.7(4) |
| C1 | C9 | C10 | C11 | -106.5(2) | | C22 | C30 | C31 | C32 | -112.2(2) |
| C1 | C9 | C10 | C12 | 74.5(2) | | C22 | C30 | C31 | C34 | 68.1(2) |
| C1 | C9 | C18 | C14 | 108.3(2) | | C22 | C30 | C39 | C35 | 112.2(2) |
| C1 | C9 | C18 | C17 | -68.0(2) | | C22 | C30 | C39 | C38 | -64.1(3) |
| C2 | C1 | C6 | O2 | 179.3(2) | | C23 | C22 | C27 | O5 | 179.7(2) |
| C2 | C1 | C6 | C5 | 0.6(3) | | C23 | C22 | C27 | C26 | 1.0(3) |
| C2 | C1 | C9 | C10 | 85.7(2) | | C23 | C22 | C30 | C31 | 73.0(2) |
| C2 | C1 | C9 | C18 | -37.9(2) | | C23 | C22 | C30 | C39 | -50.6(3) |
| C2 | C3 | C4 | C5 | 0.8(3) | | C23 | C24 | C25 | C26 | 1.0(4) |
| C2 | C3 | C4 | C8 | -178.3(2) | | C23 | C24 | C25 | C29 | -176.7(2) |
| C3 | C4 | C5 | O1 | -179.7(2) | | C24 | C25 | C26 | O6 | -179.5(3) |
| C3 | C4 | C5 | C6 | -1.0(3) | | C24 | C25 | C26 | C27 | -1.6(4) |
| C4 | C5 | C6 | O2 | -178.6(2) | | C25 | C26 | C27 | O5 | -178.3(2) |
| C4 | C5 | C6 | C1 | 0.3(3) | | C25 | C26 | C27 | C22 | 0.6(4) |
| C5 | O1 | C7 | O2 | 0.7(3) | | C26 | O6 | C28 | O5 | 2.6(4) |
| C6 | O2 | C7 | O1 | -0.5(3) | | C27 | O5 | C28 | O6 | -2.6(4) |
| C6 | C1 | C2 | C3 | -0.7(3) | | C27 | C22 | C23 | C24 | -1.6(3) |
| C6 | C1 | C9 | C10 | -96.7(2) | | C27 | C22 | C30 | C31 | -111.0(2) |
| C6 | C1 | C9 | C18 | 139.66(19) | | C27 | C22 | C30 | C39 | 125.4(2) |
| C7 | O1 | C5 | C4 | 178.3(3) | | C28 | O5 | C27 | C22 | -177.2(3) |
| C7 | O1 | C5 | C6 | -0.6(3) | | C28 | O5 | C27 | C26 | 1.7(3) |
| C7 | O2 | C6 | C1 | -178.7(2) | | C28 | O6 | C26 | C25 | 176.5(3) |
| C7 | O2 | C6 | C5 | 0.2(3) | | C28 | O6 | C26 | C27 | -1.6(4) |
| C8 | C4 | C5 | O1 | -0.6(4) | | C29 | C25 | C26 | O6 | -1.9(4) |
| C8 | C4 | C5 | C6 | 178.1(2) | | C29 | C25 | C26 | C27 | 176.0(2) |
| C9 | C1 | C2 | C3 | 176.97(19) | | C30 | C22 | C23 | C24 | 174.7(2) |
| C9 | C1 | C6 | O2 | 1.6(3) | | C30 | C22 | C27 | O5 | 3.4(4) |
| C9 | C1 | C6 | C5 | -177.13(19) | | C30 | C22 | C27 | C26 | -175.2(2) |
| C9 | C10 | C11 | N3 | -5.8(3) | | C30 | C31 | C32 | N7 | -1.2(3) |
| C9 | C10 | C11 | C13 | 178.8(2) | | C30 | C31 | C32 | C33 | -178.0(2) |
| C9 | C10 | C12 | O3 | -135.5(2) | | C30 | C31 | C34 | O7 | -157.0(2) |
| C9 | C10 | C12 | N2 | 44.2(3) | | C30 | C31 | C34 | N6 | 21.6(3) |
| C10 | C9 | C18 | C14 | -16.1(3) | | C31 | C30 | C39 | C35 | -11.6(3) |
| C10 | C9 | C18 | C17 | 167.58(18) | | C31 | C30 | C39 | C38 | 172.1(2) |
| C11 | N3 | C14 | C15 | -167.6(2) | | C32 | N7 | C35 | C36 | -173.9(2) |
| C11 | N3 | C14 | C18 | 10.7(3) | | C32 | N7 | C35 | C39 | 6.0(4) |
| C11 | C10 | C12 | O3 | 45.5(3) | | C32 | C31 | C34 | O7 | 23.4(3) |
| C11 | C10 | C12 | N2 | -134.8(2) | | C32 | C31 | C34 | N6 | -158.1(3) |
| C12 | C10 | C11 | N3 | 173.19(19) | | C34 | C31 | C32 | N7 | 178.5(2) |
| C12 | C10 | C11 | C13 | -2.3(3) | | C34 | C31 | C32 | C33 | 1.7(4) |
| C14 | N3 | C11 | C10 | -9.5(3) | | C35 | N7 | C32 | C31 | -7.7(4) |
| C14 | N3 | C11 | C13 | 166.5(2) | | C35 | N7 | C32 | C33 | 169.6(3) |
| C14 | C15 | C16 | N4 | 0.5(3) | | C35 | C36 | C37 | N8 | 0.2(5) |
| C15 | C14 | C18 | C9 | -178.33(18) | | C36 | C35 | C39 | C30 | -175.7(2) |
| C15 | C14 | C18 | C17 | -1.8(3) | | C36 | C35 | C39 | C38 | 0.8(4) |
| C16 | N4 | C17 | O4 | 179.1(2) | | C37 | N8 | C38 | O8 | 177.2(2) |
| C16 | N4 | C17 | C18 | -0.4(3) | | C37 | N8 | C38 | C39 | -0.8(4) |
| C17 | O4 | C20 | C21 | -118.5(4) | | C38 | O8 | C41 | C42 | -158.2(3) |
| C17 | N4 | C16 | C15 | -0.8(3) | | C38 | N8 | C37 | C36 | 0.6(5) |
| C18 | C9 | C10 | C11 | 17.3(3) | | C39 | C30 | C31 | C32 | 10.0(3) |
| C18 | C9 | C10 | C12 | -161.65(16) | | C39 | C30 | C31 | C34 | -169.68(18) |
| C18 | C14 | C15 | C16 | 0.8(3) | | C39 | C35 | C36 | C37 | -0.9(4) |
| C18 | C14 | C15 | C19 | -178.5(2) | | C39 | C35 | C36 | C40 | 178.6(3) |
| C19 | C15 | C16 | N4 | 179.9(2) | | C40 | C36 | C37 | N8 | -179.3(3) |
| C20 | O4 | C17 | N4 | -16.4(4) | | C41 | O8 | C38 | N8 | -1.9(4) |
| C20 | O4 | C17 | C18 | 163.0(3) | | C41 | O8 | C38 | C39 | 176.3(3) |

**Table 18 Hydrogen atomic coordinates (Å×10⁴) and isotropic displacement parameters (Å2×10³) of compound 62**

| Atom | *x* | *y* | *z* | U(eq) |
|---|---|---|---|---|
| H2A | 6200(12) | -2290(30) | 1065(15) | 42 |
| H2B | 6632(13) | -1940(30) | 748(14) | 42 |
| H3 | 8341(12) | 2360(30) | 2350(13) | 41 |
| H2 | 6902 | 4039 | 689 | 36 |
| H3A | 6095 | 5742 | 93 | 41 |
| H7A | 4650 | -151 | -798 | 66 |
| H7B | 4780 | -26 | -1463 | 66 |
| H9 | 6802 | 51 | 303 | 27 |
| H13A | 7776 | 1667 | 3020 | 69 |
| H13B | 8244 | 382 | 3064 | 69 |
| H13C | 7529 | -25 | 2794 | 69 |
| H16 | 8864 | 3162 | 615 | 37 |
| H19A | 9046 | 3942 | 2236 | 73 |
| H19B | 9461 | 3517 | 1888 | 73 |
| H19C | 9327 | 2281 | 2338 | 73 |
| H20A | 6831 | 1016 | -1663 | 85 |
| H20B | 7236 | 2502 | -1274 | 85 |
| H21A | 5930 | 2161 | -1834 | 196 |
| H21B | 6230 | 3223 | -2180 | 196 |
| H21C | 6326 | 3617 | -1389 | 196 |
| H6A | 9191(14) | 3300(40) | 4170(20) | 68 |
| H6B | 8930(17) | 3900(50) | 4633(15) | 68 |
| H7 | 6585(12) | 6490(40) | 2593(13) | 51 |
| H23 | 8045 | 8669 | 3991 | 44 |
| H24 | 8742 | 10639 | 4528 | 51 |
| H28A | 10386 | 5315 | 6295 | 91 |
| H28B | 10089 | 5736 | 6786 | 91 |
| H30 | 8262 | 4888 | 4720 | 33 |
| H33A | 7217 | 5922 | 1938 | 71 |
| H33B | 6781 | 4636 | 1966 | 71 |
| H33C | 7500 | 4282 | 2239 | 71 |
| H37 | 6136 | 7861 | 4318 | 57 |
| H40A | 5907 | 8207 | 2629 | 102 |
| H40B | 5518 | 7982 | 3029 | 102 |
| H40C | 5643 | 6580 | 2650 | 102 |
| H41A | 7927 | 6033 | 6462 | 81 |
| H41B | 7870 | 7777 | 6241 | 81 |
| H42A | 8935 | 7933 | 6746 | 132 |
| H42B | 9013 | 6160 | 6896 | 132 |
| H42C | 8817 | 7213 | 7356 | 132 |

### Example: In-vitro activity test

### 1. Experimental principle:

By the characteristic of chemiluminescence reaction produced by combination of luciferase and substrate, a Gal4 DNA binding domain (DBD) fused plasmid containing a mineralocorticoid receptor (MR) ligand binding domain (LBD) and a firefly luciferase reporter gene plasmid under the control of Gal4 UAS (upstream activation sequence) were transfected into human embryonic kidney cells (HEK293). Changes of glucocorticoid receptor activity before and after stimulation or effects of different stimulations on glucocorticoid receptor activity were judged by a level of firefly luciferase activity.

### 2. Experimental method:

### 1. Preparation and treatment of compounds

### 1.1 Preparation of DMSO stock solution of compounds

All compounds were dissolved in DMSO to prepare a 25 mM stock solution, and stored in a refrigerator at - 20°C.

### 1.2 Preparation of working solution

1) A compound to be tested was subjected to 3-times gradient dilution with the DMSO, with 10 concentration gradients and an initial concentration of 10 mM.
2) A positive compound (Finerenone) was subjected to 3-times gradient dilution with the DMSO, with 10 concentration gradients and an initial concentration of 1 mM.
3) 1000 × positive control (1 mM, Finerenone) and 1000 × negative control (100% DMSO) were prepared.
4) A compound plate was sealed and shaken for 5 minutes.

### 2. Preparation of cell suspension

1) All cells were cultured by ATCC standard operation, and HEK293T was experimented in an exponential growth phase.
2) A culture medium supernatant was gently discarded. The cells were washed with PBS twice.
(3) The cells were digested with pancreatin digestive juice, the digestion was stopped with a complete culture solution, and then the cells were collected and counted.
(4) 6 X106 HEK293T cells were inoculated into a 100 mm cell culture dish.
(5) The culture dish inoculated with the cells was cultured in a 5% CO₂ incubator at 37°C for 16 hours overnight.

### 3. Cell transfection

1) Lipofectamine^{®} 3000&P3000 transfection reagents were placed at room temperature.
2) The Lipofectamine^{®} 3000 reagent was added into an Opti-MEM^{™} culture medium, and meanwhile, the P3000, the plasmid and the Opti-MEM^{™} culture medium were added into another tube, taking care not to touch the tube wall.
3) The mixture was mixed evenly by a pipette, and allowed to stand at room temperature for 5 minutes.
4) The plasmid was added into the diluted transfection reagent, mixed evenly by a pipette, and allowed to stand at room temperature for 20 minutes.
5) The transfection reagent mixed with the plasmid was added into a 60 mm cell culture dish.
6) The culture dish was cultured in a 5% CO₂ incubator at 37°C for 5 hours to 6 hours.

### 4. Compound treatment

1) 25 nL of diluted compound was transferred to a cell culture plate with Echo655.
2) The cells (referring to step 1.3) were inoculated into a 384 cell culture plate, with 17,000 cells, 25 µL of phenol red-free DMEM culture medium containing 5% CFBS and 0.8 nM Aldosteron per well.
(3) The cell culture plate was cultured in a 5% CO₂ incubator at 37°C for 18 hours to 20 hours overnight.

### 5. Compound detection

1) A Britelite plus detection reagent was placed at room temperature.
2) The 384 cell plate was placed at room temperature.
3) 25 µL of Britelite plus detection reagent was added into each well of the cell culture plate.
4) A luminescence was detected with Envision.

### 6. Result treatment:

1) a firefly luciferase signal was obtained by reading the Luminescence to calculate an inhibition rate;
2) % inhibition rate = 100 - [(RLU compound - avgRLU positive control)/(RLU negative control - avgRLU positive control) × 100%, wherein the avgRLU positive control was an average value of Luminescences of all positive control wells in the whole plate; the avgRLU negative control was an average value of Luminescences of all negative control wells in the whole plate; and the RLU compound was Luminescence values of compounds to be tested with different concentrations; and
3) IC50 of the compound was calculated by Graphpad8.0 Drawing software.

### 7. Experimental results:

**Table 19 In-vitro activity data of compounds of the present invention**

| Serial number of embodiment | MR IC₅₀ (nM) |
|---|---|
| Example 1 | 192.3 |
| Example 2 | 48.9 |
| Example 3 | 113.1 |
| Example 4 | 111.2 |
| Example 5 | 235.5 |
| Example 6 | 21.6 |
| Example 7 | 322.4 |
| Example 8 | 20.4 |
| Example 9 | 661.1 |
| Example 10 | 348.1 |
| Example 11 | 92.1 |
| Example 12 | 215.0 |
| Example 13 | 232.2 |
| Example 14 | 46.2 |
| Example 15 | 18.2 |
| Example 16 | 34.1 |
| Example 17 | 19.2 |
| Example 18 | 28.5 |
| Example 19 | 27.7 |
| Example 20 | 15.2 |
| Example 21 | 20.2 |
| Example 22 | 8.7 |
| Example 23 | 24.8 |
| Example 24 | 7.9 |
| Example 25 | 37.5 |
| Example 32 | 8.0 |
| Example 36 | 11.5 |
| Example 40 | 12.7 |
| Example 62 | 14.8 |

### Conclusion:

According to the experimental results in Table 19, the compounds of the present invention had good mineralocorticoid receptor (MR) antagonistic activity, and could be used as an effective mineralocorticoid receptor antagonist.

### Example: Study on pharmacokinetic test of rats

This experiment was intended to evaluate a pharmacokinetic behavior of an implementing compound after intravenous injection or intragastric administration in the rats. Intravenous injection administration: a test compound was prepared into 0.2 mg/mL clear solution, a solvent was 10% ethanol, 40% polyethylene glycol 400 and 50% water, and after administration, plasma was collected 0.083 hour, 0.25 hour, 0.5 hour, 1 hour, 2 hours, 4 hours, 8 hours and 24 hours later. Intragastric administration: a test compound was prepared into 1 mg/mL clear solution, a solvent was 10% ethanol/40% polyethylene glycol 400/50% water, and after administration, plasma was collected 0.25 hour, 0.5 hour, 1 hour, 2 hours, 4 hours, 8 hours and 24 hours later.

A concentration of the test compound in the plasma was determined by high-performance liquid chromatography-tandem mass spectrometry (LC-MS/MS). Retention time, chromatogram collection and chromatogram integration of compounds and internal standards were treated by software Analyst (Applied Biosystems), and data statistics was treated by software Analyst (Applied Biosystems).

A plasma concentration was treated by a non-atrioventricular model of Winnonlintm Version 6.1 (Pharsight, Mountain View, CA) pharmacokinetic software, and pharmacokinetic parameters were calculated by a linear logarithmic trapezoid method.

Pharmacokinetic parameters of the compounds in the rats with an intravenous injection dose of 1 mg/kg and an intragastric and oral dose of 5 mg/kg were as shown in Table 20 below.

**Table 20 Pharmacokinetic (PK) data of SD rats**

| Administration mode | Parameter | **36** | **62** | (Finerenone) |
|---|---|---|---|---|
| Intravenous drip (1 mg/kg) | CL (mL/min/kg) | 0.14 | 0.13 | 0.34 |
| | t_{1/2} (h) | 12.09 | 10.06 | 9.62 |
| | AUCₗₐₛₜ (h*ng/mL) | 94066.18 | 106105.60 | 91211.00 |
| Intragastric and oral administration (5 mg/kg) | Cₘₐₓ (ng/mL) | 37000 | 24000 | 23800 |
| | AUCₗₐₛₜ (h*ng/mL) | 364780 | 361487.92 | 306577 |
| | F (%) | 73.74 | 78.09 | 60.3 |

According to the experimental results in Table 20, the compounds of the present invention had good in-vivo pharmacokinetic property.

### Example: Study on pharmacokinetic test of mice

This experiment was intended to evaluate a pharmacokinetic behavior of a compound after intravenous injection or intragastric administration in the mice. Intravenous injection administration: a test compound was prepared into 0.2 mg/mL clear solution, a solvent was 10% ethanol/40% polyethylene glycol 400/50% ultrapure water, and after administration, plasma was collected 0.083 hour, 0.25 hour, 0.5 hour, 1 hour, 2 hours, 4 hours, 8 hours, 24 hours and 48 hours later.

A concentration of the test compound in the plasma was determined by high-performance liquid chromatography-tandem mass spectrometry (LC-MS/MS). Retention time, chromatogram collection and chromatogram integration of compounds and internal standards were treated by software Analyst (Applied Biosystems), and data statistics was treated by software Analyst (Applied Biosystems).

A plasma concentration was treated by a non-atrioventricular model of Winnonlintm Version 6.1 (Pharsight, Mountain View, CA) pharmacokinetic software, and pharmacokinetic parameters were calculated by a linear logarithmic trapezoid method.

Pharmacokinetic parameters of the compounds in the mice with an intravenous injection dose of 1 mg/kg were as shown in Table 21 below.

**Table 21 Pharmacokinetic (PK) data of CD1 mice**

| Administration mode | Parameter | **36** | **38** | **62** | Finerenone |
|---|---|---|---|---|---|
| Intravenous drip (1 mg/kg) | AUCₗₐₛₜ (h*ng/mL) | 205717.65 | 2552.24 | 201109.89 | 142177.43 |

### Example 70: Pharmacodynamic effects of compounds 36 and 62 on diabetic nephropathy mouse model

### 1. Research purpose

The pharmacodynamic effects of the compounds 36 and 62 on the diabetic nephropathy mouse model were evaluated.

### 2. Drug preparation

Solvents and positive drugs should be prepared on the same day by methods as shown in Table 70-1.

**Table 70-1 Drug preparation**

| Compound | Preparation method | Concentration (mg/mL) |
|---|---|---|
| Solvent | 10% ethanol/40% PEG400/50% water | - |
| Finerenone: 3 mg/kg | 1.2 mg of Finerenone was added into 0.396 mL of ethanol, stirred and ultrasonically treated for dissolution, added with 1.59 mL of PEG400, stirred and ultrasonically treated, and then added with 1.984 mL of deionized water, stirred and ultrasonically treated to obtain a uniform solution. | 0.3 |
| Compound 62: 10 mg/kg | 4 mg of compound 62 was added into 0.398 mL of ethanol, stirred and ultrasonically treated for dissolution, added with 1.592 mL of PEG400, stirred and ultrasonically treated, and then added with 1.99 mL of deionized water, stirred and ultrasonically treated to obtain a uniform solution. | 1 |
| Compound 62: 3 mg/kg | 0.3 mg/mL compound 62 solution was obtained by diluting 1 mg/mL compound 62 by 3.33 times. | 0.3 |
| Compound 62: 1 mg/kg | 0.1 mg/mL compound 62 solution was obtained by diluting 0.3 mg/mL compound 62 by 3 times. | 0.1 |
| Compound 36: | 4 mg of compound 36 was added into 0.398 mL of ethanol, | 1 |
| 10 mg/kg | stirred and ultrasonically treated for dissolution, added with 1.592 mL of PEG400, stirred and ultrasonically treated, and then added with 1.99 mL of deionized water, stirred and ultrasonically treated to obtain a uniform solution. | |
| Compound 36: 3 mg/kg | 0.3 mg/mL compound 36 solution was obtained by diluting 1 mg/mL compound 36 by 3.33 times. | 0.3 |
| Compound 36: 1 mg/kg | 0.1 mg/mL compound 36 solution was obtained by diluting 0.3 mg/mL compound 36 by 3 times. | 0.1 |

### 3. Experimental animals, and feeding and management

### 3.1 Experimental animals

Strain: C57BL/6 mice, and KK-Ay mice
Weeks of age: the animals were raised to 6 weeks to 8 weeks of age, and the experiment was started when the animals were 7 weeks to 9 weeks of age
Sex: male
Weight: C57BL/6 mice: 21g to 23 g, and KK-Ay mice: 25 g to 30 g
Number: 72 experimental animals

### 3.2 Animal feeding

### 3.2.1 Quarantine

Quarantine and adaptation periods were 7 days in total, a routine health examination was completed by a veterinarian, and animals with abnormal performances were eliminated before the experiment.

### 3.2.2 Animal feeding conditions

The experimental animals were raised in an SPF constant temperature and humidity laminar clean room of an animal center (AAALAC certification unit) of Pharmaron (Beijing) New Drug Technology Co., Ltd., and kept in separate cages. A feeding room was set at a temperature of 22°C to 25°C and a humidity of 40% to 80% under a light alternately turned on and off for 12 hours. The cages were made of polycarbonate. A soft corncob padding sterilized and cleaned under a high pressure was used, and replaced twice a week. Drinking water was subjected to autoclaved sterilization under a high pressure, and food was irradiated by a cobalt 60 ray. The animals could take sterile food and drinking water freely.

### 3.2.3 Animal serial number

Each cage had a cage label, which indicated the number, sex, strain, receiving time, group and experiment start time of the animals. Each animal was marked with a separate animal serial number at a tail.

### 4. Grouping and administration of experimental animals

On the day of administration, the animals were randomly divided into 9 groups according to their weights, comprising a model group (KK-Ay + solvent), a Finerenone group (KK-Ay + Finerenone: 3 mg/kg), a low-dose compound 62 group (KK-Ay + compound 62: 1 mg/kg), a middle-dose compound 62 group (KK-Ay + compound 62: 3 mg/kg) and high-dose compound 62 group (KK-Ay + compound 62: 10 mg/kg), a low-dose compound 36 group (KK-Ay + compound 36: 1 mg/kg), a middle-dose compound 36 group (KK-Ay + compound 36: 3 mg/kg) and a high-dose compound 36 group (KK-Ay + compound 36: 10 mg/kg). Specific grouping and administration schemes referred to Table 70-2.

**Table 70-2 Grouping and administration of experimental animals**

| Group | Strain | Feedstuff | Drug | Number of animals (N) | Administration route | Administration dosage (mg/kg) |
|---|---|---|---|---|---|---|
| 1 | C57BL/6 | Normal | Solvent | 8 | Oral administration | - |
| | | feedstuff | | | | |
| 2 | KK-Ay | 4%Nacl | Solvent | 8 | Oral administration | - |
| 3 | KK-Ay | 4%Nacl | Finerenone | 8 | Oral administration | 3 |
| 4 | KK-Ay | 4%Nacl | Compound 62 | 8 | Oral administration | 1 |
| 5 | KK-Ay | 4%Nacl | Compound 62 | 8 | Oral administration | 3 |
| 6 | KK-Ay | 4%Nacl | Compound 62 | 8 | Oral administration | 10 |
| 7 | KK-Ay | 4%Nacl | Compound 36 | 8 | Oral administration | 1 |
| 8 | KK-Ay | 4%Nacl | Compound 36 | 8 | Oral administration | 3 |
| 9 | KK-Ay | 4%Nacl | Compound 36 | 8 | Oral administration | 10 |

### 7 Experimental method and detection index

### 7.1 Urine collection

When the experiment was started, 24-hour urine of the mice was collected by a metabolic cage in 2^{nd}, 4^{th}, 6^{th} and 8^{th} weeks before and after modeling, and microalbumin (MALB) and creatinine (CREA) contents of the urine were detected by CANON TBA-120FR automatic blood biochemistry to calculate (MALB/CREA).

### 7.2 Insulin content and serum ion detection

When the experiment was ended, the mice were euthanized, the blood was taken by heart puncture and put into an anticoagulant-free EP tube, allowed to stand at room temperature for 0.5 hour, and then centrifuged at 4°C and 6000 g for 15 minutes, and the serum was collected and transferred to a new EP tube, and stored in a refrigerator at -80°C. An insulin content of the serum and serum ions were detected by an ELISA method.

### 7.3 Tissue collection

After the experiment was ended, the kidneys of all animals were dissected and collected, fixed in formalin, embedded and made into sections for PAS staining and Masson staining, and then quantitatively analyzed.

### 8. Statistics

Experimental results were expressed as "mean value ± standard deviation". The data of each group were statistically analyzed by GraphPad 8.0 software, and p<0.05 was considered to be statistically significant.

### 9. Results

The compound 62 and the compound 36 could both significantly promote urinary sodium excretion, significantly reduce a urinary protein/creatinine ratio, and improve diabetic nephropathy and hypertension.

### Example 71: Study on in-vitro CPY enzyme inhibition

Results showed that IC50 (µM) data of main drug metabolizing enzyme CYP3A4 in human body were as follows: CYP3A4-5M and CYP3A4-5T were Finerenone (9.98, 8.02), compound 36 (22.86, 15.78) and compound 62 (13.66, 5.35) respectively.

Therefore, the compounds 36 and 62 had higher safety than the Finerenone. The compound 36 and the Finerenone had higher median inhibition concentration on two subtype enzymes CYP3A4-5M/CYP3A4-5T of main metabolic enzyme CYP3A4 (which metabolized about 50% drug) in human body.

In the descriptions of the specification, the descriptions with reference to the terms "one embodiment", "some embodiments", "example", "specific example" or "some examples", etc., refer to that specific features, structures, materials, or characteristics described with reference to the embodiments or examples are included in at least one embodiment or example of the present invention. In the specification, the schematic representation of the above terms does not necessarily refer to the same embodiment or example. Moreover, the specific features, structures, materials or characteristics described may be combined in any one or more embodiments or examples in a suitable manner. In addition, in the case of having no mutual contradiction, those skilled in the art may join and combine different embodiments or examples described in the specification and the characteristics of the different embodiments or examples.

Although the embodiments of the present invention have been shown and described above, it may be understood that the above embodiments are exemplary and cannot be understood as limiting the present invention, and those of ordinary skills in the art may make changes, modifications, substitutions and variations to the above embodiments within the scope of the present invention.

## Claims

1. A compound of formula (I) and a stereoisomer, geometric isomer, tautomer, nitrogen oxide, hydrate, solvate, metabolite, ester, pharmaceutically acceptable salt or prodrug thereof; 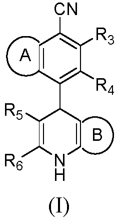 wherein, 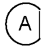 is 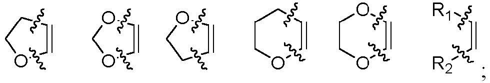
R₁, R₂, R₃ and R₄ are independently hydrogen, deuterium, amino, hydroxyl, sulfhydryl, cyano, nitro, C₁₋₆ alkyl, C₁₋₆ alkoxy, C₁₋₆ haloalkyl, C₁₋₆ haloalkoxy, C₁₋₆ alkylamino, carboxyl, C₁₋₆ alkylacyl, C₁₋₆ alkylsulfonyl, aminoacyl, aminosulfonyl, C₃₋₈ cycloalkyl, C₆₋₁₀ aryl, heterocyclyl consisting of 3 to 8 atoms or heteroaryl consisting of 5 to 10 atoms;
R₅ is 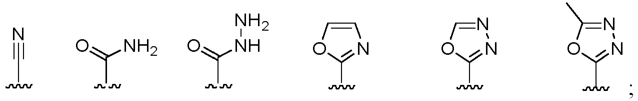
R₆ is hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms; 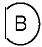 is 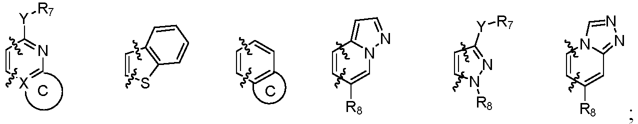
X is C or N;
Y is O or S;
R₇ is C₁₋₆ alkyl, C₃₋₈ cycloalkyl, heterocyclyl consisting of 3 to 8 atoms, heteroaryl consisting of 5 to 6 atoms, C₃₋₈ cycloalkyl C₁₋₆ alkyl, (heterocyclyl consisting of 3 to 8 atoms) C₁₋₆ alkyl, (heteroaryl consisting of 5 to 6 atoms) C₁₋₆ alkyl, phenyl or phenyl C₁₋₆ alkyl; and R₇ is unsubstituted or substituted by 1, 2, 3 or 4 Rz;
each Rz is independently O, deuterium, fluorine, chlorine, bromine, iodine, hydroxyl, cyano, NH₂, C₁₋₆ alkyl, C₁₋₆ alkoxy, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; wherein each Rz is independently unsubstituted or substituted by 1, 2, 3 or 4 Rw;
each Rw is independently O, deuterium, fluorine, chlorine, bromine, iodine, hydroxyl, cyano, amino, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; 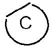
is 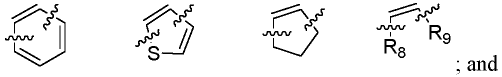
R₈ and R₉ are hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms.

2. A compound of formula (I) and a stereoisomer, geometric isomer, tautomer, nitrogen oxide, hydrate, solvate, metabolite, ester, pharmaceutically acceptable salt or prodrug thereof; 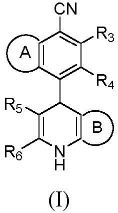 wherein, 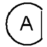 is 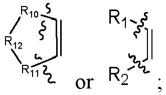
R₁, R₂, R₃ and R₄ are independently hydrogen, deuterium, amino, hydroxyl, sulfhydryl, cyano, nitro, C₁₋₆ alkyl, C₁₋₆ alkoxy, C₁₋₆ haloalkyl, C₁₋₆ haloalkoxy, C₁₋₆ alkylamino, carboxyl, C₁₋₆ alkylacyl, C₁₋₆ alkylsulfonyl, aminoacyl, aminosulfonyl, C₃₋₈ cycloalkyl, C₆₋₁₀ aryl, heterocyclyl consisting of 3 to 8 atoms or heteroaryl consisting of 5 to 10 atoms;
R₅ is 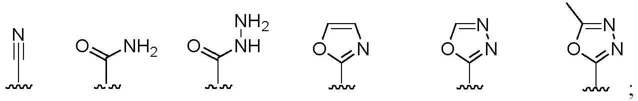 R₆ is hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms; 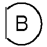 is 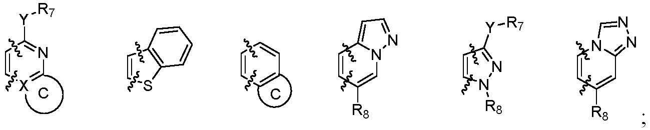
X is C or N;
Y is O or S;
R₇ is C₁₋₆ alkyl, C₃₋₈ cycloalkyl, heterocyclyl consisting of 3 to 8 atoms, heteroaryl consisting of 5 to 6 atoms, C₃₋₈ cycloalkyl C₁₋₆ alkyl, (heterocyclyl consisting of 3 to 8 atoms) C₁₋₆ alkyl, (heteroaryl consisting of 5 to 6 atoms) C₁₋₆ alkyl, phenyl or phenyl C₁₋₆ alkyl; and R₇ is unsubstituted or substituted by 1, 2, 3 or 4 Rz;
each Rz is independently O, deuterium, fluorine, chlorine, bromine, iodine, hydroxyl, cyano, NH₂, C₁₋₆ alkyl, C₁₋₆ alkoxy, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; wherein each Rz is independently unsubstituted or substituted by 1, 2, 3 or 4 Rw;
each Rw is independently O, deuterium, fluorine, chlorine, bromine, iodine, hydroxyl, cyano, amino, C₁₋₆ alkyl, C₁₋₆ alkoxy, C₁₋₆ haloalkyl, C₁₋₆ haloalkoxy, C₁₋₆ alkylamino, C₁₋₆ alkylsulfonyl, C₁₋₆ alkylacyl, C₃₋₈ cycloalkyl, heterocyclyl consisting of 3 to 8 atoms, heteroaryl consisting of 5 to 6 atoms, or C₆₋₁₀ aryl; 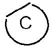 is 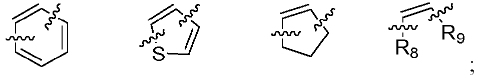
R₈ and R₉ are hydrogen, deuterium, C₁₋₆ alkyl, C₁₋₆ alkoxy, C₁₋₆ haloalkyl, C₁₋₆ haloalkoxy, C₁₋₆ alkylamino, C₃₋₈ cycloalkyl, C₆₋₁₀ aryl, heterocyclyl consisting of 3 to 8 atoms or heteroaryl consisting of 5 to 10 atoms;
R₁₀ and R₁₁ are independently selected from -CH₂- or O, and at least one of which is O;
R₁₂ is -CH₂- or -CH₂-CH₂-;
when R₁₂ is -CH₂-, R₁₀ and R₁₁ are independently selected from -CH₂- or O, and at least one of which is O; and when R₁₂ is -CH₂-CH₂-, R₁₀ and R₁₁ are both O, or R₁₀ is -CH₂- and R₁₁ is O.

3. The compound according to claim 1, wherein the compound is selected from the group consisting of compounds represented by formula (Ia) or formula (Ib); and further preferred is the compound represented by formula (Ia);

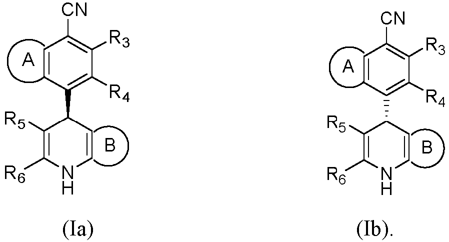

4. The compound according to any one of claims 1 to 3, wherein R₁, R₂, R₃ and R₄ are independently hydrogen, deuterium, amino, hydroxyl, sulfhydryl, cyano, nitro, C₁₋₄ alkyl, C₁₋₄ alkoxy, C₁₋₄ haloalkyl, C₁₋₄ haloalkoxy, C₁₋₄ alkylamino, carboxyl, C₁₋₄ alkylacyl, C₁₋₄ alkylsulfonyl, aminoacyl, aminosulfonyl, C₃₋₆ cycloalkyl, C₆₋₁₀ aryl, heterocyclyl consisting of 3 to 6 atoms or heteroaryl consisting of 5 to 6 atoms;
R₆ is hydrogen, deuterium, C₁₋₄ alkyl, C₁₋₄ alkoxy, C₁₋₄ haloalkyl, C₁₋₄ haloalkoxy, C₁₋₄ alkylamino, C₃₋₆ cycloalkyl, C₆₋₁₀ aryl, heterocyclyl consisting of 3 to 6 atoms or heteroaryl consisting of 5 to 6 atoms;
R₈ and R₉ are independently hydrogen, deuterium, halogen, cyano, C₁₋₄ alkoxyacyl, carboxyl, C₁₋₄ alkyl, C₁₋₄ alkoxy, C₁₋₄ haloalkyl, C₁₋₄ haloalkoxy, C₁₋₄ alkylamino, C₁₋₄ alkanoyl, C₁₋₄ alkylsulfonyl, aminoacyl or aminosulfonyl.

5. The compound according to any one of claims 1 to 3, wherein R₁, R₂, R₃ and R₄ are independently hydrogen, deuterium, amino, hydroxyl, sulfydryl, cyano, nitro, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethoxy, difluoromethoxy, monofluoromethoxy, methylamino, dimethylamino, carboxyl, methacryl, ethylacyl, methylsulfonyl, aminoacyl or aminosulfonyl;
R₆ is hydrogen, deuterium, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethyl, difluoromethoxy, monofluoromethoxy, methylamino, dimethylamino, cyclopropyl, cyclobutyl, cyclopentyl, cyclohexyl, phenyl, naphthyl, epoxyethyl, pyrrolidinyl, piperidinyl, piperazinyl, morpholinyl, pyridyl, pyrrolyl, thiazolyl, pyrazolyl or pyrimidinyl; and
R₈ is hydrogen, deuterium, cyano, methylacyl, ethylacyl, propylacyl, methoxyacyl, ethoxyacyl, propoxyacyl, carboxyl, methyl, ethyl, propyl, butyl, methoxy, ethoxy, propoxy, butoxy, trifluoromethyl, difluoromethyl, monofluoromethyl, 2,2-difluoroethyl, 1,2-difluoroethyl, trifluoroethyl, trifluoromethyl, difluoromethoxy, monofluoromethoxy, methylamino or dimethylamino.

6. The compound according to any one of claims 1 to 3, wherein the compound is selected from the group consisting of compounds represented by formula (II);

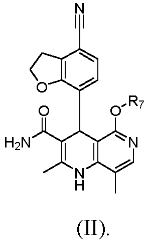

7. The compound according to any one of claims 1 to 3, wherein the compound is selected from the group consisting of compounds represented by formula (IIa) or formula (IIb);

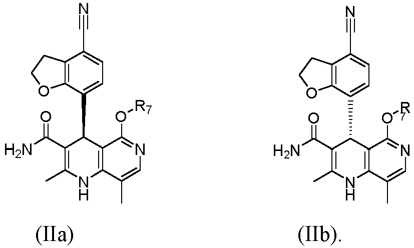

8. The compound according to any one of claims 1 to 7, wherein R₇ is C₃₋₆ cycloalkyl, heterocyclyl consisting of 3 to 6 atoms, heteroaryl consisting of 5 to 6 atoms, C₃₋₆ cycloalkyl C₁₋₄ alkyl, (heterocyclyl consisting of 3 to 6 atoms) C₁₋₄ alkyl or (heteroaryl consisting of 5 to 6 atoms) C₁₋₄ alkyl; wherein R₇ is unsubstituted or substituted by 1, 2, 3 or 4 Rz.

9. The compound according to any one of claims 1 to 7, wherein R₇ is C₁₋₃ alkyl, wherein R₇ is unsubstituted or substituted by 1, 2, 3 or 4 Rz; and preferably, R₇ is methyl, ethyl or isopropyl, wherein R₇ is unsubstituted or substituted by 1, 2, 3 or 4 Rz.

10. The compound according to any one of claims 1 to 7, wherein R₇ is cyclopropyl, cyclobutyl, cyclopentyl, cyclohexyl, oxiranyl, azacyclobutyl, oxacyclobutyl, pyrrolidinyl, tetrahydrofuryl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl, cyclopropyl methyl, cyclopropyl ethyl, cyclobutyl methyl, cyclobutyl ethyl, cyclopentyl methyl, cyclopentyl ethyl, cyclohexyl methyl, cyclohexyl ethyl, azacyclobutyl methyl, azacyclobutyl ethyl, oxacyclobutyl methyl, oxacyclobutyl ethyl, pyrrolidinyl methyl, pyrrolidinyl ethyl, tetrahydrofuryl methyl, tetrahydrofuryl ethyl, tetrahydrothienyl methyl, tetrahydrothienyl ethyl, piperidinyl methyl, piperidinyl ethyl, piperazinyl methyl, piperazinyl ethyl, morpholinyl methyl, morpholinyl ethyl, pyrrolyl methyl, pyrrolyl ethyl, furyl methyl, furyl ethyl, thienyl methyl, thienyl ethyl, thiazolyl methyl, thiazolyl ethyl, pyrazolyl methyl, pyrazolyl ethyl, imidazolyl methyl, imidazolyl ethyl, triazolyl methyl, triazolyl ethyl, tetrazolyl methyl, tetrazolyl ethyl, pyridyl methyl, pyridyl ethyl, pyrimidinyl methyl or pyrimidinyl ethyl; wherein R₇ is unsubstituted or substituted by 1, 2, 3 or 4 Rz.

11. The compound according to any one of claims 1 to 8, wherein each Rz is independently O, deuterium, fluorine, chlorine, bromine, iodine, hydroxyl, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkylacyl, C₃₋₆ cycloalkyl, heterocyclyl consisting of 3 to 6 atoms, heteroaryl consisting of 5 to 6 atoms or C₆₋₁₀ aryl; wherein each Rz is independently unsubstituted or substituted by 1, 2, 3 or 4 Rw; each Rw is independently O, deuterium, fluorine, chlorine, bromine, iodine, hydroxyl, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkylacyl, C₃₋₆ cycloalkyl, heterocyclyl consisiting of 3 to 6 atoms, heteroaryl consisting of 5 to 6 atoms or C₆₋₁₀ aryl.

12. The compound according to any one of claims 1 to 8, wherein each Rz is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl; wherein each Rz is independently unsubstituted, or substituted by 1, 2, 3 or 4 Rw; each Rw is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl.

13. The compound according to claim 1, wherein the compound is selected from the group consisting of compounds represented by formula (III): 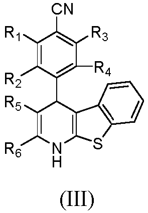 wherein, R₅ is selected from

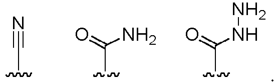

14. The compound according to claim 13, wherein:
R₁, R₂, R₃ and R₄ are independently hydrogen, deuterium or C₁₋₆ alkoxy;
R₅ is 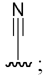
R₆ is selected from hydrogen, deuterium or C₁₋₆ alkyl.

15. The compound according to claim 13, wherein:
R₁, R₂, R₃ and R₄ are independently hydrogen, deuterium or C₁₋₃ alkoxy;
R₅ is 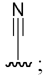
R₆ is selected from hydrogen, deuterium or C₁₋₃ alkyl.

16. The compound according to claim 14, wherein:
R₁, R₂, R₃ and R₄ are independently hydrogen, deuterium or methoxy;
R₅ is 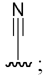
R₆ is selected from hydrogen, deuterium or methyl.

17. The compound according to claim 1, wherein the compound is selected from the group consisting of compounds represented by formula (IV): 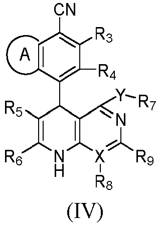 wherein, 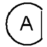 is 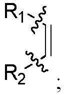
R₁, R₂, R₃ and R₄ are independently hydrogen, deuterium or C₁₋₆ alkoxy;
R₅ is 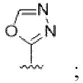
R₆ is C₁₋₆ alkyl;
R₇ is C₁₋₆ alkyl;
R₈ and R₉ are hydrogen, deuterium or C₁₋₆ alkyl;
Y is O; and
X is C.

18. The compound according to claim 17, wherein:
R₁, R₂, R₃ and R₄ are independently hydrogen, deuterium or C₁₋₃ alkoxy;
R₆ is C₁₋₃ alkyl;
R₇ is C₁₋₃ alkyl; and
R₈ and R₉ are hydrogen, deuterium or C₁₋₃ alkyl.

19. The compound according to claim 1, wherein the compound is selected from the group consisting of compounds represented by formula (V): 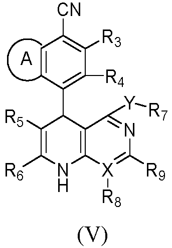 wherein, 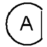 is 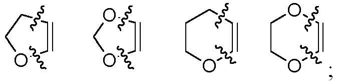
R₃ and R₄ are independently hydrogen or deuterium;
R₅ is 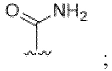
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted or substituted by 1, 2, 3 or 4 Rz; and each Rz is independently halogen;
R₈ and R₉ are hydrogen, deuterium or C₁₋₆ alkyl;
X is C; and
Y is O.

20. The compound according to claim 19, wherein:
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted or substituted by 1, 2, 3 or 4 Rz; and each Rz is independently fluorine, chlorine, bromine or iodine; and
R₈ and R₉ are hydrogen, deuterium or C₁₋₃ alkyl.

21. The compound according to claim 19, wherein:
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted or substituted by 1, 2, 3 or 4 Rz; and each Rz is independently fluorine; and
R₈ and R₉ are hydrogen or methyl.

22. The compound according to any one of claims 1 to 3, wherein the compound is selected from the group consisting of compounds represented by formula (V): 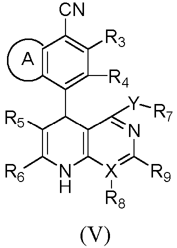 wherein, 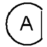 is 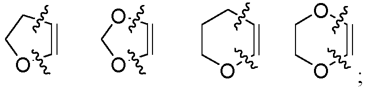
R₃ and R₄ are independently hydrogen or deuterium;
R₅ is 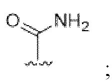
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted or substituted by 1, 2, 3 or 4 Rz; and each Rz is independently halogen or C₁₋₆ alkyl;
R₈ and R₉ are hydrogen, deuterium or C₁₋₆ alkyl;
X is C; and
Y is O.

23. The compound according to claim 22, wherein the compound is selected from the group consisting of compounds represented by formula (Va) or formula (Vb); 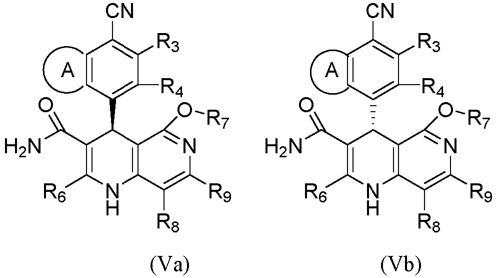 wherein, 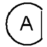 is 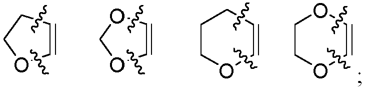
R₃ and R₄ are independently hydrogen or deuterium;
R₅ is 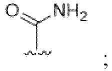
R₆ is C₁₋₆ alkyl;
R₇ is C₁₋₆ alkyl, C₃₋₈ cycloalkyl or C₃₋₈ cycloalkyl C₁₋₆ alkyl; wherein R₇ is unsubstituted or substituted by 1, 2, 3 or 4 Rz; and each Rz is independently halogen or C₁₋₆ alkyl;
R₈ and R₉ are hydrogen, deuterium or C₁₋₆ alkyl;
X is C; and
Y is O.

24. The compound according to any one of claims 22 to 23, wherein:
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted or substituted by 1, 2, 3 or 4 Rz; and each Rz is independently fluorine, chlorine, bromine, iodine or C₁₋₄ alkyl; and
R₈ and R₉ are hydrogen, deuterium or C₁₋₃ alkyl.

25. The compound according to any one of claims 22 to 23, wherein:
R₆ is C₁₋₃ alkyl;
R₇ is C₁₋₃ alkyl, C₃₋₅ cycloalkyl or C₃₋₄ cycloalkyl C₁₋₃ alkyl; wherein R₇ is unsubstituted or substituted by 1, 2, 3 or 4 Rz; and each Rz is independently fluorine or methyl; and
R₈ and R₉ are hydrogen or methyl.

26. The compound according to any one of claims 1 to 3, wherein the compound is selected from the group consisting of compounds represented by formula (VI), formula (VIa) or formula (VIb); 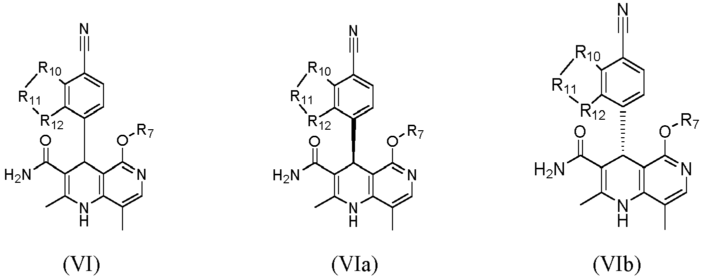
wherein, R₁₀ and R₁₁ are independently selected from -CH₂- or O, and at least one of which is O;
R₁₂ is -CH₂- or -CH₂-CH₂-; when R₁₂ is -CH₂-, R₁₀ and R₁₁ are independently selected from -CH₂- or O, and at least one of which is O; and when R₁₂ is -CH₂-CH₂-, R₁₀ and R₁₁ are both O, or R₁₀ is -CH₂-, R₁₁ is O;
R₇ is C₁₋₃ alkyl, C₃₋₆ cycloalkyl, heterocyclyl consisting of 3 to 6 atoms, heteroaryl consisting of 5 to 6 atoms, C₃₋₆ cycloalkyl C₁₋₄ alkyl, (heterocyclyl consisting of 3 to 6 atoms) C₁₋₄ alkyl or (heteroaryl consisting of 5 to 6 atoms) C₁₋₄ alkyl; wherein R₇ is unsubstituted or substituted by 1, 2, 3 or 4 Rz;
preferably, R₇ is methyl, ethyl, isopropyl, cyclopropyl, cyclobutyl, cyclopentyl, cyclohexyl, oxiranyl, azacyclobutyl, oxacyclobutyl, pyrrolidinyl, tetrahydrofuryl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl, cyclopropyl methyl, cyclopropyl ethyl, cyclobutyl methyl, cyclobutyl ethyl, cyclopentyl methyl, cyclopentyl ethyl, cyclohexyl methyl, cyclohexyl ethyl, azacyclobutyl methyl, azacyclobutyl ethyl, oxacyclobutyl methyl, oxacyclobutyl ethyl, pyrrolidinyl methyl, pyrrolidinyl ethyl, tetrahydrofuryl methyl, tetrahydrofuryl ethyl, tetrahydrothienyl methyl, tetrahydrothienyl ethyl, piperidinyl methyl, piperidinyl ethyl, piperazinyl methyl, piperazinyl ethyl, morpholinyl methyl, morpholinyl ethyl, pyrrolyl methyl, pyrrolyl ethyl, furyl methyl, furyl ethyl, thienyl methyl, thienyl ethyl, thiazolyl methyl, thiazolyl ethyl, pyrazolyl methyl, pyrazolyl ethyl, imidazolyl methyl, imidazolyl ethyl, triazolyl methyl, triazolyl ethyl, tetrazolyl methyl, tetrazolyl ethyl, pyridyl methyl, pyridyl ethyl, pyrimidinyl methyl or pyrimidinyl ethyl; wherein R₇ is unsubstituted or substituted by 1, 2, 3 or 4 Rz;
preferably, each Rz is independently O, deuterium, fluorine, chlorine, bromine, iodine, hydroxyl, cyano, NH₂, C₁₋₄ alkyl, C₁₋₄ alkoxy, C₁₋₄ haloalkoxy, C₁₋₄ haloalkyl, C₁₋₄ alkylamino, C₁₋₄ alkylsulfonyl, C₁₋₄ alkylacyl, C₃₋₆ cycloalkyl, heterocyclyl consisting of 3 to 6 atoms, heteroaryl consisting of 5 to 6 atoms or C₆₋₁₀ aryl; wherein each Rz is independently unsubstituted, or substituted by 1, 2, 3 or 4 Rw; each Rw is independently O, deuterium, fluorine, chlorine, bromine, iodine, hydroxyl, cyano, NH₂, C₁₋₄ alkyl, C₁₋₄ alkoxy, C₁₋₄ haloalkyl, C₁₋₄ haloalkoxy, C₁₋₄ alkylamino, C₁₋₄ alkylsulfonyl, C₁₋₄ alkylacyl, C₃₋₆ cycloalkyl, heterocyclyl consisiting of 3 to 6 atoms, heteroaryl consisting of 5 to 6 atoms or C₆₋₁₀ aryl; and
preferably, each Rz is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl; wherein each Rz is independently unsubstituted, or substituted by 1, 2, 3 or 4 Rw; each Rw is independently O, deuterium, fluorine, chlorine, bromine, iodine, hydroxyl, cyano, NH₂, methyl, ethyl, propyl, butyl, methoxy, ethoxy, propoxy, trifluoromethoxy, monofluoromethoxy, difluoromethoxy, trifluoromethyl, difluoromethyl, monofluoromethyl, methylamino, ethylamino, dimethylamino, methyl ethylamino, diethylamino, methylsulfonyl, ethylsulfonyl, methylacyl, ethylacyl, cyclopropyl, cyclobutyl, cyclopentyl, cyclohexyl, oxiranyl, azacyclobutyl, oxacyclobutyl, pyrrolidinyl, tetrahydrofuranyl, tetrahydrothienyl, thiazolidinyl, pyrazolidinyl, oxazolidinyl, imidazolidinyl, isoxazolidinyl, piperidinyl, piperazinyl, morpholinyl, pyrrolyl, furyl, thienyl, thiazolyl, pyrazolyl, pyridyl, pyrimidinyl or phenyl.

27. The compound according to any one of claims 1 to 26, wherein the compound is selected from:

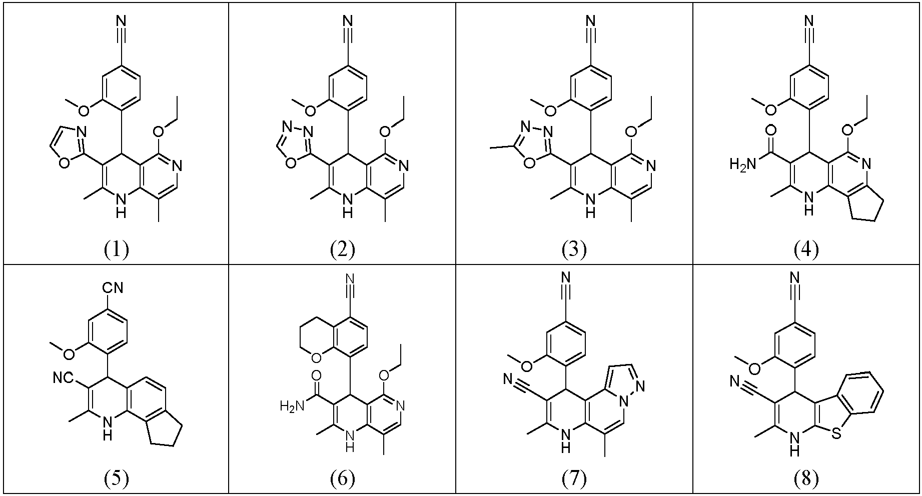

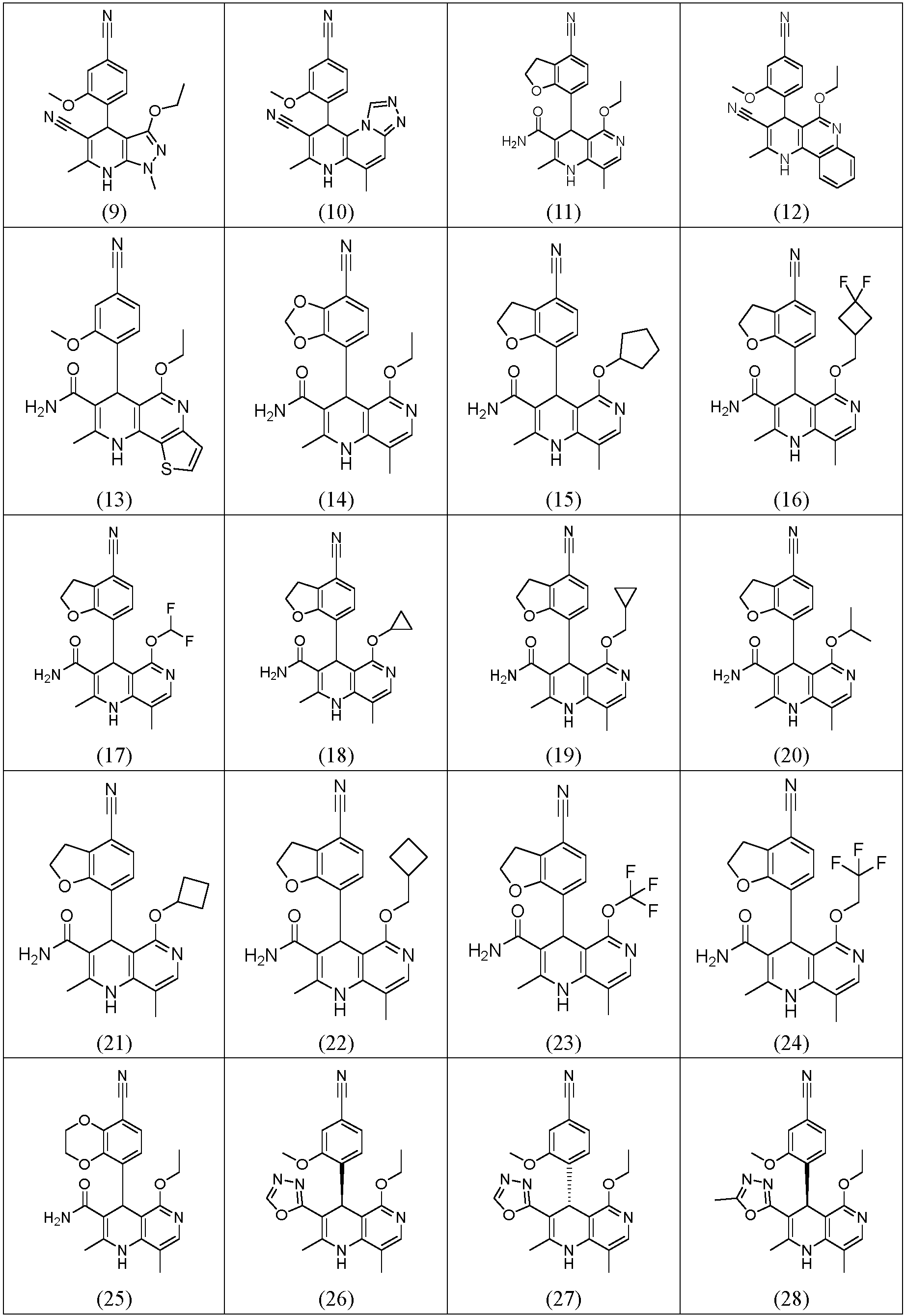

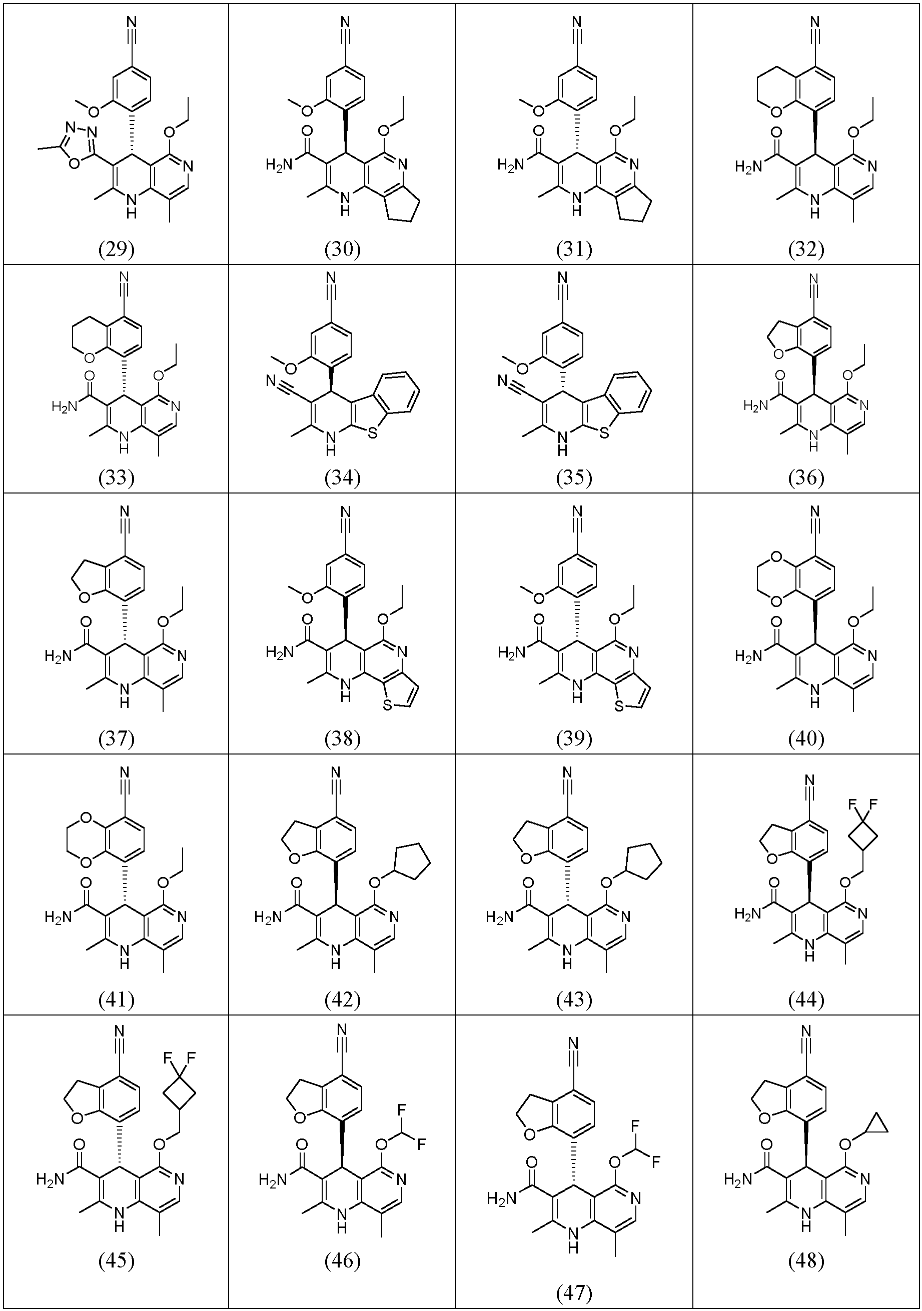

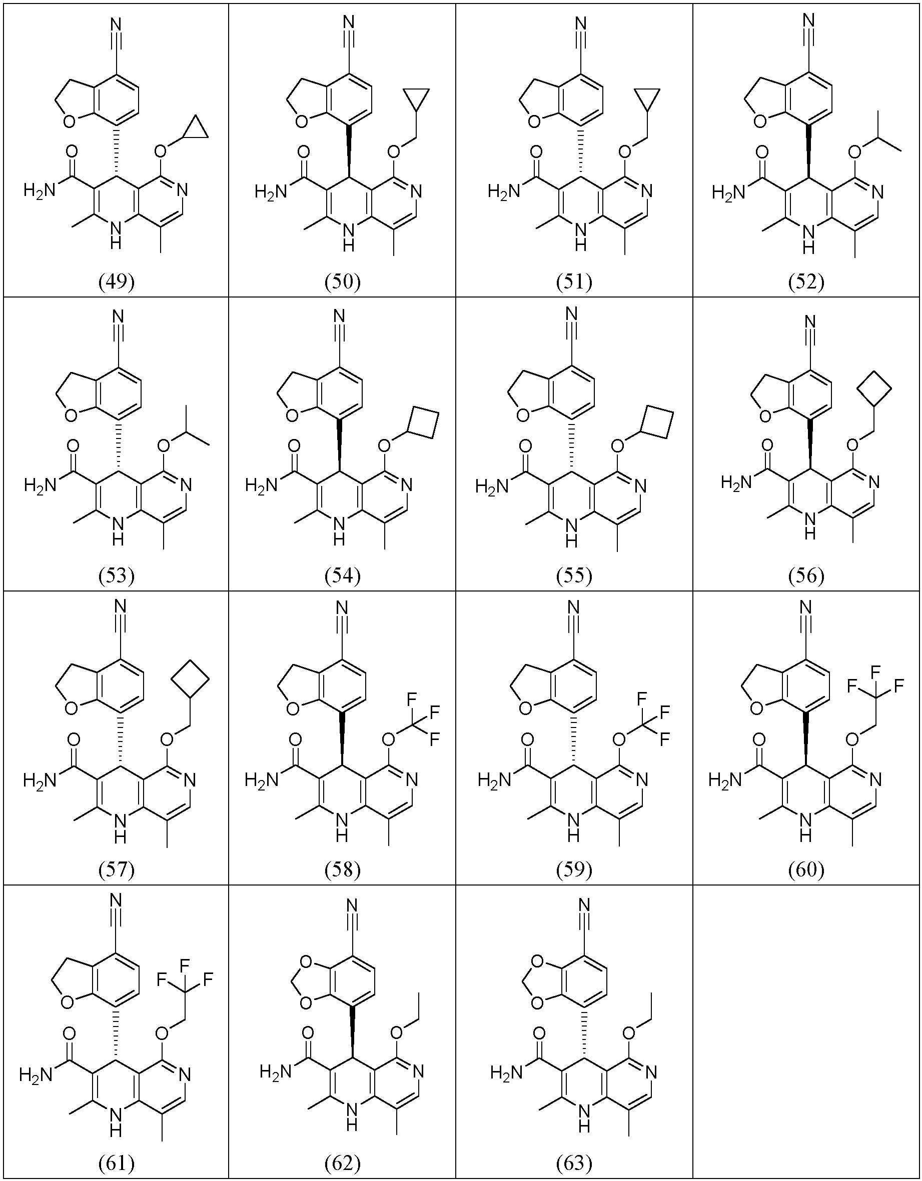

28. A use of the compound according to any one of claims 1 to 27 in preparation of a drug or in preparation of a drug for treating or preventing a mineralocorticoid-related disease or symptom, wherein the drug is preferably used for treating, preventing or alleviating the following disease of a patient: diabetic nephropathy, aldosteronism hyperplasia, hypertension, heart failure, sequelae of myocardial infarction, liver cirrhosis, renal failure or stroke.

29. A use of the compound according to any one of claims 1 to 27 in preparation of a drug, wherein the drug is used as a mineralocorticoid receptor antagonist.
